# EUROPEAN PATENT SPECIFICATION

(11) **EP 3 004 352 B1**
(45) Date of publication and mention of the grant of the patent: **27.09.2017**
(21) Application number: 14726003.8
(22) Date of filing: 26.05.2014
(51) Int. Cl.: C12N 15/113

(54) **OLIGONUCLEOTIDE MODULATORS OF B-CELL CLL/LYMPHOMA 11A (BCL11A) AND USES THEREOF**
OLIGONUKLEOTIDMODULATOREN DES B-ZELL-CLL/LYMPHOMS 11A (BCL11A) UND VERWENDUNGEN DAVON
MODULATEURS OLIGONUCLÉOTIDIQUES DE LA LEUCÉMIE LYMPHOCYTAIRE CHRONIQUE À CELLULES B/LYMPHOME 11A (BCL11A) ET UTILISATIONS DE CEUX-CI

(30) Priority: 24.05.2013 US 201361827484 P
(43) Date of publication of application: 13.04.2016
(73) Proprietor: Roche Innovation Center Copenhagen A/S, 2970 Hørsholm (DK)
(72) Inventor: HEDTJÄRN, Maj, DK-2450 Copenhagen SV (DK); NIELSEN, Niels Fisker, DK-2800 Kgs. Lyngby (DK)
(74) Representative: Hansen, Marianne Rudolph
(86) International application number: PCT/EP2014/060813
(87) International publication number: WO 2014/188001

(56) References cited:
- WO-A1-2013/055985
- WO-A2-2008/113832
- WO-A2-2010/030963
- WO-A2-2012/073047
- WO-A2-2012/079046
- CN-A- 102 329 794
- CN-A- 102 352 356
- ORAPAN SRIPICHAI ET AL: "HbF-Inducing Cytokines and BCL11A shRNA Have Combined Effects Upon Globin Gene Reprogramming In Adult Human Erythroblasts", BLOOD; 52ND ANNUAL MEETING OF THE AMERICAN-SOCIETY-OF-HEMATOLOGY (ASH); ORLANDO, FL, USA; DECEMBER 04 -07, 2010, AMERICAN SOCIETY OF HEMATOLOGY, US, [Online] vol. 116, no. 21, 19 November 2010 (2010-11-19), page 861, XP002722508, ISSN: 0006-4971 Retrieved from the Internet: URL:https://ash.confex.com/ash/2010/webpro gram/Paper33417.html>
- KAMMLER SUSANNE ET AL: "LNA ANTISENSE OLIGONUCLEOTIDES-A STRAIGHT-FORWARD CONCEPT FOR RNA THERAPEUTICS", NUCLEIC ACID THERAPEUTICS, vol. 21, no. 5, October 2011 (2011-10), page A16, XP002727548, & 7TH ANNUAL MEETING OF THE OLIGONUCLEOTIDE-THERAPEUTICS-SOCIETY; COPENHAGEN, DENMARK; SEPTEMBER 08 -10, 2011
- PERALTA RAECHEL ET AL: "Targeting BCL11A and KLF1 For The Treatment Of Sickle Cell Disease and beta-Thalassemia In Vitro using Antisense Oligonucleotides", BLOOD, vol. 122, no. 21, November 2013 (2013-11), page 1022, XP002727549, & 55TH ANNUAL MEETING OF THE AMERICAN-SOCIETY-OF-HEMATOLOGY; NEW ORLEANS, LA, USA; DECEMBER 07 -10, 2013

## Description

### BACKGROUND

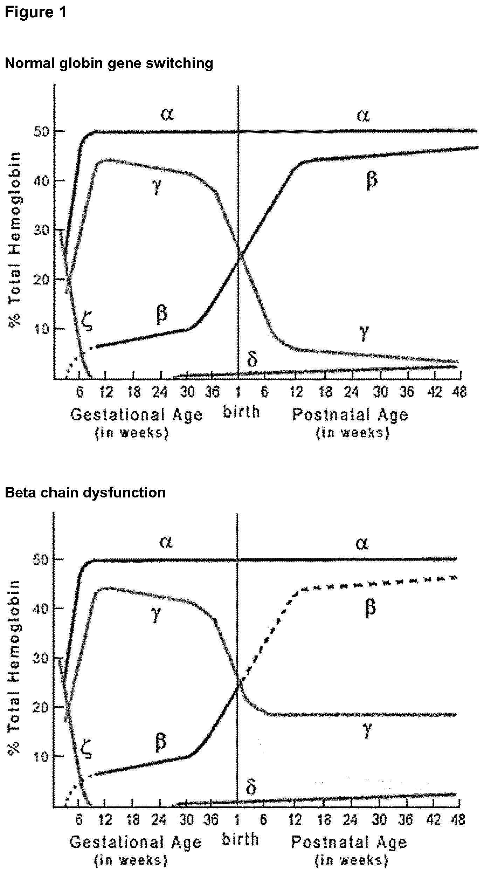

Hemoglobinopathies are diseases that relate to the dysfunction of the hemoglobin protein. Typically, these diseases involve either a lack of or malfunctioning hemoglobin protein, which originate from genetic mutations in globin genes (*e.g*., alpha, beta, *etc.*; Figure 1). Hemoglobinopathies are one group of a broad spectrum of red blood cell associated disorders that are characterized by single-gene inherited disorders that, in most cases, are autosomal co-dominant traits. It is estimated that about 7% of the world's population are carriers. Hereditary hemoglobinopathies manifest in one of three forms: thalassemia (alpha, beta, delta), sickle-cell disease and hereditary persistence of fetal hemoglobin (HbF). Sickle cell disease (SCD) and beta-thalassemia are the most common forms of hemoglobinopathies and are major causes of morbidity and mortality world-wide. SCD, as the name implies, involves changes in the structure of a globin protein arising from a mutation and results in a malfunctioning hemoglobin protein, while thalassemias are associated with mutations in globin genes that yield an underproduction of normal globin proteins. This can occur through mutations in regulatory proteins. Anemia, in some cases sever, is a common result of hemoglobin dysfunction.

Various treatments for hemoglobinopathies have been explored over time. A major focus has typically been on restoring hemoglobin function, for example, by increasing the level of fetal hemoglobin (HbF). However, not many effective treatments have been successfully developed. Recently, the understanding of mechanisms that regulate HbF has been an area of much research. It was reported that BCL11A is expressed in adult erythroid precursor cells in the bone marrow and functions to repress γ-globin production (Sankaran et al. 2008 Science vol 322 page 1839-1842).

WO 2010/030963 describes modulation of BCL11A using a pool of siRNA samples against 4 target sequences. There is no indication as to whether the individual target sequences are able to down regulate BCL11A.
WO 2012/079046 describes double-stranded ribonucleic acid (dsRNA) compositions targeting the BCL11A gene.

### SUMMARY

The present invention provides, among other things, antisense oligonucleotide modulators (e.g., inhibitors) of BCL11A and methods and compositions for treating BCL11A-related diseases, disorders or conditions based on such modulators. It is contemplated that antisense oligonucleotides provided by the present invention are particularly useful for treating hemoglobinopathies, such as sickle cell disease and β-thalassemias.

In one aspect, the present invention provides an antisense oligonucleotide capable of down-regulating or decreasing expression of human BCL11A having a sequence that is at least 80% (e.g. at least about 85%, 90%, 95%, 96%, 97%, 98%, or 99%) identical to the reverse complement of a continuous sequence within a region selected from nucleotides 410 to 450 of the human BCL11A gene of SEQ ID NO 1 or a messenger RNA (mRNA) isoform of BCL11A, wherein the antisense oligonucleotide is a gapmer. In some embodiments, an antisense oligonucleotide of the present invention is less than 19 nucleotides in length. In some embodiments, an antisense oligonucleotide of the present invention is less than 18 nucleotides in length.

In one aspect, the present invention provides an antisense oligonucleotide capable of down-regulating or decreasing expression of human BCL11A having less than 18 nucleotides (e.g, less than 17, 16, 15, 14, 13, or 12) in length and a sequence that is at least about 80% (e.g., at least about 85%, 90%, 95%, 96%, 97%, 98%, or 99%) identical to the reverse complement of a continuous sequence within a region selected from nucleotides 410 to 450 of the human BCL11A gene of SEQ ID NO 1 or an messenger RNA (mRNA) isoform of BCL11A.

In one aspect, the present invention provides an antisense oligonucleotide capable of down-regulating or decreasing expression of human BCL11A having a sequence at least about 80% (e.g, at least about 85%, 90%, 95%, 96%, 97%, 98%, or 99%) identical to the reverse complement of a continuous sequence within a region selected from nucleotides 410 to 450 of the human BCL11A gene of SEQ ID NO 1 or a messenger RNA (mRNA) isoform of BCL11A and is represented by the formula X*ₐ*-Y*_{b}*-X_{*a*'}, wherein X is a nucleotide analogue; Y is a continuous sequence of DNA; *a* is 1, 2, 3, 4 or 5; *a'* is 1, 2, 3, 4 or 5; and *b* is an integer number between 5 and 15. In some embodiments, the nucleotide analogue is a locked nucleic acid (LNA).

In some embodiments, a and *a'* are different. In some embodiments, a and a*'* are the same. In some embodiments, *a* and/or *a'* is 1. In some embodiments, *a* and/or *a'* is 2. In some embodiments, *a* and/or *a'* is 3. In some embodiments, *a* and/or *a'* is 4. In some embodiments, *a* and/or *a'* is 5.

In some embodiments, *b* is an integer number between 5 and 15, inclusive. In some embodiments, *b* is an integer number between 5 and 10, inclusive. In some embodiments, *b* is an integer number between 7 and 11, inclusive. In some embodiments, *b* is an integer number selected from the group consisting of 5, 6, 7, 8, 9, 10, 11, 12, 13, 14 and 15.

In some embodiments, the isoform of BCL11A is selected from the group consisting of XL, L, M, S and XS or homologs or orthologs thereof. In some embodiments, an antisense oligonucleotide of the present invention is capable of down-regulating or decreasing the expression of the mouse BCL11A gene.

In some embodiments, antisense oligonucleotides of the present invention comprises or contains at least one, at least two, at least three, at least four, at least five, at least six, at least seven, at least eight or more nucleoside analogues. In some embodiments, an antisense oligonucleotide of the present invention comprises from 3-8 nucleotide analogues, *e.g.* 6 or 7 nucleotide analogues. In some embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments, all the nucleotides analogues may be LNA.

In some embodiments, an oligonucleotide of the present invention comprises or contain at least one, at least two, at least three, at least, four, at least five, at least six, at least seven, at least eight or more LNA units. In some embodiments, the one or more LNA units are located at the 5' and/or '3 ends of the antisense oligonucleotide. In some embodiments, an antisense oligonucleotide of the present invention comprises at least one, at least two or at least three LNA units at the 5' and/or '3 ends. In some embodiments, an antisense oligonucleotide of the present invention comprises at least one, at least two or at least three LNA units internally.

In some embodiments, the LNA unit(s) is a beta-D-oxy-LNA nucleotide. In some embodiment, an antisense oligonucleotide of the present invention comprises one or more additional chemical modifications. In some embodiments, an antisense oligonucleotide of the present invention comprises one or more additional chemical modifications that include a 2'O-methyl modification and/or a phosphorothioate linkage. In some embodiments, an antisense oligonucleotide of the present invention comprises at least one LNA unit that is a LNA 5-methylcytosine nucleotide.

In some embodiments, an antisense oligonucleotide of the present invention has 10-17, 10-16, 10-15, 10-14, 10-13, 10-12, 11-17, 11-16, 11-15, 11-14, 11-13, 12-17, 12-16, 12-15, or 12-14 nucleotides in length. In some embodiments, an antisense oligonucleotide of the present invention has 12-16 nucleotides in length.

In some embodiments, an antisense oligonucleotide of the present invention has a sequence that is identical to the reverse complement of a continuous sequence within a region selected from nucleotides 410 to 450 of the human BCL11A gene of SEQ ID NO 1 or an messenger RNA (mRNA) isoform of human BCL11A.

An alternative aspect of the present invention is an antisense oligonucleotide capable of decreasing expression of human BCL11A comprising a sequence that is at least 80% identical to the reverse complement of a continuous sequence within a region selected from nucleotides 1-283 (Exon 1), nucleotides 284 - 613 (Exon 2), or nucleotides 614 - 715 (Exon 3) of the human BCL11A gene.

In some embodiments, a continuous sequence according to the present invention is within nucleotides 410 - 450 of the human BCL11A mRNA isoform XL.

In some embodiments, a continuous sequence according to the present invention is within nucleotides 415 - 436 of the human BCL11A mRNA isoform XL.

In some embodiments, a continuous sequence according to the present invention is within nucleotides 420 - 450 of the human BCL11A mRNA isoform XL.

In some embodiments, the oligonucleotide of the invention comprises or consists a sequence motif selected from the group consisting of 5'- ATTGCATTGTTTCCG-3' (SEQ ID NO: 63), 5'- GTTTGTGCTCGAT-3' (SEQ ID NO: 64), 5'- CATTGCATTGTTTCCG-3'(SEQ ID NO: 65), 5'- CGTTTGTGCTCGAT-3'(SEQ ID NO: 66), 5'- CGTTTGTGCTCGATAA-3'(SEQ ID NO: 67), 5'- CCGTTTGTGCTCGA-3'(SEQ ID NO: 68), 5'- CGTTTGTGCTCGA-3' (SEQ ID NO: 69), 5'- TTTGTGCTCGATAA-3'(SEQ ID NO: 70), 5'- TTGTGCTCCATAA-3' (SEQ ID NO: 71) and 5'- TTTCCGTTTGTGCTCG (SEQ ID NO: 72), 5'- ATTGCATTGTTTCCGT-3' (SEQ ID NO: 73), 5'-CGTTTGTGCTCGATA-3' (SEQ ID NO: 74).

In some embodiments, an antisense oligonucleotide of the present invention has a sequence selected from Table 2.

In some embodiments, an antisense oligonucleotide of the present invention has a sequence selected from SEQ ID NO: 11, SEQ ID NO: 15, SEQ ID NO: 32, SEQ ID NO: 21, SEQ ID NO: 34, SEQ ID NO:10, SEQ ID NO: 12, SEQ ID NO: 13, SEQ ID NO: 14, SEQ ID NO: 16, SEQ ID NO: 17, SEQ ID NO: 18, SEQ ID NO: 19, SEQ ID NO: 20, SEQ ID NO: 22, SEQ ID NO: 23, SEQ ID NO: 24, SEQ ID NO: 25, SEQ ID NO: 26, SEQ ID NO:27, SEQ ID NO: 28, SEQ ID NO: 29, SEQ ID NO: 30 SEQ ID NO: 31, SEQ ID NO: 33, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 60, SEQ ID NO: 61 or SEQ ID NO: 62,

In some embodiments, an antisense oligonucleotide of the present invention is selected from 5'-^{m}Cₛ^{o}Aₛ^{o}Tₛ^{o}tₛgₛcₛaₛtₛtₛgₛtₛtₛtₛ^{m}Cₛ^{om}Cₛ^{o}G^{o}-3' (SEQ ID NO: 11), 5' - **^{m}Cₛ^{o}Gₛ^{o}Tₛ^{o}**tₛ tₛgₛtₛgₛcₛtₛ^{m}cₛgₛaₛ**Tₛ^{o}Aₛ^{o}A^{o}**- 3' (SEQ ID NO: 15) or 5' - **^{m}Cₛ^{o}Gₛ^{o}Tₛ^{o}**tₛtₛgₛtₛgₛcₛtₛ^{m}cₛgₛ**Aₛ^{o} Tₛ^{o}A^{o}**- 3' (SEQ ID NO: 32), 5'-**Tₛ^{o}Tₛ^{o}Gₛ^{o}**tₛgₛcₛtₛ^{m}cₛgₛaₛtₛ**Aₛ^{o}Aₛ^{o}A^{o}**-3' (SEQ ID NO: 14) and 5' - **^{m}Cₛ^{o}Gₛ^{o}Tₛ^{o}**tₛtₛgₛtₛgₛcₛtₛcₛ**Gₛ^{o}Aₛ^{o}T^{o}**- 3' (SEQ ID NO: 35), wherein upper case letters indicate locked nucleic acid (LNA) units, subscript "s" represents phosphorothioate linkage, and lower case letters represent deoxyribonucleotide (DNA) units, "^{m}C" represents 5' methyl-cytosine LNA unit, and "^{m}c" represents 5' methyl-cytosine DNA unit.

In some embodiments, an antisense oligonucleotide of the present invention is 5'-^{m}Cₛ^{o}Aₛ^{o} Tₛ^{o}tₛgₛcₛaₛtₛtₛgₛtₛtₛtₛ^{m}Cₛ^{om}Cₛ^{o}G^{o}-3'(SEQ ID NO: 11).

In another aspect, the present invention provides an antisense oligonucleotide capable of down-regulating or decreasing the expression of the human BCL11A gene having a sequence at least 80% (*e.g.,* 85%, 90%, 91%, 92%, 93%, 94%, 95%, 96%, 97%, 98%, 99% or more) identical to an oligonucleotide sequence selected from Table 2.

In some embodiments, an antisense oligonucleotide according to the present invention has a sequence at least 80% (*e.g.,* 85%, 90%, 91%, 92%, 93%, 94%, 95%, 96%, 97%, 98%, 99% or more) identical to an oligonucleotide sequence selected from 5'- ^{m}Cₛ^{o}Aₛ^{o}Tₛ^{o}tₛgₛcₛaₛtₛ tₛgₛtₛtₛtₛ^{m}Cₛ^{om}Cₛ^{o}G^{o}-3' (SEQ ID NO: 11), 5' - **^{m}Cₛ^{o}Gₛ^{o}Tₛ^{o}**tₛtₛgₛtₛgₛcₛtₛ^{m}cₛgₛaₛ**Tₛ^{o}Aₛ^{o}A^{o}-** 3' (SEQ ID NO: 15) or 5' - **^{m}Cₛ^{o}Gₛ^{o}Tₛ^{o}**tₛtₛgₛtₛgₛcₛtₛ^{m}cₛgₛ**Aₛ^{o}Tₛ^{o}A^{o}-** 3' (SEQ ID NO: 32) 5'-**^{m}Cₛ^{o}Gₛ^{o}Tₛ^{o}**ts ts gs ts gs cₛts ^{m}cₛgs **Aₛ^{o}Tₛ^{o}A^{o}-** 3' (SEQ ID NO: 32), 5' - **^{m}Cₛ^{o}Gₛ^{o}Tₛ^{o}**tₛtₛgₛtₛgₛ cₛtₛcₛ**Gₛ^{o}Aₛ^{o}T^{o}**- 3' (SEQ ID NO: 35), 5' - **Tₛ^{o}Tₛ^{o}Gₛ^{o}**tₛgₛcₛtₛ^{m}cₛgₛaₛtₛ**Aₛ^{o}Aₛ^{o}A^{o}**- 3' (SEQ ID NO: 14) and 5' - **^{m}Cₛ^{o}Gₛ^{o}Tₛ^{o}**tₛtₛgₛtₛgₛcₛtₛcₛ**Gₛ^{o}Aₛ^{o}T^{o}-** 3' (SEQ ID NO: 35), wherein upper case letters indicate locked nucleic acid (LNA) units, subscript "s" represents phosphorothioate linkage, and lower case letters represent deoxyribonucleotide (DNA) units, "^{m}C" represents 5' methyl-cytosine LNA unit, and "^{m}c" represents 5' methyl-cytosine DNA unit.

In some embodiments, a pharmaceutical composition comprising an antisense oligonucleotide as described herein and a pharmaceutically acceptable carrier is provided.

Among other things, the present invention provides a method of inhibiting BCL11A in a subject comprises administering to a subject in need of treatment an antisense oligonucleotide or a pharmaceutical composition as described herein.

Among other things , the present invention provides an antisense oligonucleotide for use in a method of inhibiting BCL11A comprising a step of administering to a subject in need of treatment an antisense oligonucleotide or a pharmaceutical composition as described herein.

In some embodiments, the present invention provides use of an antisense oligonucleotide or pharmaceutical composition of the present invention in the manufacture of a medicament for the treatment of an anemic disease, disorder or condition, such as sickle cell disease or β-thalassemia. In particular in the manufacture of a medicament for inhibiting BCL11A comprising administering an antisense oligonucleotide or pharmaceutical composition as described herein to a subject.

In some embodiments, the present invention provides, the antisense oligonucleotide according pharmaceutical composition of the present invention, for use as a medicament, such as for the treatment of an anemic disease, disorder or condition, such as sickle cell disease or β-thalassemia.

In some embodiments, the present invention provides a method of treating an anemic disease, disorder or condition in a subject comprises administering to a subject in need of treatment an antisense oligonucleotide or a pharmaceutical composition as described herein.

In some embodiments, the present invention provides an antisense oligonucleotide for use in the treatment of a disease or disorder such as those referred to herein, such as a hemoglobinopathie, such as an anemic disease, disorder or condition, such as thalassemia (α, β, δ), sickle-cell disease and hereditary persistence of fetal hemoglobin (HbF.)

In some embodiments, the present invention provides an antisense oligonucleotide for use in a method of treating an anemic disease, disorder or condition in a subject comprising administering to a subject in need of treatment an antisense oligonucleotide or a pharmaceutical composition as described herein. In some embodiments, treatment methods of the present invention further comprise administering a second agent to a subject for the treatment of a disease or disorder, such as for the treatment of an anemic disease, disorder or condition.

In some embodiments, the anemic disease, disorder or condition treated by a method of the present invention is sickle cell disease.

In some embodiments, the anemic disease, disorder or condition treated by a method of the present invention is β-thalassemia.

In some embodiments, the administering of the antisense oligonucleotide or the pharmaceutical composition results in reduced expression of BCL11A in one or more target tissues. In some embodiments, the administering of the antisense oligonucleotide or the pharmaceutical composition results in increased γ-globin expression in one or more target tissues. In some embodiments, the administering of the antisense oligonucleotide or the pharmaceutical composition results in increased fetal hemoglobin production in one or more target tissues. In some embodiments, one or more target tissues are selected from bone marrow, liver, kidney, spleen, plasma cells, thymus, tonsillar epithelium, erythroid progenitor cells, pluripotent stem cells, dendritic cells and/or peripheral blood B-cells. In some embodiments, an antisense oligonucleotide or pharmaceutical composition is administered intravenously. In some embodiments, an antisense oligonucleotide or pharmaceutical composition is administered subcutaneously.

In some embodiments, the present invention provides a container comprising an antisense oligonucleotide or pharmaceutical composition as described herein. In some embodiments, an antisense oligonucleotide or pharmaceutical compostion of the present invention is provided in a single dosage form. In some embodiments, an antisense oligonucleotide or pharmaceutical composition of the present invention is provided in multiple (e.g, two, three, four, five or more) dosage form. In some embodiments, an antisense oligonucleotide or pharmaceutical composition of the present invention is provided in lyophilized form. In some embodiments, an antisense oligonucleotide or pharmaceutical composition of the present invention is provided in liquid form.

In some embodiments, the container is selected from an ampule, a vial, a cartridge, a reservoir, a lyo-ject, and a pre-filled syringe. In some embodiments, the container is a pre-filled syringe and is optionally selected from a borsilicate glass syringe with baked silicone coating, a borosilicate glass syringe with sprayed silicone, and a plastic resin syringe without silicone.

As used in this application, the terms "about" and "approximately" are used as equivalents. Any numerals used in this application with or without about/approximately are meant to cover any normal fluctuations appreciated by one of ordinary skill in the relevant art. Other features, objects, and advantages of the present invention are apparent in the detailed description that follows. It should be understood, however, that the detailed description, while indicating embodiments of the present invention, is given by way of illustration only, not limitation. Various changes and modifications within the scope of the invention will become apparent to those skilled in the art from the detailed description.

### BRIEF DESCRIPTION OF DRAWINGS

The drawings are for illustration purposes only, not for limitation.
**Figure 1** shows an exemplary illustration of the percent of total hemoglobin (y-axis) versus gestational and postnatal age in weeks (x-axis) for different globin chains in normal individuals (left) and individuals with beta globin chain dysfunction (right). Adapted and modified from Figure 167-2, Chapter 167. Cecil Medicine 23rd ed., Lee W. Goldman, Dennis A. Ausiello. W.B. Saunders Elsevier 2008.
**Figure 2** shows a schematic illustration, not to scale, of the three major isoforms of human BCL11A (S, L, and XL). Exons are labeled as they are found in each isoform from 5' to 3'.
**Figure 3** shows exemplary inhibition of BCL11A-XL mRNA expression in human REH cells by 401 antisense oligonucleotides targeting BCL11A at a concentration of 25 µM.
**Figure 4** shows exemplary inhibition of BCL11A-XL mRNA expression in human REH cells by selected antisense oligonucleotides targeting BCL11A at oligonucleotide concentrations ranging from 0.0064 to 20 µM.
**Figure 5** shows exemplary inhibition of BCL11A-XL mRNA expression in human REH cells by selected antisense oligonucleotides designed from oligo 4 (top) and 5 (bottom) targeting BCL11A at oligonucleotide concentrations ranging from 0.25 to 60 µM.
**Figure 6** shows exemplary inhibition of the major isoforms (S, L, and XL) of BCL11A mRNA expression in human REH cells by selected antisense oligonucleotides at concentrations ranging from 0.25 to 60 µM. Measurements of mRNA of BCL11A XL, L and S isoforms are shown in the left, middle and right columns for each concentration within each treatment group, respectively.
**Figure 7** shows exemplary inhibition of mouse BCL11A mRNA expression in mouse MPC-11 cells by selected antisense oligonucleotides at concentrations ranging from 0.08 to 20 µM. Measurement of all isoforms (BCL11A-All) and isoform L (BCL11A-L) of mouse BCL11A are shown in the left and right columns for each concentration within each treatment group, respectively.
**Figure 8** shows exemplary inhibition of BCL11A mRNA expression in the bone marrow (top) and spleen (bottom) of groups of female NMRI mice dosed with 15 mg/kg of selected antisense oligonucleotides targeting BCL11A. Measurements for bone marrow include all isoforms (left column) and isoform L (right column) for each antisense oligonucleotide treatment group.
**Figure 9** shows exemplary inhibition of BCL11A mRNA in bone marrow of wild-type C57BL/6 mice four weeks after administration with 25 or 15 mg/kg of selected antisense oligonucleotides targeting BCL11A.

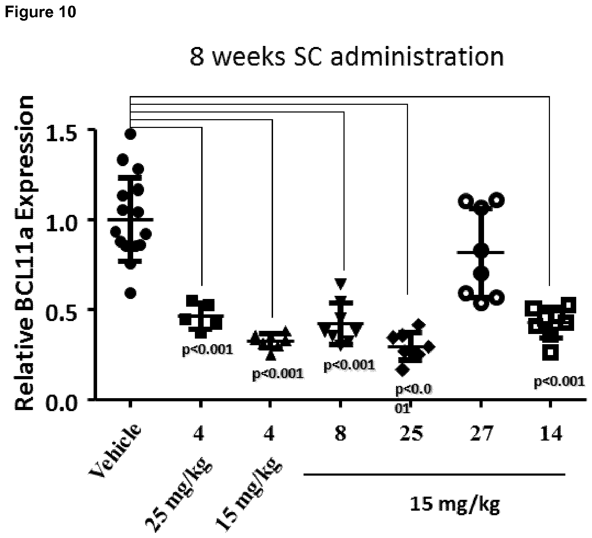

**Figure 10** shows exemplary inhibition of BCL11A mRNA in bone marrow of wild-type C57BL/6 mice eight weeks after administration with 25 or 15 mg/kg of selected antisense oligonucleotides targeting BCL11A.
**Figure 11** shows exemplary inhibition of BCL11A mRNA in bone marrow of human β-YAC transgenic mice eight weeks post administration with 15 mg/kg of selected antisense oligonucleotides targeting BCL11A.
**Figure 12** shows exemplary inhibition of BCL11A mRNA in Ter119⁺ and CD19⁺ bone marrow cell populations of human β-YAC transgenic mice eight weeks post administration with 15 mg/kg of selected antisense oligonucleotides targeting BCL11A.
**Figure 13** shows exemplary total hemoglobin (g/L) in peripheral blood of non-human primate animals in phlebotomized (phleb.) and non-phlebotomized treatment groups at various treatment days. Vehicle control (saline) and candidate oligonucleotide 4 (10 and 20 mg/kg) treatment groups are indicated.
**Figure 14** shows exemplary percent of reticulocytes in peripheral blood of non-human primate animals in phlebotomized (phleb.) and non-phlebotomized treatment groups at various treatment days. Vehicle control (saline) and candidate oligonucleotide 4 (10 and 20 mg/kg) treatment groups are indicated.
**Figure 15** shows exemplary expression of BCL11A normalized to GAPDH in humerus bone marrow of phlebotomized non-human primate animals dosed with vehicle control (saline) or candidate oligonucleotide 4 at 20 mg/kg at week seven of a study. Measurements for isoform XL (left column) and all isoforms (right column) is shown for each treatment animal.
**Figure 16** shows exemplary γ- and β-globin mRNA expression normalized to GAPDH in humerus bone marrow of phlebotomized non-human primate animals dosed with vehicle control (saline) or candidate oligonucleotide 4 at 20 mg/kg at week seven of a study. Measurements of human γ-globin (Gamma A+G ,column 1), *Macaca mulatta* γ-globin (HBG2, column 2), Macaca *mulatta* β-globin (HBB, column 3; HBB_mH, column 4) are shown for each animal within each treatment group.
**Figure 17** shows exemplary expression of BCL11A mRNA normalized to GAPDH in humerus (top) and femur (bottom) bone marrow of phlebotomized non-human primate animals for control (saline) and candidate oligonucleotide 4 (10 and 20 mg/kg) treatment groups at week 17 of a study. Measurements of isoform XL (left column) and all isoforms (right column) are shown for each animal within each treatment group.
**Figure 18** shows exemplary expression of γ-globin mRNA normalized to GAPDH in humerus (top) and femur (bottom) bone marrow in phlebotomized non-human primate animals for control (saline) and candidate oligonucleotide 4 (10 and 20 mg/kg) treatment groups at week 17 of a study. Measurements of human γ-globin (Gamma A+G, left column) and Macaca *mulatta* γ-globin (HBG2, right column) are shown for each animal within each treatment group.
Figure 19 shows exemplary expression of γ- and β-globin mRNA normalized to GAPDH in humerus bone marrow in control (saline) and candidate oligonucleotide 4 (10 and 20 mg/kg) treatment groups of phlebotomized non-human primate animals at week 17. Columns from left to right for each animal within each treatment group measurements of human γ-globin (Gamma A+G), Macaca *mulatta* γ-globin (HBG2), Macaca *mulatta* β-globin (RhHBB), and Macaca *mulatta* β-globin (RhHBB_mH), respectively.
**Figure 20** shows exemplary expression of γ- and β-globin mRNA normalized to GAPDH in femur bone marrow in control (saline) and candidate oligonucleotide 4 (10 and 20 mg/kg) treatment groups of phlebotomized non-human primate animals at week 17. Columns from left to right for each animal within each treatment group measurements of human γ-globin (Gamma A+G), *Macaca mulatta* γ-globin (HBG2), Macaca *mulatta* β-globin (RhHBB), and Macaca *mulatta* β-globin (RhHBB_mH), respectively.
**Figure 21** shows exemplary average expression of BCL11A (top) and γ-globin (bottom) mRNA normalized to GAPDH in humerus bone marrow of phlebotomized non-human primate animals in control (saline) and candidate oligonucleotide 4 (10 and 20 mg/kg) treatment groups at week 17. Measurements of isoform XL (left column) and all isoforms (right column) of BCL11A are shown for each animal within each treatment group. Measurements of human γ-globin (Gamma A+G, left column) and *Macaca mulatta* γ-globin (HBG2, right column) are shown for each animal within each treatment group.
**Figure 22** shows exemplary average expression of BCL11A (top) and γ-globin (bottom) mRNA normalized to GAPDH in femur bone marrow of phlebotomized non-human primate animals in control (saline) and candidate oligonucleotide 4 (10 and 20 mg/kg) treatment groups at week 17. Measurements of isoform XL (left column) and all isoforms (right column) of BCL11A are shown for each animal within each treatment group. Measurements of human γ-globin (Gamma A+G, left column) and *Macaca mulatta* γ-globin (HBG2, right column) are shown for each animal within each treatment group.
**Figure 23** shows exemplary fraction (‰) of F-cells in bone marrow for phlebotomized non-human primate animals at full scale (left) and zoomed-in scale (right).
**Figure 24** shows exemplary fraction (‰) of F-cells in peripheral blood for phlebotomized non-human primate animals at full scale (left) and zoomed-in scale (right).
**Figure 25** shows exemplary measurements of γ-globin protein in peripheral blood of non-human primate animals in control (top), 10 mg/kg (middle), and 20 mg/kg (bottom) treatment groups at various weeks after first dose.
**Figure 26** shows exemplary measurements of γ-globin protein in peripheral blood of non-human primate animals as a percent of control at a respective time point of a γ-globin peak ("peak 1" or "peak 2") in control treated groups.
**Figure 27** shows exemplary measurements of γ-globin protein in peripheral blood of non-human primate animals as a percent of control at a respective time point of a γ-globin peak ("peak 1" or "peak 2") in 10 mg/kg dose groups.
**Figure 28** shows exemplary measurements of γ-globin protein in peripheral blood of non-human primate animals as a percent of control at a respective time point of a γ-globin peak ("peak 1" or "peak 2") in 20 mg/kg dose groups.
**Figure 29** shows exemplary measurements of plasma concentration of antisense oligonucleotide 4 over time in wild-type mice.
**Figure 30** shows exemplary measurements of concentration of antisense oligonucleotide 4 in various tissues over time from wild-type mice.
**Figure 31** shows exemplary measurements of tissue concentration of antisense oligonucleotide 4 in various tissues over time from wild-type mice.
**Figure 32** shows an exemplary model of predicted concentration of an antisense oligonucleotide of the present invention in bone marrow based on a single dose pharmacokinetic study.

### DEFINITIONS

In order for the present invention to be more readily understood, certain terms are first defined below. Additional definitions for the following terms and other terms are set forth throughout the specification.

*Approximately or about:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1 %, or less in either direction (greater than or less than) of the stated reference value unless otherwise stated or otherwise evident from the context (except where such number would exceed 100% of a possible value).

*Biologically active:* As used herein, the phrase "biologically active" refers to a characteristic of any agent that has activity in a biological system, *in vitro* or *in vivo* (e.g., in an organism). For instance, an agent that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

*Improve, increase, reduce or inhibit*: As used herein, the terms "improve," "increase," "reduce" or "inhibit" or grammatical equivalents, indicate values that are relative to a baseline measurement, such as a measurement in the same individual prior to initiation of the treatment described herein, or a measurement in a control individual (or multiple control individuals) in the absence of the treatment described herein. A "control individual" is an individual afflicted with the same form of disease as the individual being treated, who is about the same age as the individual being treated (to ensure that the stages of the disease in the treated individual and the control individual(s) are comparable).

*Individual, subject, patient*: As used herein, the terms "subject," "individual" or "patient" refer to a human or a non-human mammalian subject. The individual (also referred to as "patient" or "subject") being treated is an individual (fetus, infant, child, adolescent, or adult human) suffering from a disease.

*Locked Nucleic Acid (LNA):* As used herein, the term "LNA" or "Locked Nucleic Acid" refers to a bicyclic nucleotide analogue, preferably a bicyclic nucleotide analogue with a bridge between the 2' and 4' position in the ribose ring (2' to 4' bicyclic nucleotide analogue). LNA is in the literature sometimes referred to as BNA (bridged nucleic acid or bicyclic nucleic acid). It may refer to an LNA monomer, or when used in the context of an "LNA oligonucleotide" refers to an oligonucleotide containing one or more such bicyclic nucleotide analogues.

*Nucleotide:* As used herein, the term "nucleotide", refers to a glycoside comprising a sugar moiety, a base moiety and a covalently linked phosphate group and covers both naturally occurring nucleotides, such as DNA or RNA, preferably DNA, and non-naturally occurring nucleotides comprising modified sugar and/or base moieties, which are also referred to as "nucleotide analogues" herein. In some embodiments, non-naturally occurring nucleotides include nucleotides which have modified sugar moieties, such as bicyclic nucleotides or 2' modified nucleotides, such as 2' substituted nucleotides. In some embodiments, non-naturally occurring nucleotides include locked nucleic acid (LNA).

*Substantial homology:* The phrase "substantial homology" is used herein to refer to a comparison between amino acid or nucleic acid sequences. As will be appreciated by those of ordinary skill in the art, two sequences are generally considered to be "substantially homologous" if they contain homologous residues in corresponding positions. Homologous residues may be identical residues. Alternatively, homologous residues may be non-identical residues with appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains. Substitution of one amino acid for another of the same type may often be considered a "homologous" substitution.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology. In some embodiments, two sequences are considered to be substantially homologous if at least 50%, 55%, 60%, 65%, 70%, 75%, 80%, 85%, 90%, 91%, 92%, 93%, 94%, 95%, 96%, 97%, 98%, 99% or more of their corresponding residues are homologous over a relevant stretch of residues. In some embodiments, the relevant stretch is a complete sequence. In some embodiments, the relevant stretch is at least 9, 10, 11, 12, 13, 14, 15, 16, 17 or more residues. In some embodiments, the relevant stretch includes contiguous residues along a complete sequence. In some embodiments, the relevant stretch includes discontinuous residues along a complete sequence. In some embodiments, the relevant stretch is at least 10, 15, 20, 25, 30, 35, 40, 45, 50, or more residues.

*Substantial identity.* The phrase "substantial identity" is used herein to refer to a comparison between amino acid or nucleic acid sequences. As will be appreciated by those of ordinary skill in the art, two sequences are generally considered to be "substantially identical" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences as well as EMBOSS needel for global alignments or EMBOSS Water for local alignments. Exemplary such programs are described in Altschul, et al., Basic local alignment search tool, J. Mol. Biol., 215(3): 403-410, 1990; Altschul, et al., Methods in Enzymology*;* Altschul et al., Nucleic Acids Res. 25:3389-3402, 1997; Baxevanis et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. In some embodiments, two sequences are considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding residues are identical over a relevant stretch of residues. In some embodiments, the relevant stretch is the complete sequence of the oligonucleotide. In some embodiments, the relevant stretch is at least 10, 15, 20, 25, 30, 35, 40, 45, 50, or more residues. *Target tissues*: As used herein , the term "target tissues" refers to any tissue that is affected by the defects in or lower than desired activity from protein subunits, or globin chains, that make up hemoglobin, especially in the liver, spleen and bone marrow. In some embodiments, target tissues include those tissues in which there is an abnormality in the expression of the globin chains, e.g., alpha, beta or gamma. In some embodiments, target tissues include those tissues that display disease-associated pathology, symptom, or feature. As used herein, a target tissue may be a liver target tissue, a spleen target tissue and/or a bone marrow target tissue. Exemplary target tissues are described in detail below.

*Therapeutically effective amount:* As used herein, the term "therapeutically effective amount" refers to an amount of a therapeutic agent which confers a therapeutic effect on the treated subject, at a reasonable benefit/risk ratio applicable to any medical treatment. The therapeutic effect may be objective (*i.e.,* measurable by some test or marker) or subjective (*i.e.,* subject gives an indication of or feels an effect). In particular, the "therapeutically effective amount" refers to an amount of a therapeutic agent or composition effective to treat, ameliorate, or prevent a desired disease or condition, or to exhibit a detectable therapeutic or preventative effect, such as by ameliorating symptoms associated with the disease, preventing or delaying the onset of the disease, and/or also lessening the severity or frequency of symptoms of the disease. A therapeutically effective amount is commonly administered in a dosing regimen that may comprise multiple unit doses. For any particular therapeutic agent, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific agent employed; the duration of the treatment; and like factors as is well known in the medical arts.

*Treatment*: As used herein, the term "treatment" (also "treat" or "treating") refers to any administration of a therapeutic agent (*e.g*., oligonucleotide) that partially or completely alleviates, ameliorates, relieves, inhibits, delays onset of, reduces severity of and/or reduces incidence of one or more symptoms or features of a particular disease, disorder, and/or condition (e.g., hemoglobin dysfunction or deficiency, sickle cell disease, thalassemia). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. Exemplary signs of a relevant disease as described herein include anemia, which may range from moderate to serve depending on the patient who manifests the signs.

### DETAILED DESCRIPTION

The present invention provides, among other things, improved compositions and methods for modulating B-cell CLL/Lymphoma 11A (BCL11A) activity and for treatment of a disease, disorder or condition associated with BCL11A. It is contemplated that reducing or inhibiting BCL11A activity results in increased expression of globin genes, e.g., gamma globin. Therefore, the present invention is particularly useful for treating hemoglobinopathies, such as sickle cell disease and β-thalassemias. In particular, the present invention is based on antisense oligonucleotide modulators of BCL11A that reduce or inhibit BCL11A activity by down-regulating or decreasing expression of BCL11A. In some embodiments, an oligonucleotide capable of down-regulating or decreasing the expression of the human BCL11A gene target a region of the human BCL11A gene or an messenger RNA (mRNA) isoform of BCL11A (e.g., XL, L, M, S or XS). In some embodiments, an oligonucleotide capable of down-regulating or decreasing the expression of the human BCL11A gene has a sequence based on the reverse complement of a continuous sequence of the human BCL11A gene or an messenger RNA (mRNA) isoform of BCL11A.

Various aspects of the invention are described in detail in the following sections. The use of sections is not meant to limit the invention. Each section can apply to any aspect of the invention. In this application, the use of "or" means "and/or" unless stated otherwise.

### BCL11A and Related Diseases and Conditions

The human BCL11A gene encodes a C₂H₂ zinc finger protein having similarity to the mouse BCL11A protein. BCL11A is a lymphoid transcription factor that functions in B cells, and, up until recently, was unknown to have a role in erythropoiesis. BCL11A is now understood to have a role in globin gene regulation and expression appears to correlate with developmental expression of globin genes. BCL11A is expressed in adult erythroid precursor cells in the bone marrow and functions in an inverse relationship with gamma globin genes, *i.e.,* BCL11A functions as a repressor of gamma globin production.

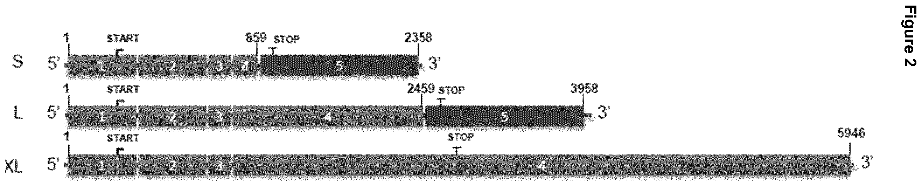

BCL11A is represented in several isoforms. Figure 2 sets forth three major isoforms of BCL11A, which differ in the usage of two potential 3' terminal exons. BCL11A is known to associate with other proteins to form complexes that function to regulate the fetal-to-adult hemoglobin switch. BCL11A is implicated in disease associated with hemoglobin dysfunction. In particular, inhibition of BCL11A upregulates gamma globin expression and, as a result, production of fetal hemoglobin, which can compensate for globin gene dysfunction encountered in hemoglobinopathies, such as sickle cell disease, β-thalassemias, and the like.

### Modulators of BCL11A

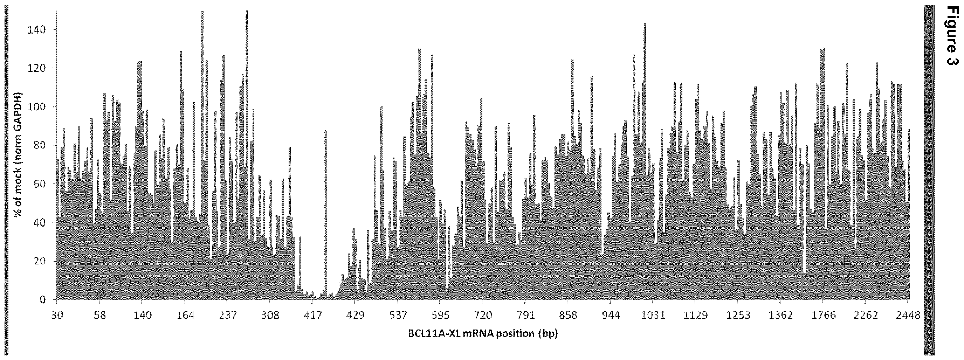

As discussed in the Examples below, the present inventors have successfully identified antisense oligonucleotide modulators that target one or more isoforms of BCL11A. In some embodiments, modulators according to the present invention target a region common to the three major isoforms depicted in Figure 2. Specifically the present inventors have identified a specific region within Exon 2 from nucleotides 410 to 450 of the human BCL11A gene that very efficiently downregulates BCL11A. The corresponding region in the mouse BCL11A gene (e.g., XL, L or S) range from nucleotides 517 to 557. Figure 3 clearly shows that across Exons 1, 2, 3 and 4, this region is a hotspot in terms of designing single stranded oligonucleotides capable of decreasing the expression of BCL11A. The knowledge of such a hotspot increases the likelihood of success in designing an oligonucleotide with good potency and which is well tolerated by the subject to be treated.

### Design of antisense oligonucleotides

Among other things, the present invention provides antisense oligonucleotides useful for modulation of nucleic acid molecules encoding human BCL11A. In particular, an antisense oligonucleotide suitable for the present invention includes any oligonucleotide that is capable of down-regulating or decreasing, reducing or inhibiting BCL11A expression or activity.

Typically, an oligonucleotide capable of down-regulating or decreasing the expression of the human BCL11A gene may be designed based on the sequence of the human BCL11A gene or an messenger RNA (mRNA) isoform of BCL11A (e.g., XL, L or S). For example, an oligonucleotide capable of down-regulating or decreasing the expression of the human BCL11A gene may have a sequence that is substantially identical to the reverse complement of a continuous sequence of the human BCL11A gene or an messenger RNA (mRNA) isoform of BCL11A. In some embodiments, an oligonucleotide according to the present invention has a sequence at least about 50% (e.g., at least about 55%, 60%, 65%, 70%, 75%, 80%, 85%, 90%, 95%, 96%, 97%, 98%, or 99%) identical to the reverse complement of a continuous sequence of the human BCL11A gene or an messenger RNA (mRNA) isoform of BCL11A. Since the human BCL11A gene and mouse BCL11A gene share high sequence identity, an oligonucleotide according to the present invention may also be designed based on the sequence of the mouse BCL11A gene or an messenger RNA (mRNA) isoform of BCL11A. In some embodiments, an oligonucleotide according to the present invention has a sequence at least about 50% (e.g., at least about 55%, 60%, 65%, 70%, 75%, 80%, 85%, 90%, 95%, 96%, 97%, 98%, or 99%) identical to the reverse complement of a continuous sequence of the mouse BCL11A gene or an messenger RNA (mRNA) isoform of BCL11A.

Alternatively, an oligonucleotide capable of down-regulating or decreasing the expression of the human BCL11A gene is capable of hybridizing or binding to a target region of one or more isoforms of BCL11A mRNA. In some embodiments, an oligonucleotide capable of decreasing the expression of the human BCL11A gene is capable of hybridizing or binding to a target region of BCL11A mRNA that is found in an exon (e.g., exon 1, exon 2, exon 3, exon 4, or exon 5). In some embodiments, an oligonucleotide capable of decreasing the expression of the human BCL11A gene is capable of hybridizing or binding to a target region of human or mouse BCL11A.

It will be appreciated that hybridization of an antisense oligonucleotide to a target region of BCL11A mRNA may be performed *in vitro* or *in vivo.* Hybridization may be performed under low, medium, and/or stringent hybridization conditions, as is well known in the art. In general, stringent hybridization conditions refer to standard hybridization conditions under which nucleic acid molecules, including oligonucleotides, are used to identify molecules having complementary nucleic acid sequences. Stringent hybridization conditions typically permit binding between nucleic acid molecules having at least about 50%, 55%, 60%, 65%, 70%, 75%, 80%, 85%, 90%, 95%, or more nucleic acid sequence identity. Standard conditions are disclosed, for example, in Sambrook et al., 1989, Molecular Cloning: A Laboratory Manual, Cold Spring Harbor Labs Press. Formulae to calculate the appropriate hybridization and wash conditions to achieve hybridization permitting 50%, 40%, 30%, 20%, 10%, 5% or less mismatch of nucleotides are available in the art, for example, in Meinkoth et al., 1984, Anal. Biochem. 138, 267-284. It will be appreciated that hybrids between oligonucleotides (14-20 bp) and immobilized DNA show decreased stability and should be taken into account when defining optimal conditions for their hybridization.

Hybridization condition stringency can be affected by buffer ionic strength, base composition of the nucleotide, the length of the shortest chain in the duplex (n), and the concentration of helix destabilizing agents such as formamide. For example, hybridization stringency can be altered by adjusting the salt and/or formamide concentrations and/or by changing the temperature. The stringency can be adjusted either during the hybridization step, or in post hybridization washes. An example of stringent hybridization conditions for hybridization of complementary nucleic acids which have more than 100 complementary residues on a filter in a Southern or Northern blot is 50% formamide with 1 mg of heparin at 42°C, with the hybridization being carried out overnight. An example of stringent wash conditions is a 0.2X SSC wash at 65°C. for 15 minutes. In some embodiments, a high stringency wash is preceded by a low stringency wash to remove back-ground probe signal. An example medium stringency wash for a duplex of, e.g., more than 100 nucleotides, is 100X SSC at 45°C for 15 minutes. An example low stringency wash for a duplex of, e.g., more than 100 nucleotides, is 4X SSC at 40°C. for 15 minutes. In general, a signal to noise ratio of 2X (or higher) than that observed for an unrelated probe in the particular hybridization assay indicates detection of a specific hybridization.

### Sequences of BCL11A mRNA Isoforms

As described above, Figure 2 sets forth the three major human BCL11A mRNA isoforms, i.e., isoform XL, L and S. Similarly, there three major mouse BCL11A mRNA isoforms, i.e., isoform XL, L and S. For both mouse and human, other BCL11A mRNA isoforms have been identified. For example, in humans several isoforms based on alternative splice variants are described in the Ensembl genebuild assemblies (European Bioinformatics Institute and Wellcome Trust Sanger Institute), which are identified by the following transcript identification numbers: ENST00000358510, ENST00000538214, ENST00000537768, ENST00000477659, ENST00000489516, ENST00000409351, ENST00000479026, ENST00000492272, ENST00000489183. Sequences of exemplary human and mouse isoforms of BCL11A are set forth in the sequence list with indication of exons:
SEQ ID NO: 1 = Human BCL11A-XL NCBI accession number NM_022893
SEQ ID NO: 2 = Human BCL11A-L NCBI accession number NM_018014
SEQ ID NO: 3 = Human BCL11A-S NCBI accession number NM_138559
SEQ ID NO: 4 = Mouse BCL11A-XL NCBI accession number NM_001242934
SEQ ID NO: 5 = Mouse BCL11A-L NCBI accession number N_016707
SEQ ID NO: 6 = Mouse BCL11A-S L NCBI accession number NM_001159289
SEQ ID NO: 7= Mouse BCL11A-XS NCBI accession number NM_001159290

In some embodiments, provided antisense oligonucleotides bind to a region within one or more isoforms of a human or mouse BCL11A as shown in SEQ ID NO: 1 to 7. In some embodiments, provided antisense oligonucleotides bind to a region within an exon of a human or mouse BCL11A isoform as shown in SEQ ID NO: 1 to 7. In some embodiments, provided antisense oligonucleotides bind to a region within an exon of an isoform of human BCL11A, mouse BCL11A, or a combination thereof. In some embodiments, provided antisense oligonucleotides bind to a region within nucleotides 1-283, 284 - 613, or 614 - 715 of a human BCL11A. Preferably, nucleotides 1-283, 284 - 613, or 614 - 715 of SEQ ID NO: 1. In some embodiments, provided antisense oligonucleotides bind to a region within nucleotides 250 - 500, 259 - 438, 284 - 613, 415 - 445, 415 - 436, 716 - 5946, 716 - 2458, 2459 - 3958, or nucleotides 859 - 2358 of a human BCL11A. In various embodiments, a human BCL11A is selected from isoforms XL, L or S as shown in SEQ ID NO: 1 to 3. In various embodiments, a mouse BCL11A is selected from isoforms XL, L or S as shown in SEQ ID NO: 4 to 7.

In some embodiments, an antisense oligonucleotide of the present invention has a sequence that is at least 50%, at least 55%, at least 60%, at least 65%, at least 70%, at least 75%, at least 80%, at least 85%, at least 90%, at least 95%, at least 97%, at least 98%, or at least 99% identical to the reverse complement of a continuous sequence of a human or mouse BCL11A gene or a messenger RNA (mRNA) isoform of a human or mouse BCL11A. In some embodiments, an oligonucleotide of the present invention has a sequence that is identical to the reverse complement of a continuous sequence of the human or mouse BCL11A gene or an messenger RNA (mRNA) isoform of human or mouse BCL11A.

In some embodiments, a continuous sequence according to the present invention is within a region selected from nucleotides 1-283 (Exon 1), nucleotides 284 - 613 (Exon 2), or nucleotides 614 - 715 (Exon 3) of the human BCL11A gene.

In some embodiments, a continuous sequence according to the present invention is within nucleotides of a human BCL11A mRNA isoform XL (SEQ ID NO: 1). In some embodiments, a continuous sequence according to the present invention is within nucleotides 200 - 620, 410 - 450, 415 - 436, 415 - 446, 420 - 450, or within nucleotides 716 - 5946 (exon 4) of a human BCL11A mRNA isoform XL (SEQ ID NO: 1).

In some embodiments, a continuous sequence according to the present invention is within nucleotides of a human BCL11A mRNA isoform L (SEQ ID NO: 2). In some embodiments, a continuous sequence according to the present invention is within nucleotides 716 - 2458 (exon 4) or nucleotides 2459 - 3958 (exon 5) of a human BCL11A mRNA isoform L.

In some embodiments, a continuous sequence according to the present invention is within nucleotides of a human BCL11A mRNA isoform S (SEQ ID NO: 3). In some embodiments, a continuous sequence according to the present invention is within nucleotides 716 - 858 (exon 4) or nucleotides 859 - 2358 (exon 5) of a human BCL11A mRNA isoform S.

In some embodiments, provided antisense oligonucleotides bind to a target region that is substantially identical to the corresponding region of the human or mouse BCL11A as shown in SEQ ID NO: 1 to 7. For example, provided antisense oligonucleotides may bind to a target region that has a sequence at least 50%, 55%, 60%, 65%, 70%, 75%, 80%, 85%, 90%, 91%, 92%, 93%, 94%, 95%, 96%, 97%, 98%, 99% or more identical to that of the corresponding region (e.g., exon 1, 2, 3, 4, or 5) of the human or mouse BCL11A as shown in SEQ ID NO: 1 to 7. Exemplary regions are described throughout the specification.

### The Oligonucleotide

The term "oligonucleotide" in the context of the present invention, refers to a molecule formed by covalent linkage of two or more nucleotides. The term is used interchangeably with the term oligomer. Herein, a single nucleotide (unit) may also be referred to as a monomer or unit. In some embodiments, the terms "nucleoside", "nucleotide", "unit" and "monomer" are used interchangeably. It will be recognized that when referring to a sequence of nucleotides or monomers, what is referred to is the sequence of bases, such as A, T, G, C or U.

The oligonucleotide of the invention is capable of decreasing expression of human BCL11A comprising a sequence that is at least 80% identical to the reverse complement of a continuous sequence within a region selected from nucleotides 410 to 450 of the human BCL11A gene or an messenger RNA (mRNA) isoform of BCL11A.

In some embodiments, the oligonucleotide of the invention comprises or consists a sequence motif selected from the group shown in Table 1. Sequence motifs are essentially a nucleotide sequence that can be used as the basis for generating oligonucleotides that essentially comprise or contain the same sequence but varies for example in the number of nucleotide analouges, length or internucleotide linkages.

**Table 1. Sequence motifs that can be used to design specific oligonucleotides.**

| Sequence (5'-3') | |
|---|---|
| ATTGCATTGTTTCCG | SEQ ID NO: 63 |
| GTTTGTGCTCGAT | SEQ ID NO: 64 |
| CATTGCATTGTTTCCG | SEQ ID NO: 65 |
| CGTTTGTGCTCGAT | SEQ ID NO: 66 |
| CGTTTGTGCTCGATAA | SEQ ID NO: 67 |
| CCGTTTGTGCTCGA | SEQ ID NO: 68 |
| CGTTTGTGCTCGA | SEQ ID NO: 69 |
| TTTGTGCTCGATAA | SEQ ID NO: 70 |
| TTGTGCTCCATAA | SEQ ID NO: 71 |
| TTTCCGTTTGTGCTCG | SEQ ID NO: 72 |
| ATTGCATTGTTTCCGT | SEQ ID NO: 73 |
| CGTTTGTGCTCGATA | SEQ ID NO: 74 |

In some embodiments, the oligonucleotide sequence motif is not TCCGTTTGTGCTCGATAAA (SEQ ID NO: 75) or not TTTGTGCTCGATAAAAATA (SEQ ID NO: 76), or not ATTGTTTCCGTTTGTGCTC (SEQ ID NO: 77).

In preferred embodiments, the oligonucleotide of the invention comprises or is a gapmer.

In some embodiments, the oligonucleotide is less than 19 nucleotides in length, preferably less than 18, more preferably less than 17 nucleotides in length.

In some embodiments, the oligonucleotide of the invention comprises affinity enhancing nucleotide analogues.

In some embodiments, the nucleotide analogues are sugar modified nucleotides, such as sugar modified nucleotides independently or dependently selected from the group consisting of: 2'-O-alkyl-RNA units, 2'-OMe-RNA units, 2'-O-alkyl-DNA, 2'-amino-DNA units, 2'-fluoro-DNA units, LNA units, arabino nucleic acid (ANA) units, 2'-fluoro-ANA units, HNA units, INA units and 2'MOE units.

In some embodiments, the nucleotide analogues comprise or consist of Locked Nucleic Acid (LNA) units.

In preferred embodiments, the oligomer is a single stranded molecule. In some embodiments, the oligonucleotide does not comprise short regions of, for example, at least 3, 4 or 5 contiguous nucleotides, which are complementary to equivalent regions within the same oligonucleotide (*i.e.* duplexes or hairpins). The oligonucleotide, in some embodiments, may be not (essentially) double stranded. In some embodiments, the oligonucleotide is essentially not double stranded, such as is not a siRNA.

### Exemplary Antisense Oligonucleotides

Exemplary antisense oligonucleotides of the present invention are listed in Table 2.

**TABLE 2**

| Oligo # | Sequence (5'-3') | |
|---|---|---|
| 1 | **^{m}Cₛ^{o}Tₛ^{o}Aₛ^{o}**tₛgₛtₛgₛtₛtₛcₛcₛ**Tₛ^{o}Gₛ^{o}T^{o}** | SEQ ID NO: 8 |
| 2 | **Gₛ^{o}Aₛ^{o}Gₛ^{o}**aₛcₛaₛtₛgₛgₛtₛgₛgₛgₛ**^{m}Cₛ^{o}Tₛ^{o}G^{o}** | SEQ ID NO: 9 |
| 3 | **Aₛ^{o}Tₛ^{o}Tₛ^{o}**gₛcₛaₛtₛtₛgₛtₛtₛtₛcₛ**^{m}Cₛ^{o}Gₛ^{o}T^{o}** | SEQ ID NO: 10 |
| 4 | **^{m}Cₛ^{o}Aₛ^{o}T**ₛ^{o}tₛgₛcₛaₛtₛtₛgₛtₛtₛtₛ**^{m}Cₛ^{om}Cₛ^{o}G^{o}** | SEQ ID NO: 11 |
| 5 | **Aₛ^{o}Tₛ^{o}Tₛ^{o}**gₛ^{m}cₛaₛtₛtₛgₛtₛtₛtₛ**^{m}Cₛ^{om}Cₛ^{o}G^{o}** | SEQ ID NO: 12 |
| 6 | **Aₛ^{o}Tₛ^{o}Tₛ^{o}**gₛcₛaₛtₛtₛgₛtₛtₛtₛ**^{m}Cₛ^{om}Cₛ^{o}G^{o}** | SEQ ID NO: 13 |
| 7 | **Tₛ^{o}Tₛ^{o}Gₛ^{o}**tₛgₛcₛtₛ^{m}cₛgₛaₛtₛ**Aₛ^{o}Aₛ^{o}A^{o}** | SEQ ID NO: 14 |
| 8 | **^{m}Cₛ^{o}Gₛ^{o}Tₛ^{o}**tₛtₛgₛtₛgₛcₛtₛ^{m}cₛgₛaₛ**Tₛ^{o}Aₛ^{o}A^{o}** | SEQ ID NO: 15 |
| 9 | **^{m}Cₛ^{om}Cₛ^{o}Gₛ^{o}**tₛtₛtₛgₛtₛgₛcₛtₛ**^{m}Cₛ^{o}Gₛ^{o}A^{o}** | SEQ ID NO: 16 |
| 10 | **Tₛ^{o}Tₛ^{o}**gₛtₛgₛcₛtₛ^{m}cₛcₛaₛ**Tₛ^{o}Aₛ^{o}A^{o}** | SEQ ID NO: 17 |
| 11 | **Tₛ^{o}Tₛ^{o}Tₛ^{o}**cₛ^{m}cₛgₛtₛtₛtₛgₛtₛgₛcₛ**Tₛ^{om}Cₛ^{o}G^{o}** | SEQ ID NO: 18 |
| 12 | **^{m}Cₛ^{o}Aₛ^{o}Tₛ^{o}**tₛgₛ^{m}cₛaₛtₛtₛgₛtₛtₛtₛ**^{m}Cₛ^{om}Cₛ^{o}G^{o}** | SEQ ID NO: 19 |
| 13 | **^{m}Cₛ^{o}Aₛ^{o}**tₛ^{o}tₛgₛcₛaₛtₛtₛgₛtₛtₛtₛ**^{m}Cₛ^{om}Cₛ^{o}G^{o}** | SEQ I D NO: 20 |
| 14 | **^{m}Cₛ^{o}Aₛ^{o}Tₛ^{o}**tₛgₛcₛaₛtₛtₛgₛtₛtₛ**Tₛ^{om}Cₛ^{om}Cₛ^{o}G^{o}** | SEQ ID NO: 21 |
| 15 | **^{m}Cₛ^{o}Aₛ^{o}Tₛ^{o}Tₛ^{o}**gₛcₛaₛtₛtₛgₛtₛtₛtₛ**^{m}Cₛ^{om}Cₛ^{o}G^{o}** | SEQ ID NO: 22 |
| 16 | **^{m}Cₛ^{o}Aₛ^{o}Tₛ^{o}Tₛ^{o}**gₛcₛaₛtₛtₛgₛtₛtₛ**Tₛ^{om}Cₛ^{om}Cₛ^{o}G^{o}** | SEQ ID NO: 23 |
| 17 | **^{m}Cₛ^{o}Aₛ^{o}Tₛ^{o}**tₛgₛcₛaₛtₛtₛgₛtₛ**Tₛ^{o}Tₛ^{om}Cₛ^{om}Cₛ^{o}G^{o}** | SEQ ID NO: 24 |
| 18 | **^{m}Cₛ^{o}Aₛ^{o}Tₛ^{o}Tₛ^{o}Gₛ^{o}**cₛaₛtₛtₛgₛtₛtₛtₛ**^{m}Cₛ^{om}Cₛ^{o}G^{o}** | SEQ ID NO: 25 |
| 19 | **Gₛ^{o}Tₛ^{o}Tₛ^{o}**tₛgₛtₛgₛcₛtₛ^{m}cₛgₛaₛtₛ**Aₛ^{o}Aₛ^{o}A^{o}** | SEQ ID NO: 26 |
| 20 | **Tₛ^{o}Tₛ^{o}Tₛ^{o}**gₛtₛgₛcₛtₛ^{m}cₛgₛaₛtₛ**Aₛ^{o}Aₛ^{o}A^{o}** | SEQ ID NO: 27 |
| 21 | **Tₛ^{o}Tₛ^{o}Tₛ^{o}**gₛtₛgₛcₛtₛ^{m}cₛgₛaₛ**Tₛ^{o}Aₛ^{o}Aₛ^{o}** | SEQ ID NO: 28 |
| 22 | **Gₛ^{o}Tₛ^{o}Tₛ^{o}**tₛgₛtₛgₛcₛtₛ^{m}cₛgₛaₛ**Tₛ^{o}Aₛ^{o}Aₛ^{o}** | SEQ ID NO: 29 |
| 23 | **Gₛ^{o}Tₛ^{o}Tₛ^{o}**tₛgₛtₛgₛcₛtₛ^{m}cₛgₛ**Aₛ^{o}Tₛ^{o}Aₛ^{o}** | SEQ ID NO: 30 |
| 24 | **^{m}Cₛ^{om}Cₛ^{o}Gₛ^{o}**tₛtₛtₛgₛtₛgₛcₛtₛ^{m}cₛgₛ**Aₛ^{o}Tₛ^{o}A^{o}** | SEQ ID NO: 31 |
| 25 | **^{m}Cₛ^{o}Gₛ^{o}Tₛ^{o}**tₛtₛgₛtₛgₛcₛtₛ^{m}cₛgₛ**Aₛ^{o}Tₛ^{o}A^{o}** | SEQ ID NO: 32 |
| 26 | **Gₛ^{o}Tₛ^{o}**tₛtₛgₛtₛgₛcₛtₛcₛ**Gₛ^{o}Aₛ^{o}T^{o}** | SEQ ID NO: 33 |
| 27 | **^{m}Cₛ^{o}Gₛ^{o}Tₛ^{o}**tₛtₛgₛtₛgₛcₛtₛcₛ**Gₛ^{o}Aₛ^{o}T^{o}** | SEQ ID NO: 34 |
| 28 | **^{m}Cₛ^{om}Cₛ^{o}Gₛ^{o}**tₛtₛtₛgₛtₛgₛcₛtₛcₛ**Gₛ^{o}Aₛ^{o}T^{o}** | SEQ ID NO: 35 |
| 29 | **Tₛ^{om}Cₛ^{om}Cₛ^{o}**gₛtₛtₛtₛgₛtₛgₛcₛtₛcₛ**Gₛ^{o}Aₛ^{o}T^{o}** | SEQ ID NO: 36 |
| 30 | **^{m}Cₛ^{o}Gₛ^{o}**tₛtₛtₛgₛtₛgₛcₛtₛ**^{m}Cₛ^{o}Gₛ^{o}A^{o}** | SEQ ID NO: 37 |
| 31 | **Tₛ^{o}Tₛ^{om}Cₛ**^{**o**m}cₛgₛtₛtₛtₛgₛtₛgₛcₛtₛ**^{m}Cₛ^{o}Gₛ^{o}A^{o}** | SEQ ID NO: 38 |
| 32 | **Tₛ^{om}Cₛ^{om}Cₛ^{o}**gₛtₛtₛtₛgₛtₛgₛcₛtₛ**^{m}Cₛ^{o}Gₛ^{o}A^{o}** | SEO ID NO: 39 |
| 33 | **Tₛ^{om}Cₛ^{om}Cₛ^{o}**gₛtₛtₛtₛgₛtₛgₛcₛ**Tₛ^{om}Cₛ^{o}G^{o}** | SEQ ID NO: 40 |
| 34 | **Tₛ^{o}Tₛ^{om}Cₛ**^{**o**m}cₛgₛtₛtₛtₛgₛtₛgₛcₛ**Tₛ^{om}Cₛ^{o}G^{o}** | SEQ ID NO: 41 |
| 35 | **Gₛ^{o}Tₛ^{o}Tₛ^{o}**tₛcₛ^{m}cₛgₛtₛtₛtₛgₛtₛgₛ**^{m}Cₛ^{o}Tₛ^{om}C^{o}** | SEQ ID NO: 42 |
| 36 | **Tₛ^{o}Tₛ^{o}Tₛ^{o}**cₛ^{m}cₛgₛtₛtₛtₛgₛtₛgₛ**^{m}Cₛ^{o}Tₛ^{om}C^{o}** | SEQ ID NO: 43 |
| 37 | **^{m}Cₛ^{o}Gₛ^{o}**tₛtₛtₛgₛtₛgₛcₛtₛ^{m}cₛgₛaₛ**Tₛ^{o}Aₛ^{o}A^{o}** | SEQ ID NO: 44 |
| 38 | **^{m}Cₛ^{o}Gₛ^{o}Tₛ^{o}**tₛtₛgₛtₛgₛcₛtₛ^{m}cₛgₛ**Aₛ^{o}Tₛ^{o}Aₛ^{o}A^{o}** | SEQ ID NO: 45 |
| 39 | **^{m}Cₛ^{o}Gₛ^{o}Tₛ^{o}Tₛ^{o}**tₛgₛtₛgₛcₛtₛ^{m}cₛgₛaₛ**Tₛ^{o}Aₛ^{o}A^{o}** | SEQ ID NO: 46 |
| 40 | **^{m}Cₛ^{o}Gₛ^{o}Tₛ^{o}Tₛ^{o}**tₛgₛtₛgₛcₛtₛ^{m}cₛgₛ**Aₛ^{o}Tₛ^{o}Aₛ^{o}A^{o}** | SEQ ID NO: 47 |
| 41 | **^{m}Cₛ^{o}Gₛ^{o}Tₛ^{o}**tₛtₛgₛtₛgₛcₛtₛ^{m}cₛ**Gₛ^{o}Aₛ^{o}Tₛ^{o}Aₛ^{o}A^{o}** | SEQ ID NO: 48 |
| 42 | **^{m}Cₛ^{o}Gₛ^{o}Tₛ^{o}Tₛ^{o}Tₛ^{o}**gₛtₛgₛcₛtₛ^{m}cₛgₛaₛ**Tₛ^{o}Aₛ^{o}A^{o}** | SEQ ID NO: 49 |
| 43 | **^{m}Cₛ^{o}Gₛ^{o}**tₛtₛtₛgₛtₛgₛcₛtₛ^{m}cₛgₛ**Aₛ^{o}Tₛ^{o}A^{o}** | SEQ ID NO: 50 |
| 44 | **^{m}Cₛ^{o}Gₛ^{o}Tₛ^{o}**tₛtₛgₛtₛgₛcₛtₛ^{m}cₛgₛaₛ**Tₛ^{o}A^{o}** | SEQ ID NO: 51 |
| 45 | **^{m}Cₛ^{o}Gₛ^{o}Tₛ^{o}Tₛ^{o}**tₛgₛtₛgₛcₛtₛ^{m}cₛgₛaₛ**Tₛ^{o}A^{o}** | SEQ ID NO: 52 |
| 46 | **^{m}Cₛ^{o}Gₛ^{o}**tₛtₛtₛgₛtₛgₛcₛtₛ^{m}cₛ**Gₛ^{o}Aₛ^{o}Tₛ^{o}A^{o}** | SEQ ID NO: 53 |
| 47 | **^{m}Cₛ^{o}Gₛ^{o}Tₛ^{o}Tₛ^{o}**tₛgₛtₛgₛcₛtₛ^{m}cₛgₛ**Aₛ^{o}Tₛ^{o}A^{o}** | SEQ ID NO: 54 |
| 48 | **^{m}Cₛ^{o}Gₛ^{o}Tₛ^{o}**tₛtₛgₛtₛgₛcₛtₛ^{m}cₛ**Gₛ^{o}A^{o}Tₛ^{o}A^{o}** | SEQ ID NO: 55 |
| 49 | **^{m}Cₛ^{o}Gₛ^{o}Tₛ^{o}**tₛtₛgₛtₛgₛcₛtₛ^{m}cₛ**Gₛ^{o}Aₛ^{o}T^{o}** | SEQ ID NO: 56 |
| 50 | **^{m}Cₛ^{o}Gₛ^{o}**tₛtₛtₛgₛtₛgₛcₛtₛ^{m}cₛgₛ**Aₛ^{o}T^{o}** | SEQ ID NO: 57 |
| 51 | **^{m}Cₛ^{o}Gₛ^{o}**tₛ^{o}tₛtₛgₛtₛgₛcₛtₛcₛ**Gₛ^{o}Aₛ^{o}T^{o}** | SEQ ID NO: 58 |
| 52 | **^{m}Cₛ^{o}Gₛ^{o}Tₛ^{o}**tₛtₛgₛtₛgₛcₛtₛ^{m}cₛgₛ**Aₛ^{o}T^{o}** | SEQ ID NO: 59 |
| 53 | **^{m}Cₛ^{o}Gₛ^{o}Tₛ^{o}Tₛ^{o}**tₛgₛtₛgₛcₛtₛ^{m}cₛgₛ**Aₛ^{o}T^{o}** | SEQ ID NO: 60 |
| 54 | **^{m}Cₛ^{o}Gₛ^{o}**tₛtₛtₛgₛtₛgₛcₛtₛ**^{m}Cₛ^{o}Gₛ^{o}Aₛ^{o}T^{o}** | SEQ ID NO: 61 |
| 55 | **^{m}Cₛ^{o}Gₛ^{o}Tₛ^{o}Tₛ^{o}**tₛgₛtₛgₛcₛtₛ**^{m}Cₛ^{o}Gₛ^{o}Aₛ^{o}T^{o}** | SEQ ID NO: 62 |

In various embodiments, antisense oligonucleotides according to the present invention include those oligonucleotides having a sequence at least 50% (*e.g*., 50%, 55%, 60%, 65%, 70%, 75%, 80%, 85%, 90%, 91%, 92%, 93%, 94%, 95%, 96%, 97%, 98%, 99% or more) identical to 12 or more (e.g., 13, 14, 15, 16, 17, or 18) contiguous nucleotides that appear in an antisense oligonucleotide sequence selected from Table 2.

In various embodiments, antisense oligonucleotides according to the present invention include those oligonucleotides having a sequence at least 50% (*e.g*., 50%, 55%, 60%, 65%, 70%, 75%, 80%, 85%, 90%, 91%, 92%, 93%, 94%, 95%, 96%, 97%, 98%, 99% or more) identical to the nucleotide sequence of an antisense oligonucleotide selected from Table 2.

### Length

In will be appreciated that an antisense oligonucleotide in accordance with the present invention may be of any appropriate length. An antisense oligonucleotide of the present invention may comprise or consist of a contiguous nucleotide sequence of a total of 10, 11, 12, 13, 14, 15, 16, 17, 18, 19, 20, 21, 22, 23, 24, 25, 26, 27, 28, 29 or 30 contiguous nucleotides in length. In some embodiments, an antisense oligonucleotide comprises or consists of a contiguous nucleotide sequence of a total of 10-18, 10-17, 10-16, 10-15, 10-14, 10-13, 10-12, 11-17, 11-16, 11-15, 11-14, 11-13, 12-17, 12-16, 12-15, or 12-14 nucleotides in length. In some embodiments, an antisense oligonucleotide of the present invention is 10 - 16 or 12 - 16 nucleotides in length In some embodiments, an antisense oligonucleotide of the present invention consists of no more than 22 nucleotides, such as no more than 20 nucleotides, such as no more than 19 nucleotides, such as 15, 16, 17 or 18 nucleotides. In some embodiments, an antisense oligonucleotide of the present invention comprises less than 20 nucleotides In some embodiments, an antisense oligonucleotide of the present invention is less than 18 nucleotides in length. Without wishing to be bound by theory, it should be understood that when a range is given for an antisense oligonucleotide of the present invention, or contiguous nucleotide sequence length, it includes the lower an upper lengths provided in the range, for example from (or between) 10 - 30, includes both 10 and 30.

"Percent (%) nucleic acid sequence identity" with respect to the nucleotide sequences identified herein is defined as the percentage of nucleotides in a candidate sequence that are identical with the nucleotides in the reference sequence, after aligning the sequences and introducing gaps, if necessary, to achieve the maximum percent sequence identity. The percentage sequence identity may be calculated by counting the number of aligned nucleic acid that are identical between the 2 sequences, dividing by the total number of monomers in the oligomer, and multiplying by 100. In such a comparison, if gaps exist, it is preferable that such gaps are merely mismatches rather than an area where a number of nucleic acid within the gap differs between the aligned sequences, e.g. between the oligonucleotide of the invention and the target region. Alignment for purposes of determining percent nucleic acid sequence identity can be achieved in various ways that are within the skill in the art, for instance, using publicly available computer software such as BLAST, ALIGN or Megalign (DNASTAR) software as well as EMBOSS needel for global alignments or EMBOSS Water for local alignments. Those skilled in the art can determine appropriate parameters for measuring alignment, including any algorithms needed to achieve maximal alignment over the full length of the sequences being compared. Preferably, the WU-BLAST-2 software is used to determine amino acid sequence identity (Altschul et al., Methods in Enzymology, 266, 460-480 (1996); http://blast.wustl/edu/blast/README.html). WU-BLAST-2 uses several search parameters, most of which are set to the default values. The adjustable parameters are set with the following values: overlap span=1, overlap fraction=0.125, world threshold (T)=11. HSP score (S) and HSP S2 parameters are dynamic values and are established by the program itself, depending upon the composition of the particular sequence, however, the minimum values may be adjusted and are set as indicated above.

### Nucleosides and Nucleoside analogues

In some embodiments, the terms "nucleoside analogue" and "nucleotide analogue" are used interchangeably.

The term "nucleotide" as used herein, refers to a glycoside comprising a sugar moiety, a base moiety and a covalently linked group (linkage group), such as a phosphate or phosphorothioate internucleotide linkage group, and covers both naturally occurring nucleotides, such as DNA or RNA, and non-naturally occurring nucleotides comprising modified sugar and/or base moieties, which are also referred to as "nucleotide analogues" herein. Herein, a single nucleotide (unit) may also be referred to as a monomer or nucleic acid unit.

In the field of biochemistry, the term "nucleoside" is commonly used to refer to a glycoside comprising a sugar moiety and a base moiety, and may therefore be used when referring to the nucleotide units, which are covalently linked by the internucleotide linkages between the nucleotides of an oligonucleotide. In the field of biotechnology, the term "nucleotide" is often used to refer to a nucleic acid monomer or unit, and as such in the context of an oligonucleotide may refer to the base - such as the "nucleotide sequence", typically refer to the nucleobase sequence (*i.e.* the presence of the sugar backbone and internucleoside linkages are implicit). Likewise, particularly in the case of oligonucleotides where one or more of the internucleoside linkage groups are modified, the term "nucleotide" may refer to a "nucleoside" for example the term "nucleotide" may be used, even when specifying the presence or nature of the linkages between the nucleosides.

As one of ordinary skill in the art would recognise, the 5' terminal nucleotide of an oligonucleotide does not comprise a 5' internucleotide linkage group, although may or may not comprise a 5' terminal group.

Non-naturally occurring nucleotides include nucleotides which have modified sugar moieties, such as bicyclic nucleotides or 2' modified nucleotides, such as 2' substituted nucleotides.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligonucleotide, *i.e.* have no functional effect on the way the oligonucleotide works to inhibit target gene expression. Such "equivalent" analogues may nevertheless be useful if, for example, they are easier or cost effective to manufacture, or are more stable to storage or manufacturing conditions, or represent a tag or label. Preferably, however, the analogues will have a functional effect on the way in which the oligonucleotide works to inhibit expression; for example by producing increased binding affinity to the target and/or increased resistance to intracellular nucleases and/or increased ease of transport into the cell. Specific examples of nucleoside analogues are described by *e.g.* Freier & Altmann; Nucl. Acid Res., 1997, 25, 4429-4443 and Uhlmann; Curr. Opinion in Drug Development, 2000, 3(2), 293-213, and in Scheme 1 and in the section "Locked Nucleic Acid (LNA)"

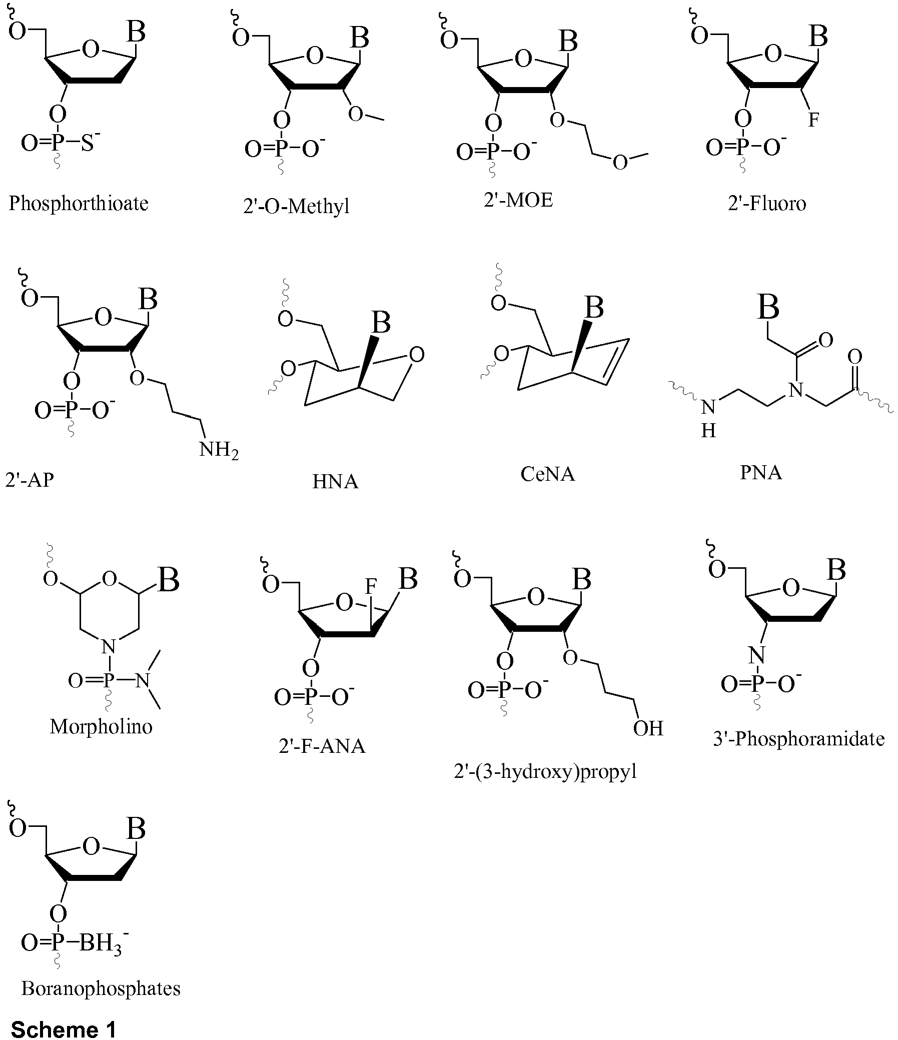

An oligonucleotide may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, *i.e.* nucleotide analogues. Such nucleotide analogues may suitably enhance the affinity of the oligomer for the target sequence. Examples of suitable and preferred nucleotide analogues are provided by WO2007/031091 or are referenced therein.

Incorporation of affinity-enhancing nucleotide analogues in the oligomer, such as LNA or 2'-substituted sugars, can allow the size of the specifically binding oligomer to be reduced, and may also reduce the upper limit to the size of the oligonucleotide before non-specific or aberrant binding takes place.

In some embodiments, an antisense oligonucleotide of the present invention comprises at least 1 nucleoside analogue. In some embodiments, an antisense oligonucleotide of the present invention comprises at least 2 nucleotide analogues. In some embodiments, an antisense oligonucleotide of the present invention comprises from 3-8 nucleotide analogues, *e.g.* 6 or 7 nucleotide analogues. In some embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments, all the nucleotides analogues may be LNA.

It will be recognized by persons of skill upon reading this disclosure that when referring to a preferred nucleotide sequence motif or nucleotide sequence, which consists of only nucleotides, an antisense oligonucleotide of the present invention which are defined by that sequence may comprise a corresponding nucleotide analogue in place of one or more of the nucleotides present in said sequence, such as LNA units or other nucleotide analogues, which raise the duplex stability/Tₘ of the oligomer/target duplex (*i.e.* affinity enhancing nucleotide analogues).

In some embodiments, any mismatches between the nucleotide sequence of the oligomer and the target sequence are preferably found in regions outside the affinity enhancing nucleotide analogues, such as region B or Y as referred to in the section "Gapmer Design", and/or region D as referred to in the section "Gapmer Design", and/or at the site of non modified such as DNA nucleotides in the oligonucleotide, and/or in regions which are 5' or 3' to the contiguous nucleotide sequence.

Examples of such modification of the nucleotide include modifying the sugar moiety to provide a 2'-substituent group or to produce a bicyclic structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments, nucleotide analogues present within an antisense oligonucleotide of the present invention (such as in regions A and C mentioned in the section "Gapmer Design") are independently selected from, for example: 2'-O-alkyl-RNA units, 2'-OMe-RNA units, 2'-O-alkyl-DNA, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925,) units and 2'MOE units.

In some embodiments, there is only one of the above types of nucleotide analogues present in an antisense oligonucleotide of the present invention, or contiguous nucleotide sequence thereof.

In some embodiments, nucleotide analogues are 2'-O-methoxyethyl-RNA (2'MOE), 2'-fluoro-DNA monomers or LNA nucleotide analogues, and as such an antisense oligonucleotide of the present invention may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments, at least one of said nucleotide analogues is 2'-fluoro DNA, such as 2, 3, 4, 5, 6, 7, 8, 9 or 10 2'-fluoro-DNA nucleotide units.

In some embodiments, an antisense oligonucleotide of the present invention comprises at least one Locked Nucleic Acid (LNA) unit, such as 1, 2, 3, 4, 5, 6, 7, or 8 LNA units, such as from 3 - 7 or 4 to 8 LNA units, or 3, 4, 5, 6 or 7 LNA units. In some embodiments, all the nucleotide analogues are LNA. In some embodiments, an antisense oligonucleotide of the present invention may comprise both beta-D-oxy-LNA, and one or more of the following LNA units: thio-LNA, amino-LNA, oxy-LNA, 5'-methyl-LNA and/or ENA in either the beta-D or alpha-L configurations or combinations thereof. In some embodiments, all LNA cytosine units are 5'-methyl-Cytosine.

In some embodiments, an antisense oligonucleotide of the present invention may comprise both nucleotide analogues (preferably LNA) and DNA units. Preferably the combined total of nucleotide analogues (preferably LNA) and DNA units is 10-25, such as 10 - 24, preferably 10-20, such as 10 - 18, even more preferably 12-16. In some embodiments, the nucleotide sequence of an antisense oligonucleotide of the present invention, such as the contiguous nucleotide sequence, consists of at least one nucleotide analogues (preferably LNA) and the remaining nucleotide units are DNA units. In some embodiments, an antisense oligonucleotide of the present invention comprises only LNA nucleotide analogues and naturally occurring nucleotides (such as RNA or DNA, most preferably DNA nucleotides), optionally with modified internucleotide linkages such as phosphorothioate.

The term "nucleobase" refers to the base moiety of a nucleotide and covers both naturally occurring a well as non-naturally occurring variants. Thus, "nucleobase" covers not only the known purine and pyrimidine heterocycles but also heterocyclic analogues and tautomeres thereof.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

In some embodiments, at least one of the nucleobases present in the oligomer is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### Locked Nucleic Acid (LNA)

The term "LNA" refers to a bicyclic nucleoside analogue, known as "Locked Nucleic Acid". It may refer to an LNA monomer, or, when used in the context of an "LNA oligonucleotide", LNA refers to an oligonucleotide containing one or more such bicyclic nucleotide analogues. LNA nucleotides are characterised by the presence of a linker group (such as a bridge) between C2' and C4' of the ribose sugar ring - for example as shown as the biradical R^{4*} - R^{2*} as described below. The LNA used in an antisense oligonucleotide of the present invention preferably has the structure of the general formula I: 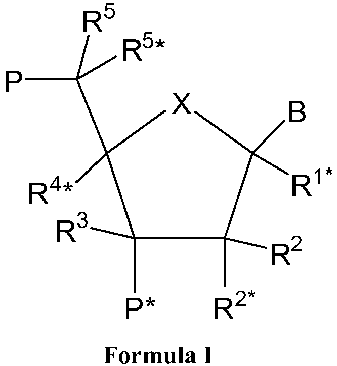
wherein for all chiral centers, asymmetric groups may be found in either R or S orientation;
wherein X is selected from -O-, -S-, -N(R^{N*})-, -C(R⁶R^{6*})-, such as, in some embodiments - O-;
B is selected from hydrogen, optionally substituted C₁₋₄-alkoxy, optionally substituted C₁₋₄-alkyl, optionally substituted C₁₋₄-acyloxy, nucleobases including naturally occurring and nucleobase analogues, DNA intercalators, photochemically active groups, thermochemically active groups, chelating groups, reporter groups, and ligands; preferably, B is a nucleobase or nucleobase analogue;
P designates an internucleotide linkage to an adjacent monomer, or a 5'-terminal group, such internucleotide linkage or 5'-terminal group optionally including the substituent R⁵ or equally applicable the substituent R^{5*};
P* designates an internucleotide linkage to an adjacent monomer, or a 3'-terminal group;
R^{4*} and R^{2*} together designate a bivalent linker group consisting of 1 - 4 groups/atoms selected from -C(R^{a}R^{b})-, -C(R^{a})=C(R^{b})-, -C(R^{a})=N-, -O-, -Si(R^{a})₂-, -S-, -SO₂-, -N(R^{a})-, and >C=Z, wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, optionally substituted C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂), wherein for all chiral centers, asymmetric groups may be found in either R or S orientation, and;
each of the substituents R^{1*}, R², R³, R⁵, R^{5*}, R⁶ and R^{6*}, which are present is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene; ; wherein R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof. For all chiral centers, asymmetric groups may be found in either R or S orientation.

In some embodiments, R^{4*} and R^{2*} together designate a biradical consisting of a groups selected from the group consisting of C(R^{a}R^{b})-C(R^{a}R^{b})-, C(R^{a}R^{b})-O-, C(R^{a}R^{b})-NR^{a}-, C(R^{a}R^{b})-S-, and C(R^{a}R^{b})-C(R^{a}R^{b})-O-, wherein each R^{a} and R^{b} may optionally be independently selected. In some embodiments, R^{a} and R^{b} may be, optionally independently selected from the group consisting of hydrogen and C₁₋₆alkyl, such as methyl, such as hydrogen.

In some embodiments, R^{4*} and R^{2*} together designate the biradical -O-CH(CH₂OCH₃)-(2'O-methoxyethyl bicyclic nucleic acid - Seth at al., 2010, J. Org. Chem) - in either the R- or S-configuration.

In some embodiments, R^{4*} and R^{2*} together designate the biradical -O-CH(CH₂CH₃)-(2'O-ethyl bicyclic nucleic acid - Seth at al., 2010, J. Org. Chem). - in either the R- or S-configuration.

In some embodiments, R^{4*} and R^{2*} together designate the biradical -O-CH(CH₃)-. - in either the R- or S- configuration.

In some embodiments, R^{4*} and R^{2*} together designate the biradical -O-CH₂-O-CH₂- - (Seth at al., 2010, J. Org. Chem).

In some embodiments, R^{4*} and R^{2*} together designate the biradical -O-NR-CH₃- - (Seth at al., 2010, J. Org. Chem).

In some embodiments, the LNA units have a structure selected from the following group:

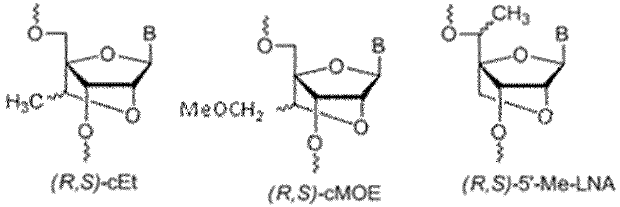

In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. For all chiral centers, asymmetric groups may be found in either R or S orientation.

In some embodiments, R^{1*}, R², R³, R⁵, R^{5*} are hydrogen.

In some embodiments, R^{1*}, R², R³ are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. For all chiral centers, asymmetric groups may be found in either R or S orientation.

In some embodiments, R^{1*}, R², R³ are hydrogen.

In some embodiments, R⁵ and R^{5*} are each independently selected from the group consisting of H, -CH₃, -CH₂-CH₃,- CH₂-O-CH₃, and -CH=CH₂. Suitably in some embodiments, either R⁵ or R^{5*} are hydrogen, whereas the other group (R⁵ or R^{5*} respectively) is selected from the group consisting of C₁₋₅ alkyl, C₂₋₆ alkenyl, C₂₋₆ alkynyl, substituted C₁₋₆ alkyl, substituted C₂₋₆ alkenyl, substituted C₂₋₆ alkynyl or substituted acyl (-C(=O)-); wherein each substituted group is mono or poly substituted with substituent groups independently selected from halogen, C₁₋₆ alkyl, substituted C₁₋₆ alkyl, C₂₋₆ alkenyl, substituted C₂₋₆ alkenyl, C₂₋₆ alkynyl, substituted C₂₋₆ alkynyl, OJ₁, SJ₁, NJ₁J₂, N₃, COOJ₁, CN, O-C(=O)NJ₁J₂, N(H)C(=NH)NJ,J₂ or N(H)C(=X)N(H)J₂ wherein X is O or S; and each J₁ and J₂ is, independently, H, C₁₋₆ alkyl, substituted C₁₋₆ alkyl, C_{2- 6} alkenyl, substituted C₂₋₆ alkenyl, C₂₋₆ alkynyl, substituted C₂₋₆ alkynyl, C₁₋₆ aminoalkyl, substituted C₁₋₆ aminoalkyl or a protecting group. In some embodiments either R⁵ or R^{5*} is substituted C₁₋₆ alkyl. In some embodiments either R⁵ or R^{5*} is substituted methylene wherein preferred substituent groups include one or more groups independently selected from F, NJ₁J₂, N₃, CN, OJ₁, SJ₁, O-C(=O)NJ₁J₂, N(H)C(=NH)NJ, J₂ or N(H)C(O)N(H)J₂. In some embodiments each J₁ and J₂ is, independently H or C₁₋₆alkyl. In some embodiments either R⁵ or R^{5*} is methyl, ethyl or methoxymethyl. In some embodiments either R⁵ or R^{5*} is methyl. In a further embodiment either R⁵ or R^{5*} is ethylenyl. In some embodiments either R⁵ or R^{5*} is substituted acyl. In some embodiments either R⁵ or R^{5*} is C(=O)NJ₁J₂. For all chiral centers, asymmetric groups may be found in either R or S orientation. Such 5' modified bicyclic nucleotides are disclosed in WO 2007/1341 81. In some embodiments B is a nucleobase, including nucleobase analogues and naturally occurring nucleobases, such as a purine or pyrimidine, or a substituted purine or substituted pyrimidine, such as a nucleobase referred to herein, such as a nucleobase selected from the group consisting of adenine, cytosine, thymine, adenine, uracil, and/or a modified or substituted nucleobase, such as 5-thiazolo-uracil, 2-thio-uracil, 5-propynyl-uracil, 2'thio-thymine, 5-methyl cytosine, 5-thiozolo-cytosine, 5-propynyl-cytosine, and 2,6-diaminopurine.

In some embodiments, R^{4*} and R^{2*} together designate a biradical selected from -C(R^{a}R^{b})-O-, -C(R^{a}R^{b})-C(R^{c}R^{d})-O-, -C(R^{a}R^{b})-C(R^{c}R^{d})-C(R^{e}R^{f})-O-, -C(R^{a}R^{b})-O-C(R^{c}R^{d})-, -C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-, -C(R^{a})=C(R^{b})-C(R^{c}R^{d})-, -C(R^{a}R^{b})-N(R^{c})-, -C(R^{a}R^{b})-C(R^{c}R^{d})-N(R^{e})-, -C(R^{a}R^{b})-N(R^{c})-O-, and -C(R^{a}R^{b})-S-, -C(R^{a}R^{b})-C(R^{c}R^{d})-S-, wherein R^{a}, R^{b}, R^{c}, R^{d}, R^{e}, and R^{f} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂). For all chiral centers, asymmetric groups may be found in either R or S orientation.

In some embodiments, R^{4*} and R^{2*} together designate a biradical (bivalent group) selected from -CH₂-O-, -CH₂-S-, -CH₂-NH-, -CH₂-N(CH₃)-, -CH₂-CH₂-O-, -CH₂-CH(CH₃)-, -CH₂-CH₂-S-, -CH₂-CH₂-NH-, -CH₂-CH₂-CH₂-, -CH₂-CH₂-CH₂-O-, -CH₂-CH₂-CH(CH₃)-, -CH=CH-CH₂-, -CH₂-O-CH₂-O-, -CH₂-NH-O-, -CH₂-N(CH₃)-O-, -CH₂-O-CH₂-, -CH(CH₃)-O-, and -CH(CH₂-O-CH₃)-O-, and/or, -CH₂-CH₂-, and -CH=CH- For all chiral centers, asymmetric groups may be found in either R or S orientation.

In some embodiments, R^{4*} and R^{2*} together designate the biradical C(R^{a}R^{b})-N(R^{c})-O-, wherein R^{a} and R^{b} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen, and; wherein R^{c} is selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen.

In some embodiments, R^{4*} and R^{2*} together designate the biradical C(R^{a}R^{b})-O-C(R^{c}R^{d})-O-, wherein R^{a}, R^{b}, R^{c}, and R^{d} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen.

In some embodiments, R^{4*} and R^{2*} form the biradical -CH(Z)-O-, wherein Z is selected from the group consisting of C₁₋₆ alkyl, C₂₋₆ alkenyl, C₂₋₆ alkynyl, substituted C₁₋₆ alkyl, substituted C₂₋₆ alkenyl, substituted C₂₋₆ alkynyl, acyl, substituted acyl, substituted amide, thiol or substituted thio; and wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ³C(=X)NJ₁J₂ and CN, wherein each J₁, J₂ and J₃ is, independently, H or C₁₋₆ alkyl, and X is O, S or NJ₁. In some embodiments Z is C₁₋₆ alkyl or substituted C₁₋₆ alkyl. In some embodiments Z is methyl. In some embodiments Z is substituted C₁₋₆ alkyl. In some embodiments said substituent group is C₁₋₆ alkoxy. In some embodiments Z is CH₃OCH₂-. For all chiral centers, asymmetric groups may be found in either R or S orientation. Such bicyclic nucleotides are disclosed in US 7,399,845. In some embodiments, R^{1*}, R², R³, R⁵, R^{5*} are hydrogen. In some some embodiments, R^{1*}, R², R^{3*} are hydrogen, and one or both of R⁵, R^{5*} may be other than hydrogen as referred to above and in WO 2007/134181.

In some embodiments, R^{4*} and R^{2*} together designate a biradical which comprise a substituted amino group in the bridge such as consist or comprise of the biradical -CH₂-N(R^{c})-, wherein R^{c} is C₁₋₁₂ alkyloxy. In some embodiments R^{4*} and R^{2*} together designate a biradical -Cq₃q₄-NOR-, wherein q₃ and q₄ are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl; wherein each substituted group is, independently, mono or poly substituted with substituent groups independently selected from halogen, OJ₁, SJ₁, NJ₁J₂, COOJ₁, CN, O-C(=O)NJ₁J₂, N(H)C(=NH)N J₁J₂ or N(H)C(=X=N(H)J₂ wherein X is 0 or S; and each of J₁ and J₂ is, independently, H, C₁₋₆ alkyl, C₂₋₆ alkenyl, C₂₋₆ alkynyl, C₁₋₆ aminoalkyl or a protecting group. For all chiral centers, asymmetric groups may be found in either R or S orientation. Such bicyclic nucleotides are disclosed in WO2008/150729. In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. In some embodiments, R^{1*}, R², R³, R⁵, R^{5*} are hydrogen. In some embodiments, R^{1*}, R², R³ are hydrogen and one or both of R⁵, R^{5*} may be other than hydrogen as referred to above and in WO 2007/134181. In some embodiments R^{4*} and R^{2*} together designate a biradical (bivalent group) C(R^{a}R^{b})-O-, wherein R^{a} and R^{b} are each independently halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJ₁ SJ₁, SOJ₁, SO₂J₁, NJ₁J₂, N₃, CN, C(=O)OJ₁, C(=O)NJ₁J₂, C(=O)J₁, O-C(=O)NJ₁J₂, N(H)C(=NH)NJ₁J₂, N(H)C(=O)NJ₁J₂ or N(H)C(=S)NJ₁J₂; or R^{a} and R^{b} together are =C(q3)(q4); q₃ and q₄ are each, independently, H, halogen, C₁-C₁₂alkyl or substituted C₁-C₁₂ alkyl; each substituted group is, independently, mono or poly substituted with substituent groups independently selected from halogen, C₁-C₆ alkyl, substituted C₁-C₆ alkyl, C₂- C₆ alkenyl, substituted C₂-C₆ alkenyl, C₂-C₆ alkynyl, substituted C₂-C₆ alkynyl, OJ₁, SJ₁, NJ₁J₂, N₃, CN, C(=O)OJ₁, C(=O)NJ₁J₂, C(=O)J₁, O-C(=O)NJ₁J₂, N(H)C(=O)NJ₁J₂ or N(H)C(=S)NJ₁J_{2.} and; each J₁ and J₂ is, independently, H, C1-C₆ alkyl, substituted C1-C₆ alkyl, C₂-C₆ alkenyl, substituted C₂-C₆ alkenyl, C₂-C₆ alkynyl, substituted C₂-C₆ alkynyl, C1-C₆ aminoalkyl, substituted C1-C₆ aminoalkyl or a protecting group. Such compounds are disclosed in WO2009/006478A. In some embodiments, R^{4*} and R^{2*} form the biradical - Q -, wherein Q is C(q₁)(q₂)C(q₃)(q₄), C(q₁)=C(q₃), C[=C(q₁)(q₂)]-C(q₃)(q₄) or C(q₁)(q₂)-C[=C(q₃)(q₄)]; q₁, q₂, q₃, q₄ are each independently. H, halogen, C₁₋₁₂ alkyl, substituted C₁₋₁₂ alkyl, C₂₋₁₂ alkenyl, substituted C₁₋₁₂ alkoxy, OJ₁, SJ₁, SOJ₁, SO₂J₁, NJ₁J₂, N₃, CN, C(=O)OJ₁, C(=O)-NJ₁J₂, C(=O) J₁, - C(=O)NJ₁J₂, N(H)C(=NH)NJ₁J₂, N(H)C(=O)NJ₁J₂ or N(H)C(=S)NJ₁J₂; each J₁ and J₂ is, independently, H, C₁₋₆ alkyl, C₂₋₆ alkenyl, C₂₋₆ alkynyl, C₁₋₆ aminoalkyl or a protecting group; and, optionally wherein when Q is C(q₁)(q₂)(q₃)(q₄) and one of q₃ or q₄ is CH₃ then at least one of the other of q₃ or q₄ or one of q₁ and q₂ is other than H. In some embodiments, R^{1*}, R², R³, R⁵, R^{5*} are hydrogen. For all chiral centers, asymmetric groups may be found in either R or S orientation. Such bicyclic nucleotides are disclosed in WO2008/154401. In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. In some embodiments, R^{1*}, R², R³, R⁵, R^{5*} are hydrogen. In some embodiments, R^{1*}, R², R³ are hydrogen and one or both of R⁵, R^{5*} may be other than hydrogen as referred to above and in WO 2007/134181 or WO2009/067647 (alpha-L-bicyclic nucleic acids analogs).

Further bicyclic nucleoside analogues and their use in antisense oligonucleotides are disclosed in WO2011/115818, WO2011/085102, WO2011/017521, WO09100320, WO2010/036698, WO2009/124295 and WO2009/006478. Such nucleoside analogues may in some aspects be useful in the compounds of present invention.

In some embodiments, the LNA used in an antisense oligonucleotide of the present invention preferably has the structure of the general formula II: 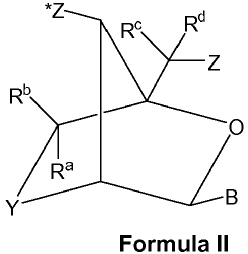 wherein Y is selected from the group consisting of -O-, -CH₂O-, -S-, -NH-, N(R^{e}) and/or-CH₂-; Z and Z* are independently selected among an internucleotide linkage, R^{H}, a terminal group or a protecting group; B constitutes a natural or non-natural nucleotide base moiety (nucleobase), and R^{H} is selected from hydrogen and C₁₋₄-alkyl; R^{a}, R^{b}R^{c}, R^{d} and R^{e} are, optionally independently, selected from the group consisting of hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂); and R^{H} is selected from hydrogen and C₁₋₄-alkyl. In some embodiments R^{a}, R^{b}R^{c}, R^{d} and R^{e} are, optionally independently, selected from the group consisting of hydrogen and C₁₋₆ alkyl, such as methyl. For all chiral centers, asymmetric groups may be found in either R or S orientation, for example, two exemplary stereochemical isomers include the beta-D and alpha-L isoforms, which may be illustrated as follows: 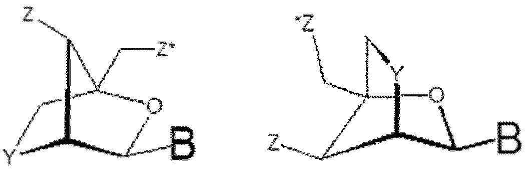 Specific exemplary LNA units are shown below:

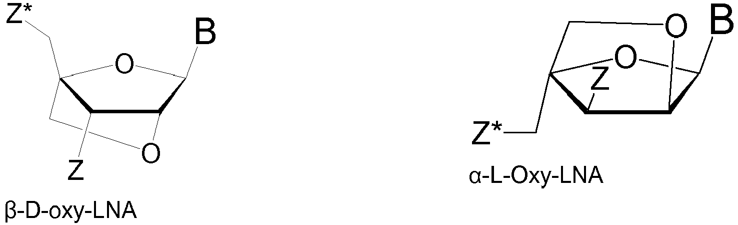

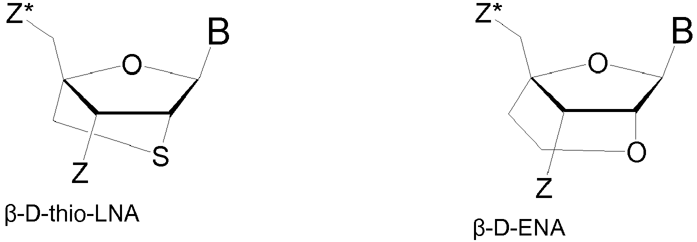

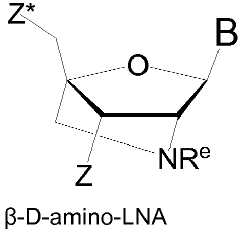

The term "thio-LNA" comprises a locked nucleotide in which Y in the general formula above is selected from S or -CH₂-S-. Thio-LNA can be in both beta-D and alpha-L-configuration.

The term "amino-LNA" comprises a locked nucleotide in which Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which Y in the general formula above represents -O-. Oxy-LNA can be in both beta-D and alpha-L-configuration.

The term "ENA" comprises a locked nucleotide in which Y in the general formula above is -CH₂-O- (where the oxygen atom of -CH₂-O- is attached to the 2'-position relative to the base B). R^{e} is hydrogen or methyl.

In some exemplary embodiments, LNA is selected from beta-D-oxy-LNA, alpha-L-oxy-LNA, beta-D-amino-LNA and beta-D-thio-LNA, in particular beta-D-oxy-LNA.

### Gapmer Design

The oligonucleotide of the present invention is preferably a gapmer. A gapmer oligonucleotide is an oligonucleotide which comprises a contiguous stretch of nucleotides which is capable of recruiting an RNAse, such as RNAseH, such as a region of at least 6 or 7 DNA nucleotides, referred to herein in as region B or region Y_{b}. The length of the RNAseH recruiting region can be indicated by an integer number _{b} between 5 and 15. Region B or Y is flanked both 5' and 3' by regions of affinity enhancing nucleotide analogues, such as between 1-6 nucleotide analogues 5' and 3' to the contiguous stretch of nucleotides which is capable of recruiting RNAse. These regions are referred to as regions A or X and C or X_{a'} respectively. The number of the nucleotide analogues can be indicated by ₐ or _{a'} and is between 1 and 6, preferably 1, 2, 3, 4 or 5.

EP 1 222 309 provides *in vitro* methods for determining RNaseH activity, which may be used to determine the ability to recruit RNaseH. An oligomer is deemed capable of recruiting RNase H if, when provided with the complementary RNA target, it has an initial rate, as measured in pmol/l/min, of at least 1 %, such as at least 5%, such as at least 10% or ,more than 20% of the of the initial rate determined using DNA only oligonucleotide, having the same base sequence but containing only DNA monomers, with no 2' substitutions, with phosphorothioate linkage groups between all monomers in the oligonucleotide, using the methodology provided by Example 91 - 95 of EP 1 222 309.

In some embodiments, an oligomer is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

In other embodiments, an oligomer is deemed capable of recruiting RNaseH if, when provided with the complementary RNA target, and RNaseH, the RNaseH initial rate, as measured in pmol/l/min, is at least 20%, such as at least 40 %, such as at least 60 %, such as at least 80 % of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309. In some embodiments, the monomers which are capable of recruiting RNAse are selected from the group consisting of DNA monomers, alpha-L-LNA monomers, C4' alkylayted DNA monomers (see WO2009/090182 and Vester et al., Bioorg. Med. Chem. Lett. 18 (2008) 2296 - 2300), and UNA (unlinked nucleic acid) nucleotides (see Fluiter et al., Mol. Biosyst., 2009, 10, 1039). UNA is unlocked nucleic acid, typically where the C2 - C3 C-C bond of the ribose has been removed, forming an unlocked "sugar" residue.

In some embodiments, a gapmer comprises a (poly)nucleotide sequence of formula (5' to 3'), A-B-C or X*ₐ*-Y*_{b}*-X*_{a'}*, or optionally A-B-C-D or D-A-B-C or X*ₐ*-Y*_{b}*-X*ₐ-D or* D- X*ₐ*-Y*_{b}*-X*_{a'},* wherein; region A or X*ₐ* (5' region) consists or comprises of at least one nucleotide analogue, such as at least one locked nucleic acid (LNA) unit, such as from 1-6 nucleotide analogues, such as LNA units, and; region B or Y consists or comprises of at least five consecutive nucleotides which are capable of recruiting RNAse (when formed in a duplex with a complementary RNA molecule, such as the mRNA target), such as DNA nucleotides, and; region C or X_{*a*'} (3' region) consists or comprises of at least one nucleotide analogue, such as at least one LNA unit, such as from 1-6 nucleotide analogues, such as LNA units, and; region D, when present consists or comprises of 1, 2 or 3 nucleotide units, such as DNA nucleotides.

In some embodiments, region A or X*ₐ* includes or consists of 1, 2, 3, 4, 5 or 6 nucleotide analogues, such as LNA units, such as from 2-5 nucleotide analogues, such as 2-5 LNA units, such as 3 or 4 nucleotide analogues, such as 3 or 4 LNA units; and/or region C or X_{*a*'} includes or consists of 1, 2, 3, 4, 5 or 6 nucleotide analogues, such as LNA units, such as from 2-5 nucleotide analogues, such as 2-5 LNA units, such as 3 or 4 nucleotide analogues, such as 3 or 4 LNA units.

In some embodiments, B or Y includes or consists or comprises of 5, 6, 7, 8, 9, 10, 11 or 12 consecutive nucleotides which are capable of recruiting RNAse, or from 5-15, or from 6-10, or from 7-9, such as 8 consecutive nucleotides which are capable of recruiting RNAse. In some embodiments, region B or Y consists or comprises at least one DNA nucleotide unit, such as 1-12 DNA units, preferably from 4-12 DNA units, more preferably from 6-10 DNA units, such as from 7-10 DNA units, most preferably 8, 9 or 10 DNA units.

In some embodiments, region A or X*ₐ* includes or consists of 3 or 4 nucleotide analogues, such as described in the "Nucleosides and Nucleoside analogues" section, preferably the analogue is LNA. Region B includes or consists of 7, 8, 9 or 10 DNA units, and region C or X*_{a'}* includes or consists of 3 or 4 nucleotide analogues, such as described in the "Nucleosides and Nucleoside analogues" section, preferably the analogue is LNA. Such designs include, for example, (A-B-C or X*ₐ*-Y*_{b}*-X*ₐ*) 2-11-3, 2-10-2, 2-8-4, 2-9-3, 2-9-4, 3-10-3, 3-10-4, 4-10-3, 3-9-3, 3-9-4, 4-9-3, 3-8-3, 3-8-4, 4-8-3, 3-7-3, 3-7-4, 4-7-3, and may further include region D, which may have one or 2 nucleotide units, such as DNA units. Examples of gapmer designs are shown in WO2004/046160. In some embodiments, a gapmer antisense oligonucleotide of the present invention may be a shortmer gapmer as described in U.S. Provisional Patent Application No. 60/977,409. In some embodiments, an oligonucleotide of the present invention comprises a contiguous nucleotide sequence of a total of 10, 11, 12, 13, 14, 15, 16, 17 or 18 nucleotide units, wherein the contiguous nucleotide sequence is of formula (5'-3'), A-B-C or X*ₐ*-Y*_{b}*-X_{*a*'}, or optionally A-B-C-D or D-A-B-C or X*ₐ*-Y*_{b}*-X*_{a'}*-*D or D-* X*ₐ*-Y*_{b}*-X*_{a'}*, wherein; A or X*_{a'}* consists of 1, 2, 3 or 4 nucleotide analogue units, such as LNA units; B or Y consists of 7, 8, 9, 10 or 11 contiguous nucleotide units which are capable of recruiting RNAse when formed in a duplex with a complementary RNA molecule (such as a mRNA target); and C or X*_{a'}* consists of 1, 2, 3 or 4 nucleotide analogue units, such as LNA units. When present, D consists of a single DNA unit.

In some embodiments, A or X*ₐ* consists of 1 LNA unit. In some embodiments, A or X*ₐ* consists of 2 LNA units. In some embodiments, A or X*ₐ* consists of 3 LNA units. In some embodiments, A or *Xₐ* consists of 4 LNA units. In some embodiments, C or X*_{a'}* consists of 1 LNA unit. In some embodiments, C or X*_{a'}* consists of 2 LNA units. In some embodiments, C or X*_{a'}*consists of 3 LNA units. In some embodiments, C or X*_{a'}*consists of 4 LNA units. In some embodiments, B or Yconsists of 7 nucleotide units. In some embodiments, B or Y consists of 8 nucleotide units. In some embodiments, B or Yconsists of 9 nucleotide units. In certain embodiments, region B consists of 10 nucleoside monomers. In certain embodiments, region B or Y comprises 1 - 10 DNA monomers. In some embodiments, B consists of 10 nucleotide units. In some embodiments, B or Y consists of 11 nucleotide units. In some embodiments, B or Y comprises of between 1-11 DNA units, inclusive, such as 2, 3, 4, 5, 6, 7, 8, 9, 10, or 11 DNA units. In some embodiments, B or Y consists of DNA units. In some embodiments B or Y comprises of at least one LNA unit which is in the alpha-L configuration, such as 2, 3, 4, 5, 6, 7, 8 or 9 LNA units in the alpha-L-configuration. In some embodiments, B or Y comprises of at least one alpha-L-oxy LNA unit or wherein all the LNA units in the alpha-L-configuration are alpha-L-oxy LNA units. In some embodiments, the number of nucleotides present in A-B-C or X*ₐ*-Y*_{b}*-X*_{a'}* are selected from the group consisting of (nucleotide analogue units--region B orYnucleotide analogue units): 1-8-1, 1-8-2, 2-8-1, 2-8-2, 3-8-3, 2-8-3, 3-8-2, 4-8-1, 4-8-2, 1-8-4, 2-8-4, or; 1-9-1, 1-9-2, 2-9-1, 2-9-2, 2-9-3, 3-9-2, 1-9-3, 3-9-1, 4-9-1, 1-9-4, or; 1-10-1, 1-10-2, 2-10-1, 2-10-2, 1-10-3, 3-10-1, or; 1-11-1, 1-11-2, 2-11-1, 2-11-2, 2-11-3, 3-11-2, 4-11-1, 1-11-4. In some embodiments, the number of nucleotides in A-B-C are selected from the group consisting of 3-8-3, 3-10-3, 3-9-3, 2-8-3, 2-11-3, 3-9-4, 4-9-3, 4-8-4, 3-8-5, 5-8-3, 2-10-3, 3-10-2, 4-9-2, 2-9-4, 4-8-3, 3-8-4, 2-10-2, 2-9-3, 3-9-2, 4-8-2, 2-8-4 and 4-7-4. In certain embodiments, each of regions A and C or X*ₐ* and X*_{a'}* consists of three LNA monomers, and region B or Y consists of 8 or 9 or 10 nucleoside monomers, preferably DNA monomers. In some embodiments, both A and C consists of two, three or four LNA units each, and B consists of 8, 9, 10 or 11 nucleotide units, preferably DNA units.

In various embodiments, other gapmer designs include those where regions A and/or C or X*ₐ* and/or X*_{a'}*consists of 3, 4, 5 or 6 nucleoside analogues, such as monomers containing a 2'-O-methoxyethyl-ribose sugar (2'-MOE) or monomers containing a 2'-fluoro-deoxyribose sugar, and region B consists of 8, 9, 10, 11 or 12 nucleosides, such as DNA monomers, where regions A-B-C have 3-9-3, 3-10-3,5-10-5 or 4-12-4 monomers. Further gapmer designs are disclosed in WO2007/146511.

### Internucleotide Linkages

Monomers of an antisense oligonucleotide as described herein are coupled together via linkage groups. Suitably, each monomer is linked to the 3' adjacent monomer via a linkage group.

Upon reading the present disclosure, persons of ordinary skill in the art would understand that the 5' monomer at the end of an antisense oligonucleotide of the present invention does not comprise a 5' linkage group, although it may or may not comprise a 5' terminal group.

The terms "linkage group" or "internucleotide linkage" are intended to mean a group capable of covalently coupling together two nucleotides. Specific and preferred examples include phosphate groups and phosphorothioate groups. An antisense oligonucleotide of the present or contiguous nucleotides sequence thereof are coupled together via linkage groups. Suitably, each nucleotide is linked to the 3' adjacent nucleotide via a linkage group. Exemplary internucleotide linkages include those described in WO2007/03109/. In some embodiments, an internucleotide linkage may be modified from its normal phosphodiester to one that is more resistant to nuclease attack, such as phosphorothioate or boranophosphate-these two, being cleavable by RNase H, also allow that route of antisense inhibition in reducing the expression of the target gene.

Suitable sulphur (S) containing internucleotide linkages as provided herein may be preferred. Phosphorothioate internucleotide linkages are also preferred, particularly for the gap region (B or Y) of gapmers. Phosphorothioate linkages may also be used for the flanking regions (A/Xₐ and C/ X_{a'}, and for linking A/Xₐ or C/ X*_{a'}* to D, and within region D, as appropriate).

Regions A or Xₐ, B or Y and C or X_{a'}, may however comprise internucleotide linkages other than phosphorothioate, such as phosphodiester linkages, particularly, for instance when the use of nucleotide analogues protects the internucleotide linkages within regions A or Xₐ and C or Xₐ from endo-nuclease degradation,such as when regions A or Xₐ and C or X_{a'} comprise LNA nucleotides.

Intemucleotide linkages of an oligonucleotide of the present invention may be phosphodiester, phosphorothioate or boranophosphate to allow RNase H cleavage of targeted RNA. Phosphorothioate is preferred, for improved nuclease resistance and other reasons, such as ease of manufacture. In some embodiments, nucleotides and/or nucleotide analogues of an oligonucleotide of the present invention are linked to each other by means of phosphorothioate groups. In a preferred embodiment of the invention the oligonucleotide comprise at least one phosphorothioate linkage.

It is recognized that the inclusion of phosphodiester linkages, such as one or two linkages, into an otherwise phosphorothioate oligonucleotide, particularly between or adjacent to nucleotide analogue units (typically in region A or Xₐ and or C or X_{a'}) can modify the bioavailability and/or bio-distribution of an oligonucleotide as described in WO2008/053314. In some embodiments, such as the embodiments referred to above, where suitable and not specifically indicated, all remaining linkage groups are either phosphodiester or phosphorothioate, or a mixture thereof.

In some embodiments, all the internucleotide linkage groups of the oligonucleotide are phosphorothioate. When referring to specific gapmer oligonucleotide sequences, such as those provided herein it will be understood that, in various embodiments, when the linkages are phosphorothioate linkages, alternative linkages, such as those disclosed herein may be used, for example phosphate (phosphodiester) linkages may be used, particularly for linkages between nucleotide analogues, such as LNA, units. Likewise, when referring to specific gapmer oligonucleotide sequences, such as those provided herein, when the C nucleotide residues are annotated as 5' methyl modified cytosine, in various embodiments, one or more of the C nucleotides present in the oligonucleotide may be unmodified C residues.

### Pharmaceutical compositions

The present invention further provides pharmaceutical compositions comprising therapeutic actives in accordance with the invention (e.g., antisense oligonucleotides), together with one or more pharmaceutically acceptable excipients. Such pharmaceutical compositions may optionally comprise one or more additional therapeutically-active substances.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation.

Formulations of the pharmaceutical compositions described herein may be prepared by any method known or hereafter developed in the art of pharmacology. In general, such preparatory methods include the step of bringing the active ingredient into association with a diluent or another excipient and/or one or more other accessory ingredients, and then, if necessary and/or desirable, shaping and/or packaging the product into a desired single- or multi-dose unit.

A pharmaceutical composition in accordance with the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition in accordance with the invention will vary, depending upon the identity, size, and/or condition of the subject treated and further depending upon the route by which the composition is to be administered. By way of example, the composition may comprise between 0.1 % and 100% (w/w) active ingredient.

Pharmaceutical formulations may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006;) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

In some embodiments, a pharmaceutically acceptable excipient is at least 95%, at least 96%, at least 97%, at least 98%, at least 99%, or 100% pure. In some embodiments, an excipient is approved for use in humans and for veterinary use. In some embodiments, an excipient is approved by United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

General considerations in the formulation and/or manufacture of pharmaceutical agents may be found, for example, in Remington: The Science and Practice of Pharmacy 21st ed., Lippincott Williams & Wilkins, 2005. In some embodiments, liposomes may be used to deliver antisense oligonucleotides described herein. As used herein, a liposome is an artificially-prepared vesicle composed of a lipid bilayer. Liposomes can be prepared by disrupting biological membranes (such as by sonication). Liposomes may include natural phospholipids, or mixed lipid chains with surfactant properties (e.g., egg phosphatidylethanolamine). A liposome design may employ surface ligands for targeting desired target tissues.

### Administration

The present invention provides methods of administering an effective amount of a therapeutic active described herein (e.g., an antisense oligonucleotide) to a subject in need of treatment.

Antisense oligonucleotides described herein may be administered through various administration routes including, but not limited to, intravenous, subcutaneous, intramuscular, parenteral, transdermal, or transmucosal (e.g., oral or nasal). In some embodiments, antisense oligonucleotides described herein may be administered through intravenous administration. In some embodiments, antisense oligonucleotides described herein may be administered through subcutaneous administration. In some embodiments, a dosage regime for an oligonucleotide may be repeated after an initial dosage regime, indeed the dosage regime may be repeated as necessary in order to treat or prevent the progression of a disease. In some embodiments, antisense oligonucleotides described herein may be administered daily, twice a week, once a week, bi-weekly, monthly, once every two months, once every three months, once every four months, once every six months, or at variable intervals.

### Applications

Antisense oligonucleotides of the present invention may be utilized as research reagents for, for example, diagnostics, therapeutics and prophylaxis.

In research, an antisense oligonucleotide of the present invention may be used to specifically inhibit the synthesis of BCL11A protein (typically by degrading or inhibiting the mRNA and thereby prevent protein formation) in cells and experimental animals thereby facilitating functional analysis of the target or an appraisal of its usefulness as a target for therapeutic intervention.

In diagnostics, an antisense oligonucleotide of the present invention may be used to detect and quantitate BCL11A expression in cell and tissues by northern blotting, *in-situ* hybridisation or similar techniques.

For therapeutics, an animal or a human, suspected of having a disease or disorder, which can be treated by modulating the expression of BCL11A is treated by administering an antisense oligonucleotide of the present invention. Further provided are methods of treating a mammal, such as treating a human, suspected of having or being prone to a disease or condition, associated with expression of BCL11A by administering a therapeutically or prophylactically effective amount of one or more of an antisense oligonucleotide or composition of the present invention. An antisense oligonucleotide, a conjugate or a pharmaceutical composition of the present invention is typically administered in an effective amount.

Antisense oligonucleotides of the present invention are suitable for the manufacture of a medicament for the treatment of a disorder as referred to herein, or for a method of the treatment of as a disorder as referred to herein.

A method for treating a disorder as referred to herein is provided, said method comprising administering an antisense oligonucleotide as described herein, and/or a conjugate, and/or a pharmaceutical composition to a patient in need thereof.

The present invention provides a method of treating an anemic disease, disorder or condition comprising administering to a subject in need of treatment an oligonucleotide according to the invention or a pharmaceutical composition of the invention.

In one embodiment the anemic disease, disorder or condition is sickle cell disease.

In another embodiment the anemic disease, disorder or condition is β-thalassemia.

When applied in a method of treatment the administeration of an oligonucleotide of the invention or the pharmaceutical composition of the invention results in reduced expression of BCL11A in one or more target tissues. Preferably, the administration of the oligonucleotide of the inventionor the pharmaceutical composition of the invention results in increased γ-globin expression in one or more target tissues. The administion of the oligonucleotide of the invention or the pharmaceutical composition of the invention may result in increased fetal hemoglobin production in one or more target tissues. Preferably, the target tissues are selected from bone marrow, liver, kidney, spleen and/or plasma cells, peripheral blood B-cells, dendritic cells, erythroid progenitor cells, pluripotent stem cells, thymus, tonsillar epithelium.

### Therapeutic uses

Antisense oligo nucleotide modulators of BCL11A described herein may be used to treat various BCL11A related diseases, disorders and conditions.

### Sickle Cell Disease (SCD)

Sickle Cell Disease, or sickle cell anemia is an inherited genetic disorder characterized by red blood cells having an abnormal, rigid, sickle shape, which reduces the flexibility of the cell. This results from a mutation in a beta globin chain gene and is manifested in an autosomal recessive manner with overdominance. SCD is associated with various severe complications, such as reduced life expectancy, and causes a pathological condition that can lead to death. However, due to genetic polymorphism of mutations, not all inherited hemoglobin variants are detrimental.

SCD is more commonly reported in populations from tropical and sub-tropical sub-Saharan regions. These are also regions where malaria is commonly observed. Interestingly, carriers of SCD (*i.e.,* having one copy of the mutation) are found to be more resistant to infection and show less severe symptoms when infected.

Antisense oligonucleotide modulators of BCL11A described herein may be used to treat SCD. The terms, "treat" or "treatment," as used herein, refers to amelioration of one or more symptoms associated with the disease, prevention or delay of the onset of one or more symptoms of the disease, and/or lessening of the severity or frequency of one or more symptoms of the disease.

In some embodiments, treatment refers to partially or completely alleviation, amelioration, relief, inhibition, delaying onset, reducing severity and/or incidence of one or more symptoms in a SCD patient, including, but not limited to, anemia; yellowing of the eyes; paleness, coldness and/or yellowing of the skin; shortness of breath; muscular weakness; intestinal changes (e.g., changes in stool color); fatigue; dizziness; fainting; changes to blood vessels (e.g., low blood pressure); changes affecting the heart (e.g., heart palpitations, rapid heart rate, chest pain, angina, heart attack), and organ enlargement (e.g., spleen).

In some embodiments, treatment refers to reduced symptoms of anemia in a subject in need of treatment. In certain embodiments, the amount of symptoms of anemia may be reduced by at least about 10%, 20%, 30%, 40%, 50%, 60%, 70%, 80%, 90%, 95%, or more as compared to a pre-treatment or no-treatment control (e.g., the amount of symptoms of anemia by a control subject with similar diseased or developmental stage but without treatment).

In some embodiments, treatment refers to increased gamma globin expression (e.g., total expression, percent expression increase per week, per month, per two months, per six months, *etc*.). In various embodiments, increased gamma globin expression compensates for a lack of or reduced expression of beta globulin in a SCD patient.

### Thalassemia (α, β and δ)

Thalassemia, like SCD, is an inherited genetic disorder that affects the blood. Thalassemia manifests as an autosomal recessive condition and leads to weakening and destruction of red blood cells. Thalassemia is caused by mutations or deletions of genes that affect how the body makes hemoglobin, which is the protein within red blood cells that is responsible for carrying oxygen. Individuals suffering from thalassemia are characterized by low hemoglobin production and have fewer circulating red blood cells than normal, which results in mild or severe anemia. Thalassemia originated in the Mediterranean region.

Thalassemia can cause significant complications, including pneumonia, iron overload, bone deformities and cardiovascular sickness. However, like SCD, this disease has been observed to confer a degree of protection against malaria for those that are carriers of the disease.

Hemoglobin is composed of four protein chains, two alpha and two beta globin chains, which are arranged in a heterodimer. In humans, the beta globin chains are encoded by a single gene on chromosome 11, while the alpha globin chains are encoded by two gene on chromosome 16 that are linked. This sets up a genetic situation where normal individuals contain two beta chain loci and four alpha chain loci. In patients with thalassemia, mutations in either the alpha or beta chain, which gives rise to the low and/or abnormal production of red blood cells. As a result, thalassemias are categorized according to which chain has a mutation. Alpha and beta thalassemias are common in African, Asian, Greek and Italian ethnic groups.

Alpha thalassemas (mutations in the alpha chain) concern the HBA1 and HBA2 genes, and result in decreased production of alpha globin. This creates a situation where there is an increase in beta globin production in adults and increase gamma globin production in infants. The increase in beta globin production leads to the formation of tetramers that are unstable and have an impaired ability to dissociate with oxygen.

Beta thalassemias (mutations in the beta chain) concern the HBB gene, and the severity of the disease that results is dependent on the mutation. Some mutations prevent the formation of beta chains, which the most severe form of the disease, while others allow some formation of beta chains, albeit at a reduced level. As a result of beta chain mutation, there is an excess of alpha chain production, which do not form tetramers as in the case of alpha thalassemias. Alternatively, the excess alpha chains bind to the membranes of red blood cells and result in damage to the membrane, and can be toxic if the alpha chains aggregate.

Although at a low frequency, delta thalassemias can occur. Similarly, they result from mutations in delta globin chain genes and result in an abnormal production of these chains. It has been reported that about 3% of hemoglobin of adults is made of alpha and delta chains.

Antisense oligonucleotide modulators of BCL11A described herein may be used to treat thalassemias, e.g., alpha, beta and/or delta thalassemas. The terms, "treat" or "treatment," as used herein, refers to amelioration of one or more symptoms associated with the disease, prevention or delay of the onset of one or more symptoms of the disease, and/or lessening of the severity or frequency of one or more symptoms of the disease.

In some embodiments, treatment refers to partial or complete alleviation, amelioration, relief, inhibition, delaying onset, reducing severity and/or incidence of one or more symptoms in a thalassemia patient, including, but not limited to, pneumonia, iron overload, bone deformities and cardiovascular sickness. In some embodiments, treatment refers to partial or complete alleviation, amelioration, relief, inhibition, delaying onset, reducing severity and/or incidence of one or more symptoms in a thalassemia patient, including, but not limited to, anemia; yellowing of the eyes; paleness, coldness and/or yellowing of the skin; shortness of breath; muscular weakness; intestinal changes (e.g., changes in stool color); fatigue; dizziness; fainting; changes to blood vessels (*e.g.,* low blood pressure); changes affecting the heart (*e.g.,* heart palpitations, rapid heart rate, chest pain, angina, heart attack), and organ enlargement (e.g., spleen).

In some embodiments, treatment refers to reduced symptoms of anemia in a subject in need of treatment. In certain embodiments, the amount of symptoms of anemia may be reduced by at least about 10%, 20%, 30%, 40%, 50%, 60%, 70%, 80%, 90%, 95%, or more as compared to a pre-treatment or no-treatment control (e.g., the amount of symptoms of anemia by a control subject with similar diseased or developmental stage but without treatment).

In some embodiments, treatment refers to increased gamma globin expression (e.g., total expression, percent expression increase per week, per month, per two months, per six months, *etc*.). In various embodiments, increased gamma globin expression compensates for a lack of or reduced expression of alpha, beta or delta globulin in a thalassemia patient.

The invention will be more fully understood by reference to the following examples. They should not, however, be construed as limiting the scope of the invention.

### EXAMPLES

### Example 1. Design and synthesis of oligonucleotides that target BCL11A

This example illustrates exemplary methods of designing and synthesis of LNA oligonucleotides that can effectively down-regulate BCL11A expression and activity. In this example, the primary target region is the overlapping regions among the XL, L and S isoforms (Figure 2).

A total of 401 LNA oligonucleotides were designed and synthesized in seven libraries based on the sequences of the three major isoforms of human BCL11A (*i.e.,* XL, L or S; Table 3) resulting in oligonucleotides of various specificities, lengths (e.g., 12-16 mers) and LNA designs.

**TABLE 3**

| BCL11A | Isoform | Accession No. | |
|---|---|---|---|
| Human | XL | NM_022893 | SEQ ID NO: 1 |
| | L | NM_018014 | SEQ ID NO: 2 |
| | S | NM_138559 | SEQ ID NO: 3 |
| Mouse | XL | NM_001242934 | SEQ ID NO: 4 |
| | L | NM_016707 | SEQ ID NO: 5 |
| | S | NM_001159289 | SEQ ID NO: 6 |

Exemplary methods for designing LNA units are described in Wahlestedt, C. et al. 2000, PNAS 91(10):5633-5638. Exemplary LNA oligonucleotides are shown in Table 4.

**TABLE 4**

| Oligo # | BCL11A-XL Position | Length | LNA Design | Sequence (5'-3') |
|---|---|---|---|---|
| 1 | 597 | 14 | 3-8-3 | **^{m}Cₛ^{o}Tₛ^{o}Aₛ^{o}**tₛgₛtₛgₛtₛtₛcₛcₛ**Tₛ^{o}Gₛ^{o}T^{o}** |
| 2 | 220 | 16 | 3-10-3 | **Gₛ^{o}Aₛ^{o}Gₛ^{o}**aₛcₛaₛtₛgₛgₛtₛgₛgₛgₛ**^{m}Cₛ^{o}Tₛ^{o}G^{o}** |
| 3 | 429 | 16 | 3-10-3 | **Aₛ^{o}Tₛ^{o}Tₛ^{o}**gₛcₛaₛtₛtₛgₛtₛtₛtₛcₛ**^{m}Cs^{o}Gs^{o}T^{o}** |
| 4 | 430 | 16 | 3-10-3 | **^{m}Cₛ^{o}Aₛ^{o}Tₛ**^{o}tₛgₛcₛaₛtₛtₛgₛtₛtₛtₛ^{m}**Cₛ^{om}Cₛ^{o}G**^{o} |
| 5 | 430 | 15 | 3-9-3 | **Aₛ^{o}Tₛ^{o}Tₛ**^{o}gₛ^{m}Cₛaₛtₛtₛgₛtₛtₛtₛ**^{m}Cₛ^{om}Cₛ^{o}G^{o}** |
| 6 | 430 | 15 | 3-9-3 ^{m}c | **Aₛ^{o}Tₛ^{o}Tₛ^{o}**gₛcₛaₛtₛtₛgₛtₛtₛtₛ**^{m}Cₛ^{om}Cₛ^{o}G^{o}** |
| 7 | 415 | 14 | 3-8-3 ^{m}c | **Tₛ^{o}Tₛ^{o}Gₛ^{o}**tₛgₛcₛtₛ^{m}cₛgₛaₛtₛ**Aₛ^{o}Aₛ^{o}A^{o}** |
| 8 | 416 | 16 | 3-10-3 | **^{m}Cₛ^{o}Gₛ^{o}Tₛ^{o}**tₛtₛgₛtₛgₛcₛtₛ^{m}cₛgₛaₛ**Tₛ^{o}Aₛ^{o}A^{o}** |
| 9 | 419 | 14 | 3-8-3 | **^{m}Cₛ^{om}Cₛ^{o}Gₛ^{o}**tₛtₛtₛgₛtₛgₛcₛtₛ^{m}Cₛ^{o}Gₛ^{o}A^{o} |
| 10 | 416 | 13 | 2-8-3 ^{m}c | **Tₛ^{o}Tₛ^{o}**gₛtₛgₛcₛtₛ^{m}cₛxₛaₛ**Tₛ^{o}Aₛ^{o}A^{o}** |
| 11 | 420 | 16 | 3-10-3 ^{m}c | **Tₛ^{o}Tₛ^{o}Tₛ^{o}**cₛ^{m}cₛgₛtₛtₛtₛgₛtₛgₛcₛ**T**_{S}^{o}**^{m}Cₛ^{o}G^{o}** |
| 12 | 430 | 16 | 3-10-3 ^{m}c | **^{m}Cₛ^{o}Aₛ^{o}Tₛ^{o}**tₛgₛ^{m}cₛaₛtₛtₛgₛtₛtₛtₛ**^{m}Cₛ^{om}Cₛ^{o}G^{o}** |
| 13 | 430 | 16 | 2-11-3 | **^{m}Cₛ^{o}Aₛ^{o}**tₛ^{o}tₛgₛcₛaₛtₛtₛgₛtₛtₛtₛ**^{m}Cₛ^{om}Cₛ^{o}G^{o}** |
| 14 | 430 | 16 | 3-9-4 | **^{m}Cₛ^{o}Aₛ^{o}Tₛ^{o}**tₛgₛcₛaₛtₛtₛgₛtₛtₛ**T**ₛ^{om}**Cₛ**^{o}**^{m}Cₛ^{o}G^{o}** |
| 15 | 430 | 16 | 4-9-3 | **^{m}Cₛ^{o}Aₛ^{o}Tₛ^{o}Tₛ^{o}**gₛcₛaₛtₛtₛgₛtₛtₛtₛ**^{m}Cₛ^{om}Cₛ^{o}G^{o}** |
| 16 | 430 | 16 | 4-8-4 | **^{m}Cₛ^{o}Aₛ^{o}Tₛ^{o}Tₛ^{o}**gₛcₛaₛtₛtₛgₛtₛtₛ**Tₛ^{om}Cₛ^{om}Cₛ^{o} G**^{o} |
| 17 | 430 | 16 | 3-8-5 | **^{m}Cₛ^{o}Aₛ^{o}Tₛ^{o}**tₛg_{sCs}aₛtₛtₛgₛtₛ**Tₛ^{o}Tₛ^{om}Cₛ^{om}Cₛ^{o} G**^{o} |
| 18 | 430 | 16 | 5-8-3 | **^{m}Cₛ^{o}Aₛ^{o}Tₛ^{o}Tₛ^{o}Gₛ^{o}**cₛaₛtₛtₛgₛtₛtₛtₛ**^{m}Cₛ^{om}Cₛ^{o} G**^{o} |
| 19 | 415 | 16 | 3-10-3 ^{m}c | **Gₛ^{o}Tₛ^{o}Tₛ^{o}**tₛgₛtₛgₛcₛtₛ^{m}cₛgₛaₛtₛ**Aₛ^{o}Aₛ^{o}A^{o}** |
| 20 | 415 | 15 | 3-9-3 ^{m}c | **Tₛ^{o}Tₛ^{o}Tₛ^{o}**gₛtₛgₛcₛtₛ^{m}cₛgₛaₛtₛ**Aₛ^{o}Aₛ^{o}A^{o}** |
| 21 | 416 | 14 | 3-8-3 ^{m}c | **Tₛ^{o}Tₛ^{o}Tₛ^{o}**gₛtₛgₛcₛtₛ^{m}cₛgₛaₛtₛ**Aₛ^{o}Aₛ^{o}A^{o}** |
| 22 | 416 | 15 | 3-9-3 ^{m}c | **Gₛ^{o}Tₛ^{o}Tₛ^{o}**tₛgₛtₛgₛcₛtₛ^{m}cₛgₛaₛ**Tₛ^{o}Aₛ^{o}Aₛ^{o}** |
| 23 | 417 | 14 | 3-8-3 ^{m}c | **Gₛ^{o}Tₛ^{o}Tₛ^{o}**tₛgₛtₛgₛcₛtₛ^{m}cₛgₛaₛ**Tₛ^{o}Aₛ^{o}Aₛ^{o}** |
| 24 | 417 | 16 | 3-10-3 ^{m}c | **^{m}Cₛ^{om}Cₛ^{o}Gₛ^{o}**tₛtₛtₛgₛtₛgₛcₛtₛ^{m}cₛgₛ**Aₛ^{o}Tₛ^{o}A^{o}** |
| 25 | 417 | 15 | 3-9-3 | **^{m}Cₛ^{o}Gₛ^{o}Tₛ^{o}**tₛtₛgₛtₛgₛcₛtₛ^{m}cₛgₛ**Aₛ^{o}Tₛ^{o}A^{o}** |
| 26 | 418 | 13 | 2-8-3 | **Gₛ^{o}Tₛ^{o}**tₛtₛgₛtₛgₛcₛtₛcₛ**G**ₛ**^{o}Aₛ^{o}T^{o}** |
| 27 | 418 | 14 | 3-8-3 | **^{m}Cₛ^{o}Gₛ^{o}Tₛ^{o}**tₛtₛgₛtₛgₛcₛtₛcₛ**Gₛ^{o}Aₛ^{o}T^{o}** |
| 28 | 418 | 15 | 3-9-3 | ^{m}**C**ₛ^{om}**C**ₛ^{o}**G**ₛ^{o}tₛtₛtₛgₛtₛgₛcₛtₛcₛ**G**ₛ^{o}**A**ₛ^{o}T^{o} |
| 29 | 418 | 16 | 3-10-3 | **T**ₛ^{om}**C**ₛ^{om}**C**ₛ^{o}gₛtₛtₛtₛgₛtₛgₛcₛtₛcₛ**G**ₛ^{o}Aₛ^{o}T^{o} |
| 30 | 419 | 13 | 2-8-3 | ^{m}**C**ₛ^{o}**G**ₛtₛtₛtₛgₛtₛgₛcₛtₛ^{m}**C**ₛ^{o}**G**ₛ^{o}A^{o} |
| 31 | 419 | 16 | 3-10-3 ^{m}c | **T**ₛ^{o}**T**ₛ^{om}Cₛ^{om}Cₛgₛtₛtₛtₛgₛtₛgₛcₛtₛ^{m}**C**ₛ^{o}**G**ₛ^{o} **A**^{o} |
| 32 | 419 | 15 | 3-9-3 | **T**ₛ^{om}**C**ₛo^{m}**C**ₛ^{o}gₛtₛtₛtₛgₛtₛgₛcₛtₛ^{m}**C**ₛ^{o}**G**ₛ^{o}**A**^{o} |
| 33 | 420 | 14 | 3-8-3 | **T**ₛ^{om}**C**ₛ^{om}**C**ₛ^{o}gₛtₛtₛtₛgₛtₛgₛcₛ**T**s^{om}**C**ₛ^{o}**G**^{o} |
| 34 | 420 | 15 | 3-9-3 ^{m}c | **T**ₛ^{o}**T**ₛ^{om}**Cs**^{om}Cₛgₛtₛtₛtₛgₛtₛgₛcₛ**Tₛ**o^{m}**C**ₛ^{o}**G**^{o} |
| 35 | 421 | 16 | 3-10-3 ^{m}c | **G**ₛ^{o}**T**ₛ^{o}**T**ₛ^{o}tₛcₛ^{m}cₛgₛtₛtₛtₛgₛtₛgₛ^{m}**C**ₛ^{o}**T**ₛ^{om}**C**^{o} |
| 36 | 421 | 15 | 3-9-3 ^{m}c | **T**ₛ^{o}**T**ₛ^{o}**T**ₛ^{o}cₛ^{m}cₛgₛtₛtₛtₛgₛtₛgₛ^{m}**C**ₛ^{o}**T**ₛ^{om}**C**^{o} |
| 37 | 416 | 16 | 2-11-3 | ^{m}**C**ₛ^{o}**G**ₛ^{o}tₛtₛtₛgₛtₛgₛcₛtₛ^{m}cₛgₛaₛ**T**s^{o}**A**ₛ^{o}**A**^{o} |
| 38 | 416 | 16 | 3-9-4 | ^{m}**C**ₛ^{o}**G**ₛ^{o}**T**ₛ^{o}tₛtₛgₛtₛgₛcₛtₛ^{m}cₛgₛ**A**ₛ^{o}**T**ₛ^{o}**A**ₛ^{o}A^{o} |
| 39 | 416 | 16 | 4-9-3 | ^{m}**C**ₛ^{o}**G**ₛ^{o}**T**ₛ^{o}**T**ₛ^{o}tₛgₛtₛgₛcₛtₛ^{m}cₛgₛaₛ**T**ₛ^{o}**A**ₛ^{o}**A**^{o} |
| 40 | 416 | 16 | 4-8-4 | ^{m}**C**ₛ^{o}**G**ₛ^{o}**T**ₛ^{o}**T**ₛ^{o}tₛgₛtₛgₛcₛtₛ^{m}cₛgₛ**A**ₛ^{o}**T**ₛ^{o}**A**ₛ^{o}**A**^{o} |
| 41 | 416 | 16 | 3-8-5 | ^{m}**C**ₛ^{o}**G**ₛ^{o}**T**ₛ^{o}tₛtₛgₛtₛgₛcₛtₛ^{m}cₛ**G**ₛ^{o}**A**ₛ^{o}**T**ₛ^{o}**A**ₛ^{o}**A**^{o} |
| 42 | 416 | 13 | 5-8-3 | ^{m}**C**ₛ^{o}Gₛ^{o}Tₛ^{o}Tₛ^{o}Tₛ^{o}gₛtₛgₛcₛtₛ^{m}cₛgₛaₛ**T**ₛ^{o}**A**ₛ^{o}**A**^{o} |
| 43 | 417 | 15 | 2-10-3 | ^{m}**C**ₛ^{o}Gₛ^{o}tₛtₛtₛgₛtₛgₛcₛtₛ^{m}cₛgₛ**A**ₛ^{o}**T**ₛ^{o}**A**^{o} |
| 44 | 417 | 15 | 3-10-2 | ^{m}**C**ₛ^{o}GₛTₛtₛtₛgₛtₛgₛcₛtₛ^{m}cₛgₛaₛ**T**ₛ^{o}**A**^{o} |
| 45 | 417 | 15 | 4-9-2 | ^{m}**C**ₛ^{o}**G**ₛ^{o}**T**ₛ^{o}**T**ₛ^{o}tₛgₛtₛgₛcₛtₛ^{m}cₛgₛaₛ**T**_{S}^{o}**A**^{o} |
| 46 | 417 | 15 | 2-9-4 | ^{m}**C**ₛ^{o}**G**ₛ^{o}tₛtₛtₛgₛtₛgₛcₛtₛ^{m}cₛ**G**ₛ^{o}**A**ₛ^{o}**T**ₛ^{o}**A**^{o} |
| 47 | 417 | 15 | 4-8-3 | ^{m}**C**ₛ^{o}**G**ₛ^{o}**T**ₛ^{o}**T**ₛ^{o}tₛgₛtₛgₛcₛtₛ^{m}cₛgₛ**A**ₛ^{o}Tₛ^{o}**A**^{o} |
| 48 | 417 | 15 | 3-8-4 | ^{m}**C**ₛ^{o}**G**ₛ^{o}Tₛ^{o}tₛtₛgₛtₛgₛcₛtₛ^{m}cₛ**Gₛ**^{o}**A**ₛ^{o}Tₛ^{o}**A**^{o} |
| 49 | 418 | 14 | 3-8-3 ^{m}c | ^{m}**C**ₛ^{o}**G**ₛ^{o}**T**ₛ^{o}tₛtₛgₛtₛgₛcₛtₛ^{m}cₛ**G**ₛ^{o}**A**ₛ^{o}T^{o} |
| 50 | 418 | 14 | 2-10-2 | ^{m}**C**ₛ^{o}**G**ₛ^{o}tₛtₛtₛgₛtₛgₛcₛtₛ^{m}cₛgₛ**A**ₛ^{o}**T**^{o} |
| 51 | 418 | 13 | 2-9-3 | ^{m}**C**ₛ^{o}**G**ₛ^{o}tₛtₛtₛgₛtₛgₛcₛtₛcₛ**G**ₛ^{o}**A**ₛ^{o}**T**^{o} |
| 52 | 418 | 13 | 3-9-2 | ^{m}**C**ₛ^{o}**G**ₛ^{o}**T**ₛ^{o}tₛtₛgₛtₛgₛcₛtₛ^{m}cₛgₛ**A**ₛ^{o}T^{o} |
| 53 | 418 | 14 | 4-8-2 | ^{m}Cₛ^{o}**G**ₛ^{o}**T**ₛ^{o}**T**ₛ^{o}tₛgₛtₛgₛcₛtₛ^{m}cₛgₛ**A**ₛ^{o}**T**^{o} |
| 54 | 418 | 14 | 2-8-4 | ^{m}**C**ₛ^{o}Gₛ^{o}tₛtₛtₛgₛtₛgₛcₛtₛ^{m}**C**ₛ^{o}**G**ₛ^{o}**A**ₛ^{o}T^{o} |
| 55 | 418 | 15 | 4-7-4 | ^{m}**C**ₛ^{o}**G**ₛ^{o}**T**ₛ^{o}**T**ₛ^{o}tₛgₛtₛgₛcₛtₛ^{m}**C**ₛ^{o}**G**ₛ^{o}**A**ₛ^{o}**T**^{o} |

| | | | | |
|---|---|---|---|---|
| ^{m}C denotes nucleotide monomer with a 5-methylcytosin-1-yl base; subscript "s" denotes a phosphorothioate linkage; Capital/bold base denotes a locked nucleic acid; superscript "o" denotes Oxy-LNA. | | | | |

### Example 2. In vitro screening and IC₅₀ determination of BCL11A-specific oligonucleotides

The effect of the oligonucleotides on BCL11A nucleic acid expression can be tested in any of a variety of cell types provided that the target nucleic acid is present at measurable levels. BCL11A can be expressed endogenously or by transient or stable transfection of a nucleic acid. The expression level of BCL11A nucleic acid can be routinely determined using, for example, Northern blot analysis, Quantitative PCR, Ribonuclease protection assays. In this example, oligonucleotides synthesized according to Example 1 that selectively target BCL11A were tested on human REH cells and BCL11A mRNA expression was measured. Other cell types can be routinely used, provided that the target is expressed in the cell type chosen. Cells were cultured in the appropriate medium as described below and maintained at 37°C. at 95-98% humidity and 5% CO₂. When cultured under hypoxia or anoxia, O₂ levels were kept at 1-2% or 0-0.5%, respectively. Cells were routinely passaged 2-3 times weekly.

Briefly, 401 oligonucleotides were employed in three-day mammalian cell culture experiments using human REH cells to determine the effect on expression of BCL11A mRNA. Antisense oligonucleotides were added to the cells at 5 and 25 µM without any additional reagents or uptake enhancers using gymnosis delivery technology (Stein, C.A. et al. 2010, Nucleic Acids Research 38(1):e3). BCL11A mRNA was measured by quantitative real-time RT-PCR (RT-qPCR). Exemplary results for inhibition of BCL11A by antisense oligonucleotides made in accordance with Example 1 is set forth in Figure 3.

As shown in Figure 3, antisense oligonucleotides made according to Example 1 were capable of inhibiting expression of BCL11A mRNA by targeting several different positions across BCL11A isoform XL, in particular, at positions overlapping among the XL, L and S isoforms (see Figure 2).

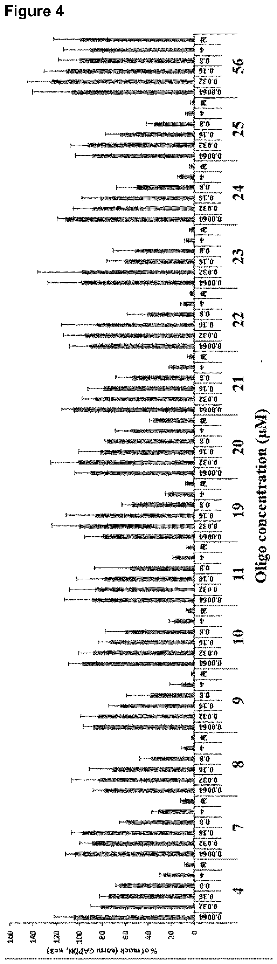

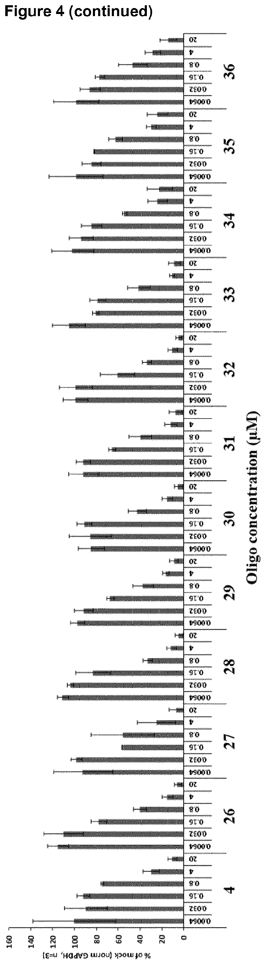

In another experiment, IC₅₀ values and effect on expression of BCL11A mRNA at various concentrations (ranging from 0.0064 to 20 µM) for selected antisense oligonucleotides was determined using human REH cells as described above. Exemplary results are shown in Table 5 (IC₅₀) and Figure 4 (BCL11A mRNA). Oligo # 56: antisense oligonucleotide that does not target BCL11A mRNA.

**TABLE 5**

| Oligo # | IC₅₀ (µM) |
|---|---|
| 4 | 1.5 |
| 7 | 1.5 |
| 8 | 0.3 |
| 9 | 0.3 |
| 10 | 0.8 |
| 11 | 0.8 |
| 19 | 1.0 |
| 20 | 6.0 |
| 21 | 0.8 |
| 22 | 0.6 |
| 23 | 0.5 |
| 24 | 0.7 |
| 25 | 0.3 |
| 26 | 0.6 |
| 27 | 0.9 |
| 28 | 0.5 |
| 29 | 0.4 |
| 30 | 0.7 |
| 31 | 0.4 |
| 32 | 0.3 |
| 33 | 0.5 |
| 34 | 1.2 |
| 35 | 1.6 |
| 36 | 0.8 |

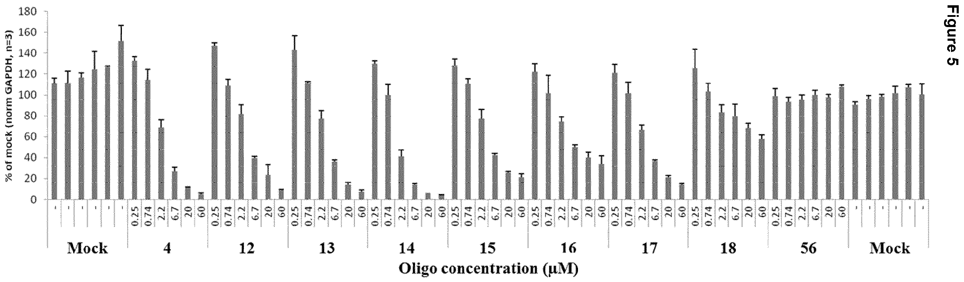

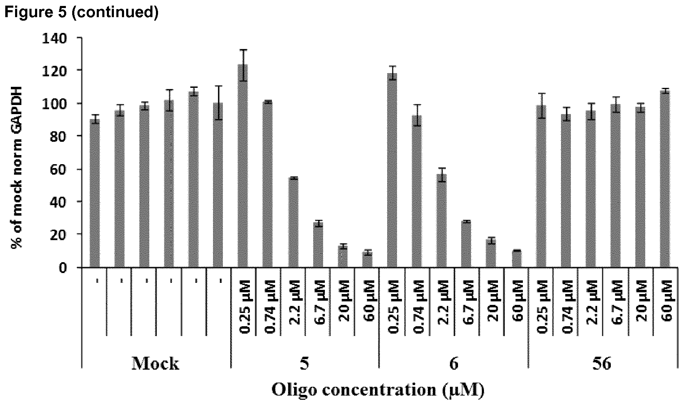

In a similar experiment, IC₅₀ values and effect on expression of BCL11A mRNA at various concentrations (ranging from 0.25 to 60 µM) for different oligonucleotides designed from oligos 4 and 5 was determined using human REH cells as described above. Exemplary results are shown in Table 6 (IC₅₀) and Figure 5 (BCL11A mRNA). Oligo # 56: antisense oligonucleotide that does not target BCL11A mRNA; Mock: no antisense oligonucleotide added to cells.

**TABLE 6**

| Oligo # | IC₅₀ (µM) |
|---|---|
| 4 | 3.9 |
| 12 | 6.2 |
| 13 | 5.1 |
| 14 | 2.0 |
| 15 | 7.1 |
| 16 | 12.6 |
| 17 | 5.1 |
| 18 | 74.8 |
| 5 | 3.2 |
| 6 | 3.4 |

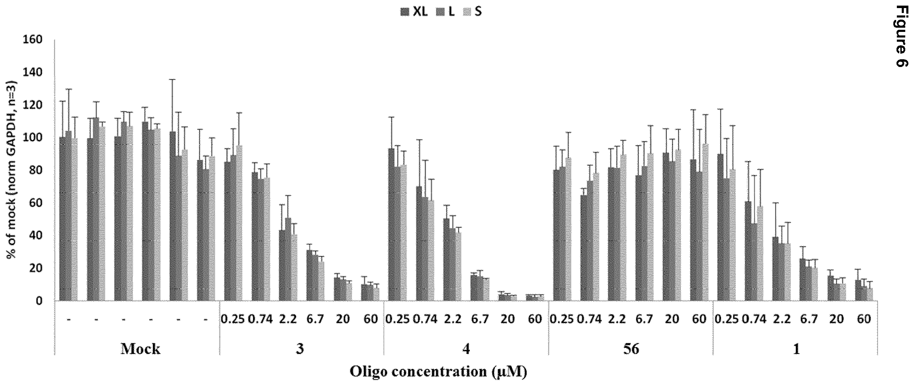

In another experiment, IC₅₀ values and effect on the expression of the different isoforms of BCL11A mRNA at various concentrations (ranging from 0.25 to 60 µM) for selected oligonucleotides was determined using human REH cells as described above. Exemplary results are shown in Table 7 (IC₅₀) and Figure 6 (isoform BCL11A mRNA). Oligo # 56: antisense oligonucleotide that does not target BCL11A mRNA; Mock: no antisense oligonucleotide added to cells.

**TABLE 7**

| Oligo # | IC₅₀ (µM) | | |
|---|---|---|---|
| | XL | L | S |
| 3-03 | 2.3 | 2.4 | 2.0 |
| 4-03 | 1.9 | 1.4 | 1.3 |
| 1 | 1.6 | 0.9 | 1.2 |

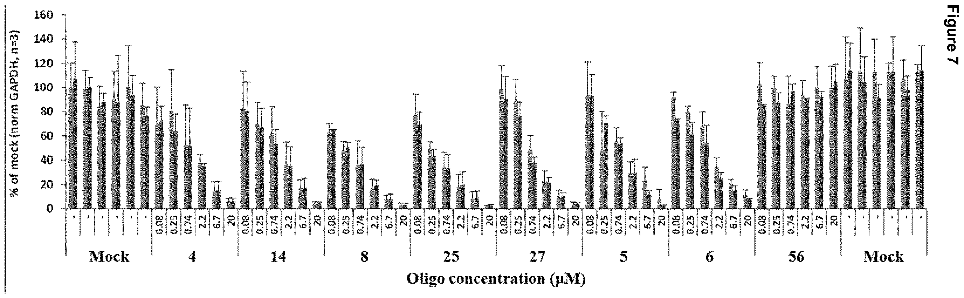

In another similar experiment, IC₅₀ values and effect on expression of BCL11A mRNA at various concentrations (ranging from 0.08 - 20 µM) for selected oligonucleotides was determined using mouse MPC-11 cells using similar experimental conditions as described above for human REH cells. Exemplary results are shown in Table 8 (IC₅₀) and Figure 7 (BCL11A mRNA). Oligo # 56: antisense oligonucleotide that does not target BCL11A mRNA; Mock: no antisense oligonucleotide added to cells.

**TABLE 8**

| Oligo # | IC50 (µM) BCL11A-All |
|---|---|
| 4 | 0.8 |
| 14 | 1.0 |
| 8 | 0.2 |
| 25 | 0.3 |
| 27 | 0.8 |
| 5 | 0.7 |
| 6 | 1.4 |

In yet another experiment, IC₅₀ values for different oligonucleotides designed from oligos 8, 25 and 27 were determined using human REH cells as described above. Typically, IC₅₀ values were determined using six-point 5x dilutions ranging from 0.0064 to 20 µM. Exemplary results are shown in Table 9.

**TABLE 9**

| Oligo # | Design | IC₅₀ (µM) |
|---|---|---|
| 8 | 3-10-3 | 1.0 |
| 37 | 2-11-3 | 2.0 |
| 38 | 3-9-4 | 1.3 |
| 39 | 4-9-3 | 0.6 |
| 40 | 4-8-4 | 1.1 |
| 41 | 3-8-5 | 0.9 |
| 42 | 5-8-3 | 1.8 |
| | | |
| 25 | 3-9-3 | 0.7 |
| 43 | 2-10-3 | 0.9 |
| 44 | 3-10-2 | 0.3 |
| 45 | 4-9-2 | 0.3 |
| 46 | 2-9-4 | 0.4 |
| 47 | 4-8-3 | 0.3 |
| 48 | 3-8-4 | 0.6 |
| | | |
| 27 | 3-8-3 | 0.5 |
| 49 | 3-8-3^{m}c | 0.4 |
| 50 | 2-10-2 | 0.8 |
| 51 | 2-9-3 | 0.4 |
| 52 | 3-9-2 | 0.5 |
| 53 | 4-8-2 | 0.4 |
| 54 | 2-8-4 | 1.8 |
| 55 | 4-7-4 | 11.4 |

Taken together, these data show that antisense oligonucleotides provided by the present invention such as those described in Example 1 can effectively inhibit BCL11A with a typical IC₅₀ ranging between 0.25 µM-60 µM. In addition, selected antisense oligonucleotides provided by the present invention can effectively inhibit mouse BCL11A with a typical IC₅₀ ranging between 0.10 µM-1.5 µM.

### Example 3. In vivo tolerance of oligonucleotides

The oligonucleotides described in the prior examples were tested for their *in vivo* tolerability using NMRI mice.

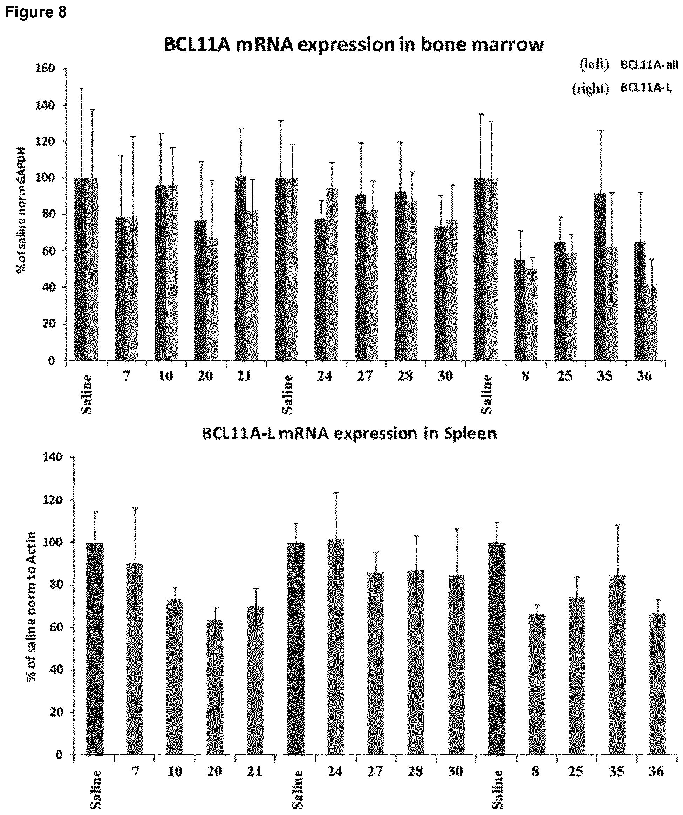

Briefly, female NMRI mice (n=5 per group) were dosed at 0, 3, 7, 10 and 14 days with either saline (control) or a selected LNA oligonucleotide (15 mg/kg) via intravenous administration. Mice were sacrificed 48 hours after the final dose. The following parameters were recorded for each animal in each group: body weight (day 0, day 5, 6 or 7, and day 10, 13, 14 or 16), organ (liver, kidney and spleen) weight at sacrifice, serum alanine aminotransferase (ALT) activity, and BCL11A mRNA expression in whole bone marrow and spleen. Exemplary results are shown in Figures 8.

As shown in Figure 8 the ability of selected antisense oligonucleotides to inhibit BCL11A mRNA expression in mice was confirmed in harvested bone marrow and spleen. For example, oligos 8 and 25 demonstrated about 40% reduction of BCL11A mRNA in bone marrow, while oligos 8 and 20 demonstrated about the same reduction of BCL11A mRNA in spleen. Generally, inhibition of BCL11A mRNA expression in bone marrow ranged on average from about 10 - 50%, whereas inhibition of BCL11A mRNA expression in spleen ranged on average from about 10 - 40%. Typically, body and organ weights of treated animals were unaffected.

Taken together, these data show that antisense oligonucleotides provided by the present invention such as those described in Example 1 are well tolerate and can effectively inhibit BCL11A mRNA expression in various target tissues *in vivo,* including but not limited to, bone marrow, spleen.

In a similar experiment, selected antisense oligonucleotides were tested as described above for their *in vivo* tolerability using NMRI mice. Typically, body and organ weights of mice administered selected antisense oligonucleotides that target BCL11A were typically unaffected. Serum ALT levels for mice administered selected antisense oligonucleotides demonstrated similar results as compared to the saline group.

In a similar experiment, selected antisense oligonucleotides were tested for *in vivo* tolerability using Wistar rats. Briefly, male Wistar rats (n=5 per group) were dosed once per week (day 0, 7, 14, 21 and 28) with either saline (control) or a selected antisense oligonucleotide (25 mg/kg) via subcutaneous administration. Rats were sacrificed at day 30. The following parameters were recorded for each animal in each group: bodyweight during study, organ (liver, kidney and spleen) weight at sacrifice, liver and kidney histopathology, and clinical serum chemistry (alanine aminotransferase, asparatate aminotransferase, alkaline phosphatase, bilirubin, urea and creatinine).

Organ (e.g., liver, kidney and spleen) weights in Wistar rats administered selected antisense oligonucleotides that target BCL11A were typically unaffected. In addition, measurements for various clinical serum chemistry markers (e.g., ALT, AST, ALP, Bilirubin, Urea, Creatine) demonstrated results that were similar as compared to control groups.

Taken together, these data demonstrates that antisense oligonucleotides provided by the present invention such as those described in Example 1 are generally safe and well tolerated.

### Example 4. In vivo efficacy in wild-type and β-YAC transgenic mice

Wild-type and transgenic mice transgenic for the human β-globin gene (β-YAC) were used to determine the *in vivo* efficacy of several LNA oligonucleotides made according to the previous Examples.

Briefly, wild-type and β-YAC transgenic mice were dosed (25 or 15 mg/kg) via subcutaneous route with selected antisense oligonucleotides according to one of two schedules: (1) dosing at day 0, 3, 6, 13, 20 and 27, with day 29 designated for necropsy (sacrifice) and (2) dosing at day 0, 3, 6, 13, 20, 27, 34, 41, 48, and 55, with day 57 designated for necropsy (sacrifice). For both dosing schedules, bleeds were take prior to day 0 and at necropsy (day 29 or 57, respectively). Endpoints used in this study included BCL11A knockdown in target tissue (*e.g*., bone marrow) as well as blood chemistry and biodistribution of oligonucleotides.

Consistent with the results shown Example 3, there was no adverse effect on body weight up to 58 days of treatment with various antisense oligonucleotides for both wild-type and transgenic mice. No significant differences were observed in AST levels among treatment groups.

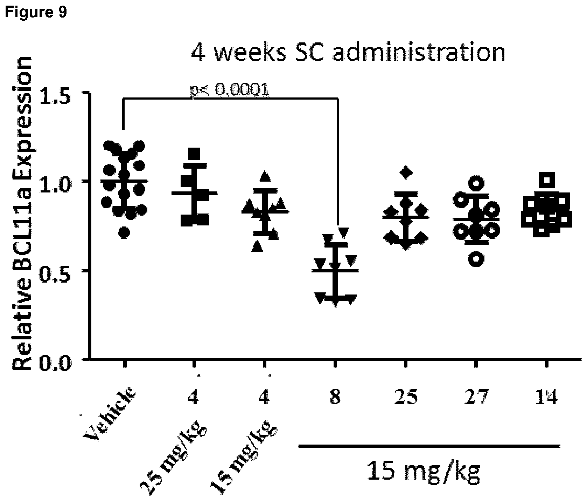

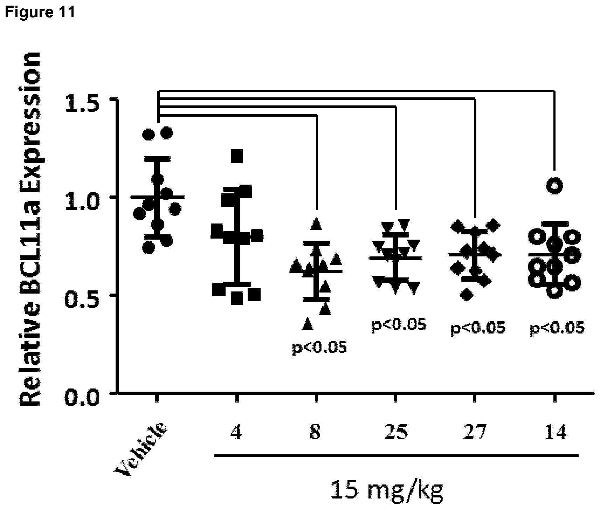

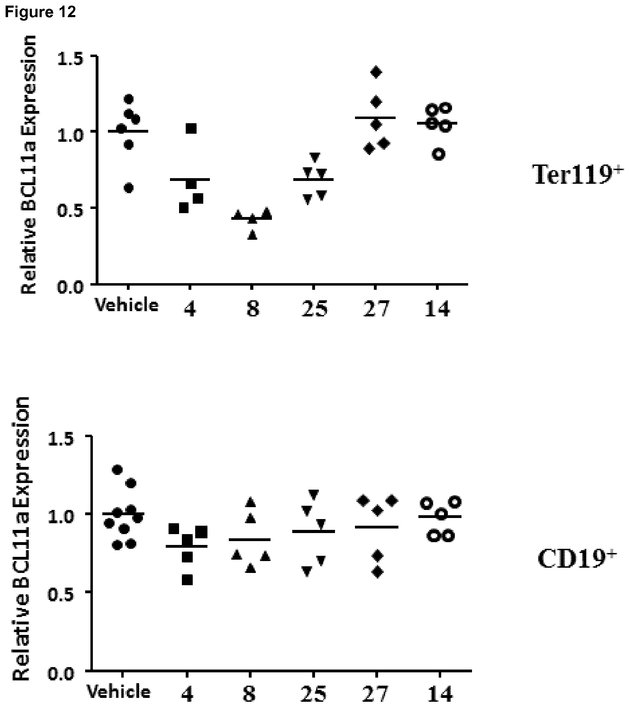

Exemplary results for knockdown of BCL11A mRNA expression in bone marrow of wild-type mice are set forth in Figure 9 (four weeks post administration) and 10 (eight weeks post administration). Exemplary results for knockdown of BCL11A mRNA expression in β-YAC transgenic mice are set forth in Figure 11. Exemplary results for knockdown of BCL11A mRNA expression in Ter119⁺ and CD19⁺ bone marrow cells of β-YAC transgenic mice eight weeks post administration are set forth in Figure 12.

As shown in the above results, knockdown of BCL11A mRNA expression was greater at eight weeks, however, candidate oligo 8 demonstrated the greatest decrease in BCL11A among the oligonucleotides tested. No difference in knockdown of BCL11A expression was observed for candidate oligo 4 when dosed at 15 or 25 mg/kg. Further, no differences in knockdown of BCL11A expression for the selected oligonucleotides was observed for either wild-type or transgenic mice when administered for eight weeks.

Taken together, this example demonstrates that antisense oligonucleotides provided by the present invention can effectively inhibit BCL11A expression in various target tissues *in vivo,* including but not limited to, bone marrow, spleen.

### Example 5. Pharmacology Study in non-human primates

A pharmacological study employing an exemplary antisense oligonucleotides that specifically target one or more isoforms of BCL11A was performed to further confirm the *in* vivo safety and efficacy.

Briefly, the study covered a six to sixteen week time period, during which female cynomologus monkeys (ranging two to four years of age and 2.5 - 4 kg in weight) were administered six weekly doses at 20 mg/kg or twelve weekly doses at 10 mg/kg and 20 mg/kg of selected BCL11A-specific LNA antisense oligonucleotides or control (saline) via subcutaneous injection. The animals were sacrificed approximately seven days after the last dosage at approximately week seven or week 17 depending on the duration of the study as described above. As the study proceeds, half of the treatment groups were rendered moderately anemic due to repeated blood sampling during a pretesting phase of the study as well as throughout the dosing period to stimulate erythropoiesis. The experimental design is set forth in Table 10.

**TABLE 10**

| Group | Dose Level (mg/kg) | No. of animals | | Phlebotomy |
|---|---|---|---|---|
| | | Interim sacrifice (week 7) | Final sacrifice (week 17) | |
| Control | 0 | 4 | 4 | - |
| Low dose | 10 | - | 4 | - |
| High dose | 20 | 4 | 4 | - |
| Control | 0 | 4 | 4 | + |
| Low dose | 10 | - | 4 | + |
| High dose | 20 | 4 | 4 | + |

| | | | | |
|---|---|---|---|---|
| Weekly s.c. administration (6 or 16 doses) Sacrifice seven days after last dose (week 7 or week 17) | | | | |

Pharmacodynamic biomarkers: Peripheral blood (every second week) and bone marrow (at necropsy, week seven and 17 according to study design) were sampled and used for determination of BCL11A and γ-globin mRNA expression, as well as fetal hemoglobin (HbF) and γ-globin protein levels by an ELISA assay. HbF levels in bone marrow was also analyzed using high-performance liquid chromatography (HPLC). F-cells were measured using the Kleihauer method. Bone marrow sampled at week seven was sampled from the humerus via live bone marrow aspiration. Bone marrow sampled at week 17 was sampled from the humerus and femur bones using multiple methodology. Sampling at week 17 from the humerus bone was performed via live bone marrow aspiration. Sampling at week 17 from the femur was performed via flushing of bone marrow with buffer followed by centrifugation and analysis of the resulting pellet. Sampling at week 17 from the femur was also performed from whole frozen femur.

Hematology analysis included counts of red blood cells, reticulocytes and total hemoglobin measured from samples every two weeks.

Peripheral blood was sampled for pharmacokinetic analysis at two, four, eight, 24 and 48 hours post first and week 12 dose (only week 17 groups) of LNA antisense oligonucleotide in week groups following the 16 week study design only.

At necropsy (week 7 and week 17), liver, kidney and bone marrow were sampled for analysis and weight measurements. Clinical chemistry analysis was also performed at necropsy.

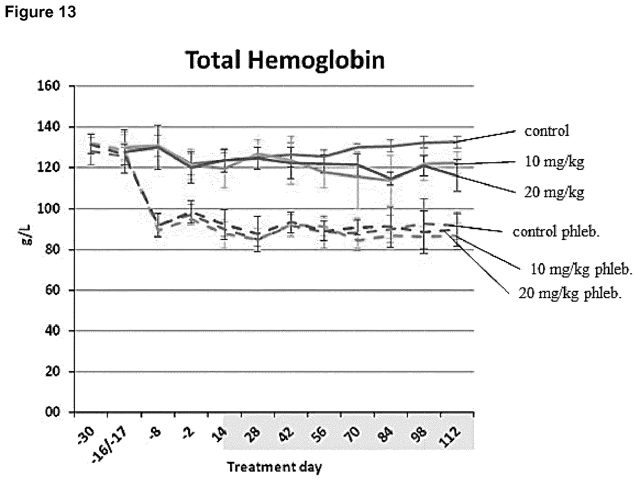

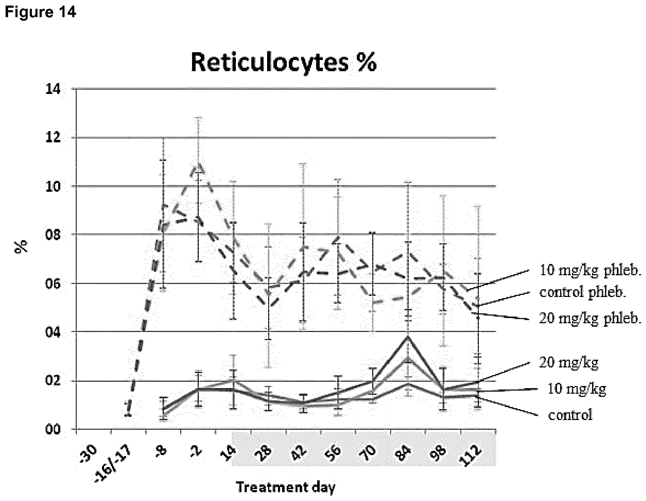

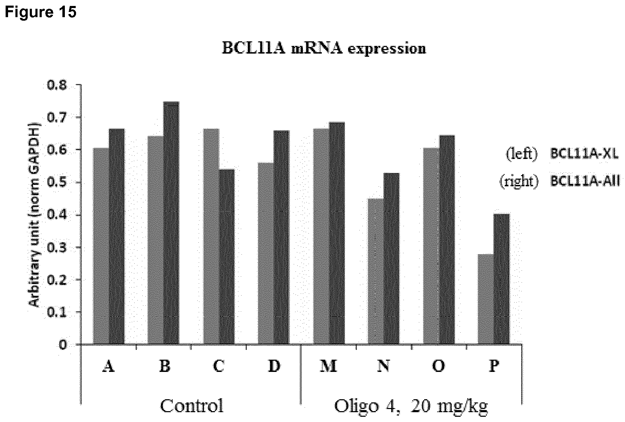

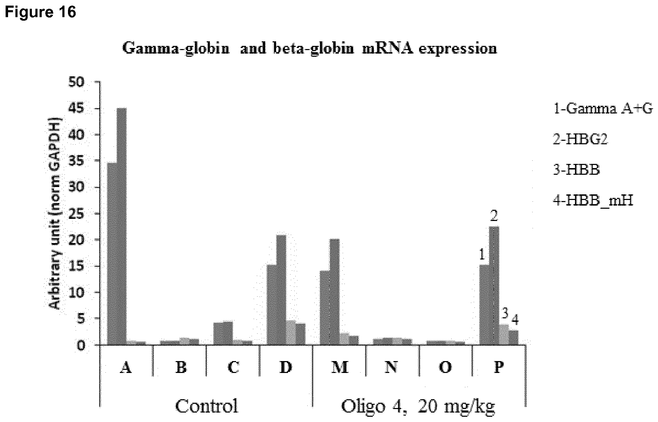

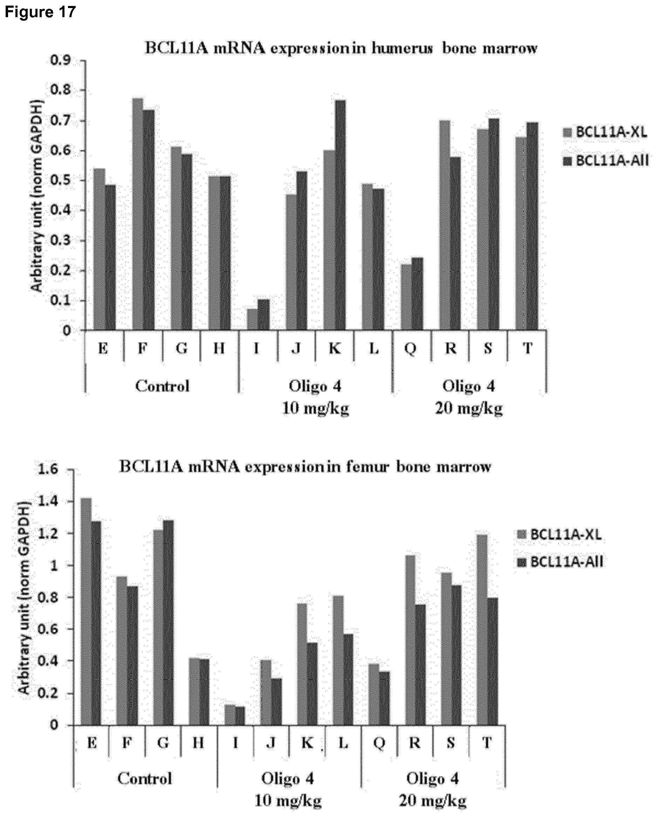

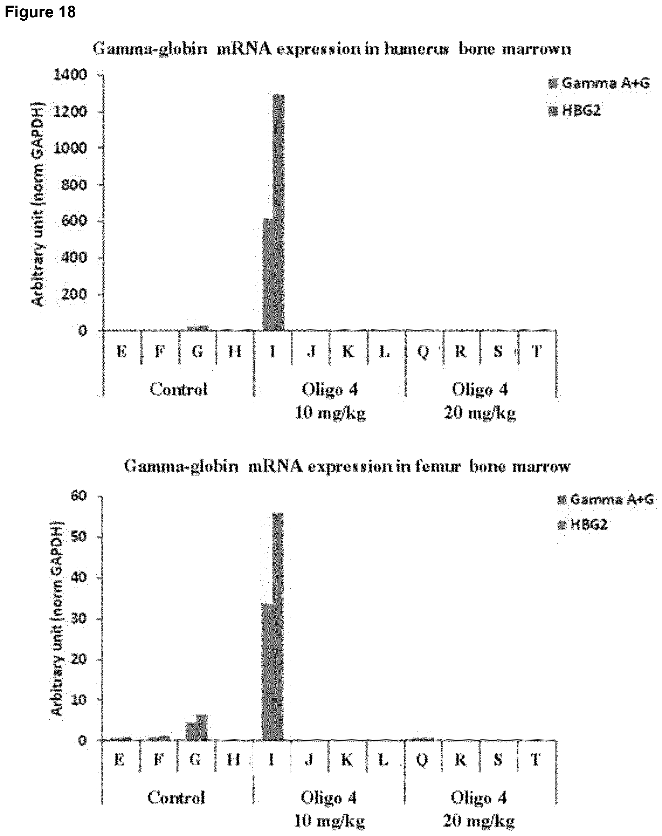

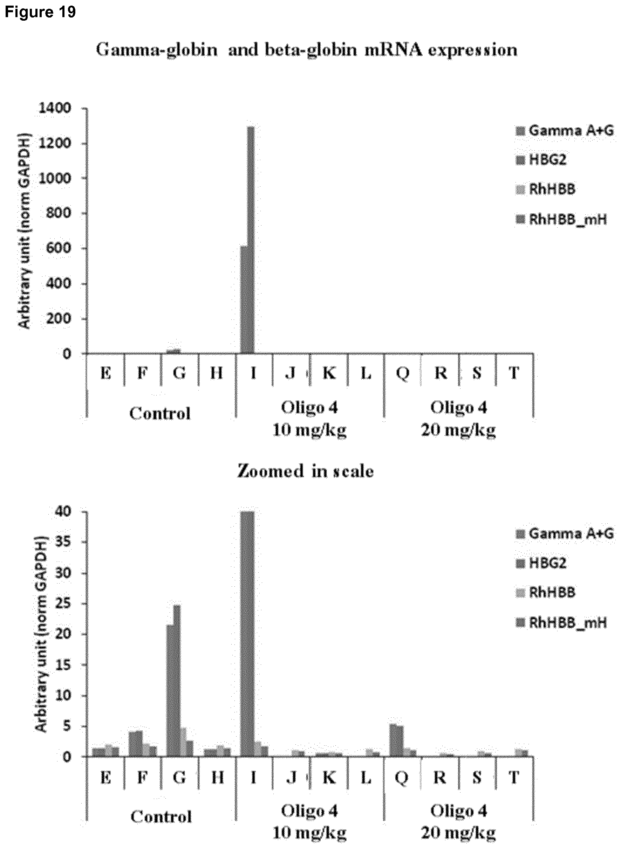

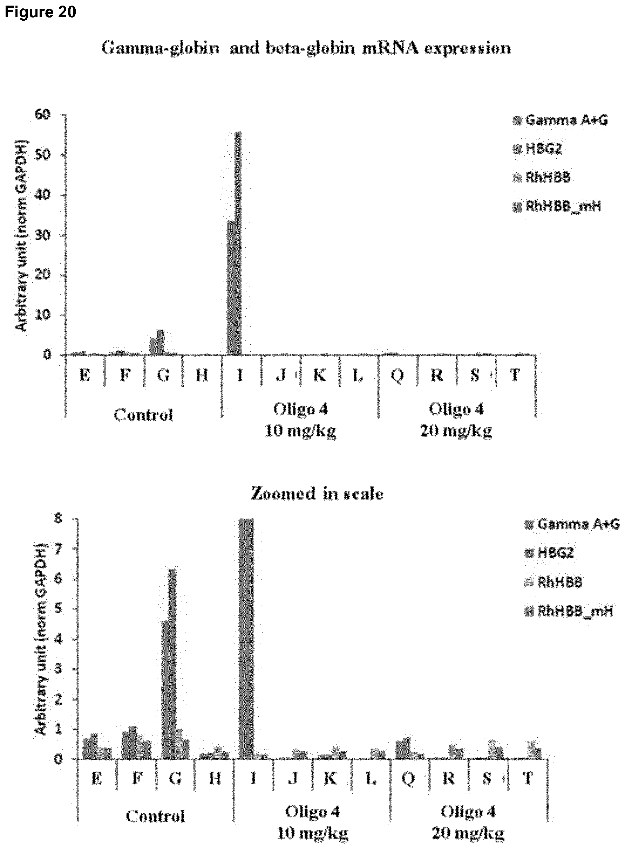

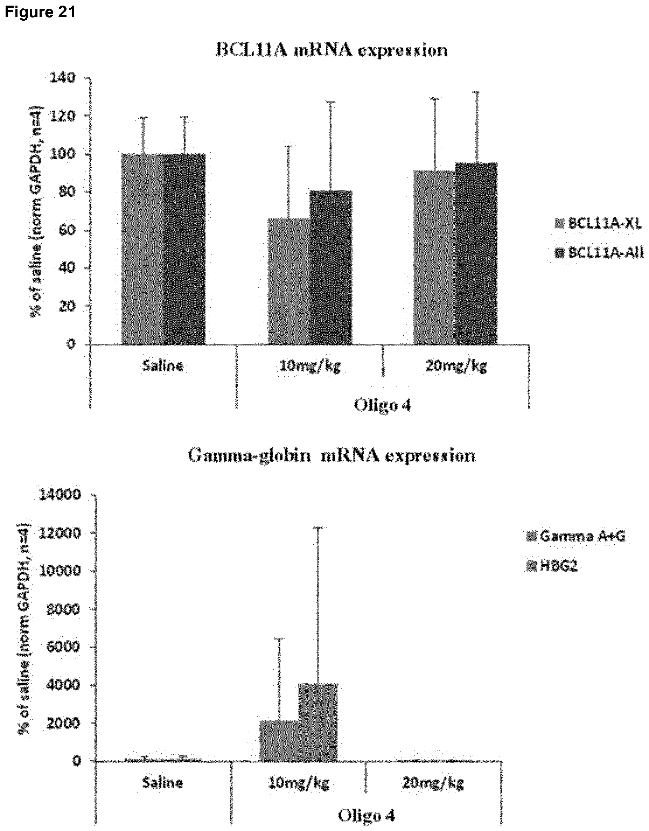

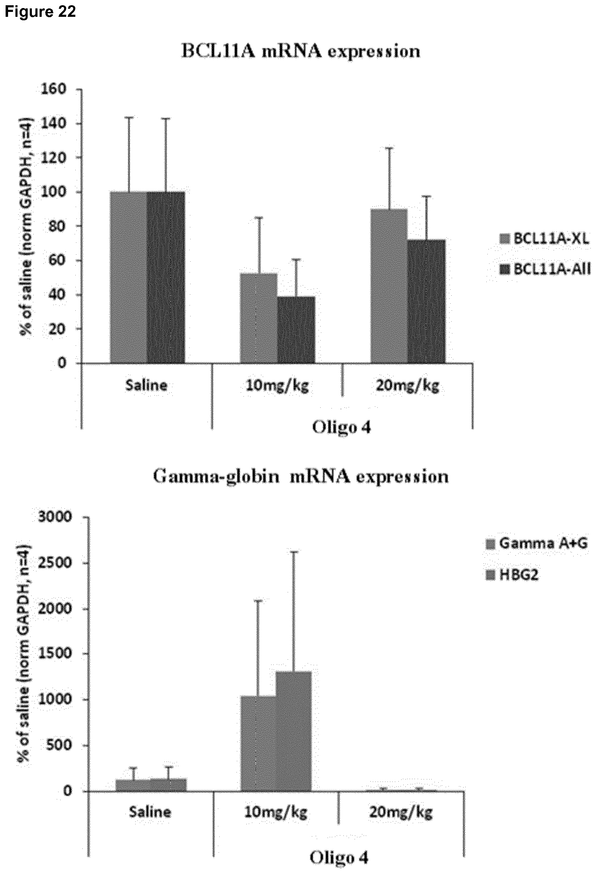

Exemplary total hemoglobin measurements from peripheral blood are shown in Figure 13. Exemplary percentage of reticulocytes in peripheral blood are shown in Figure 14. Exemplary measurements of BCL11A mRNA in humerus bone marrow by RT-qPCR at week seven are shown in Figure 15. Exemplary measurements of γ-globin and β-globin mRNA in humerus bone marrow by RT-qPCR at week seven are shown in Figure 16. Exemplary measurements of BCL11A mRNA in humerus (top) and femur (bottom) bone marrow by RT-qPCR at week 17 are shown in Figure 17. Exemplary measurements of γ-globin mRNA in humerus (top) and femur (bottom) bone marrow by RT-qPCR at week 17 are shown in Figure 18. Exemplary measurements of γ-globin and β-globin mRNA in humerus bone marrow by RT-qPCR at week 17 are shown in Figure 19. Exemplary measurements of γ-globin and β-globin mRNA in femur bone marrow by RT-qPCR at week 17 are shown in Figure 20. Exemplary average measurements of BCL11A (top) and γ-globin (bottom) mRNA in humerus bone marrow by RT-qPCR at week 17 are shown in Figure 21. Exemplary average measurements of BCL11A (top) and γ-globin (bottom) mRNA in femur bone marrow by RT-qPCR at week 17 are shown in Figure 22.

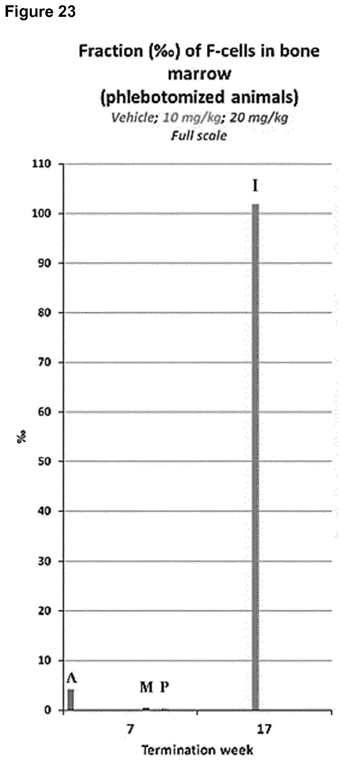

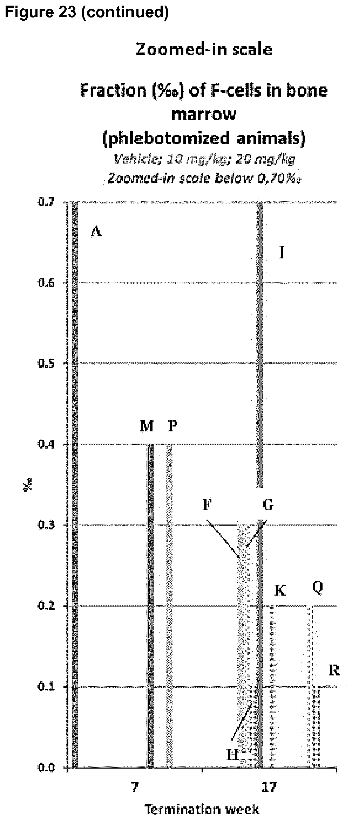

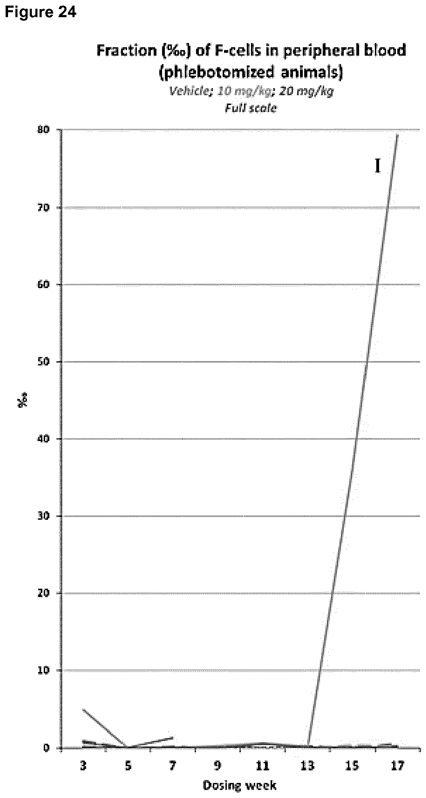

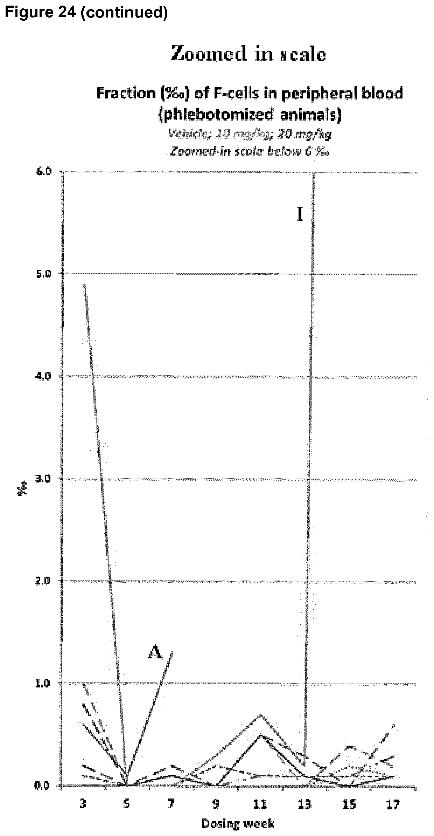

Exemplary measurements of fraction (‰) of F-cells in bone marrow for selected phlebotomized animals at full scale (left) and zoomed-in scale (right) are shown in Figure 23. Exemplary measurements of fraction (‰) of F-cells in peripheral blood for selected phlebotomized animals at full scale (left) and zoomed-in scale (right) are shown in Figure 24.

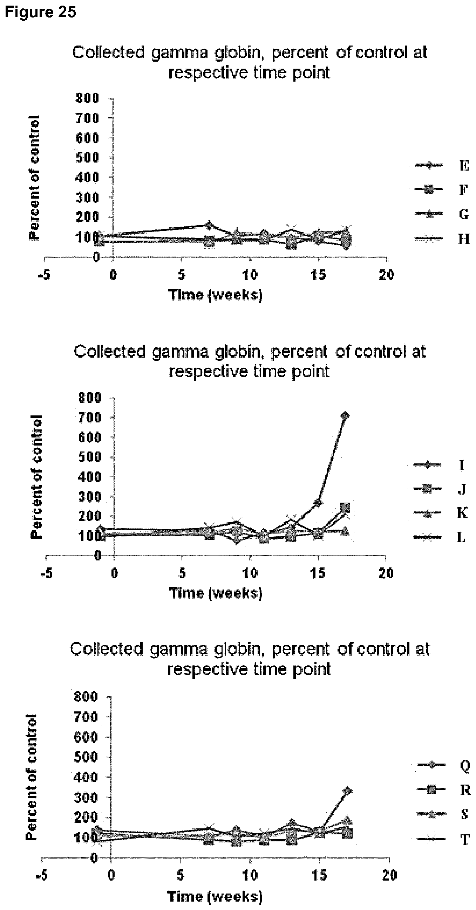

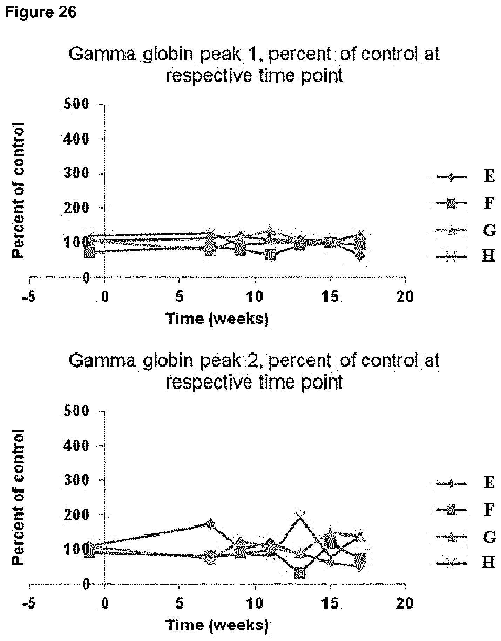

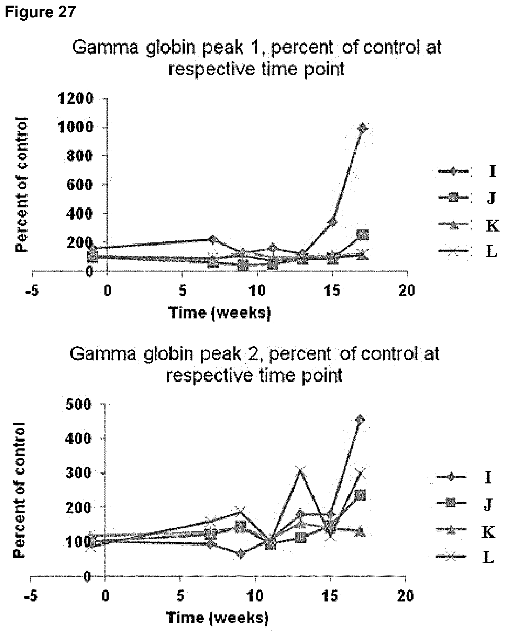

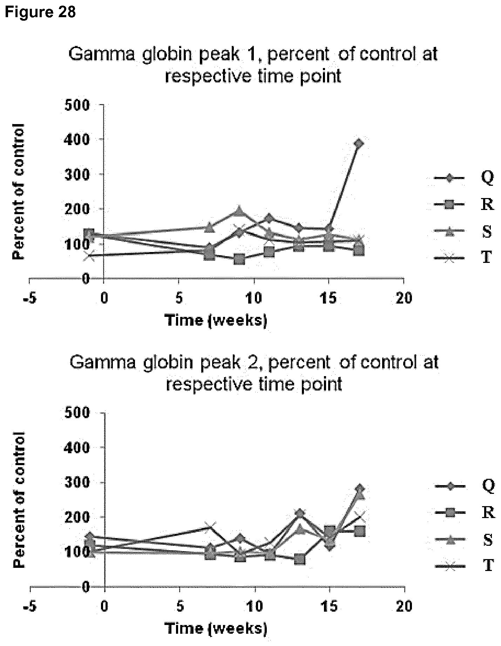

Exemplary measurements of γ-globin in peripheral blood in control (top), 10 mg/kg (middle), and 20 mg/kg (bottom) dose groups are shown in Figure 25. Exemplary measurements of γ-globin in peripheral blood as a percent of control at a respective time point of a γ-globin peak ("peak 1" or "peak 2") for control, 10 mg/kg and 20 mg/kg dose groups are shown in Figures 26, 27 and 28, respectively.

As shown in the above results, about a two-fold higher expression of BCL11A was observed in bone marrow samples from femurs as compared to humerus bones. For γ-globin expression, a two- to three-fold higher expression was observed in bone marrow samples from humerus as compared to femurs. The greatest differences were observed in certain particular animals as described below.

For measurements of F-cells in bone marrow, animal I demonstrated about 10‰ F-cells at week 17 as compared to about 0.2‰ in control animals. For measurements of F-cells in peripheral blood, animal I demonstrated about 8‰ at week 17 as compared to about 0.3‰ in control animals. Further, F-cells in this animal began to increase at about week 15 in measurements from samples obtained from peripheral blood.

For measurements of γ-globin in peripheral blood, an increase was observed at week 15 for animal I with a further increase at week 17 in the 10 mg/kg dose group. In a similar fashion, animal Q demonstrated an increase at week 17 in the 20 mg/kg dose group.

In non-phlebotomy groups, no reduction in BCL11A mRNA expression was observed as compared to control groups. Likewise, no increase in F-cells or HbF (γ-globin) was observed for any of the animals.

In summary, experimental results described in this example demonstrate effective target engagement in animal I by greater than 85% knockdown of BCL11A mRNA in bone marrow (humerus and femur) as compared to control animals and by about 60% knockdown of BCL11A mRNA in animal Q in bone marrow (humerus and femur) as compared to control animals. Further, greater than 80-fold induction of γ-globin mRNA expression in bone marrow of animal I as compared to control animals, and a seven-fold increase in γ-globin protein in peripheral blood of animal I as compared to controls were recorded. Animal Q demonstrated about three-fold increase in γ-globin protein in peripheral blood as compared to control animals. Animal I also demonstrated an increase in F-cells in bone marrow and peripheral blood as compared to control animals.

Taken together, this example demonstrates that antisense oligonucleotides provided by the present invention can effectively inhibit BCL11A expression in various target tissues *in vivo* and increase γ-globin protein in peripheral blood by at least two-fold or more as compared to vehicle control.

### Example 6. In vivo pharmacokinetics

This example determines the *in vivo* pharmacokinetics of selected LNA oligonucleotides made according to the previous Examples.

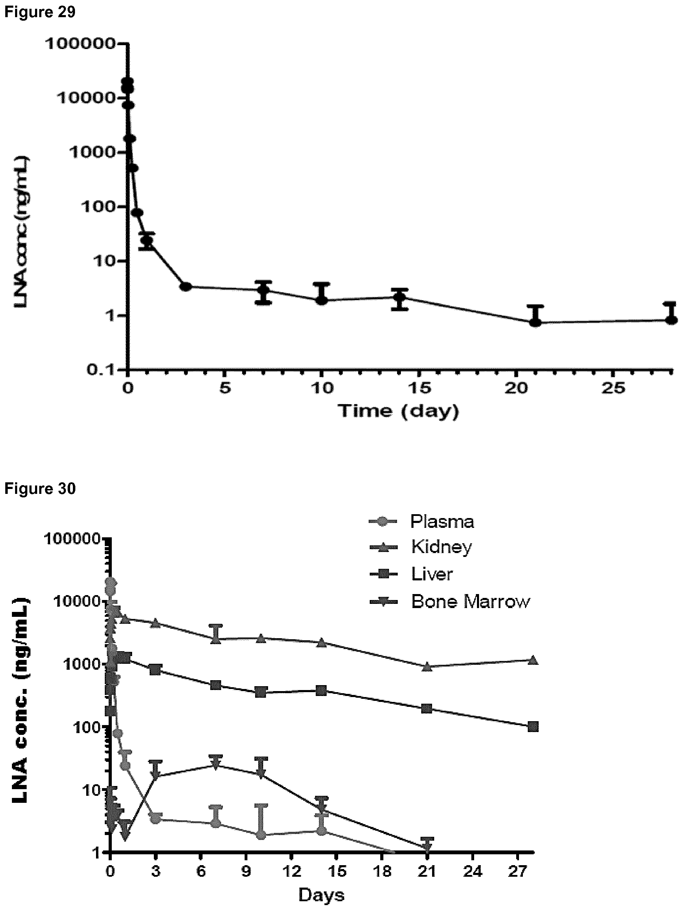

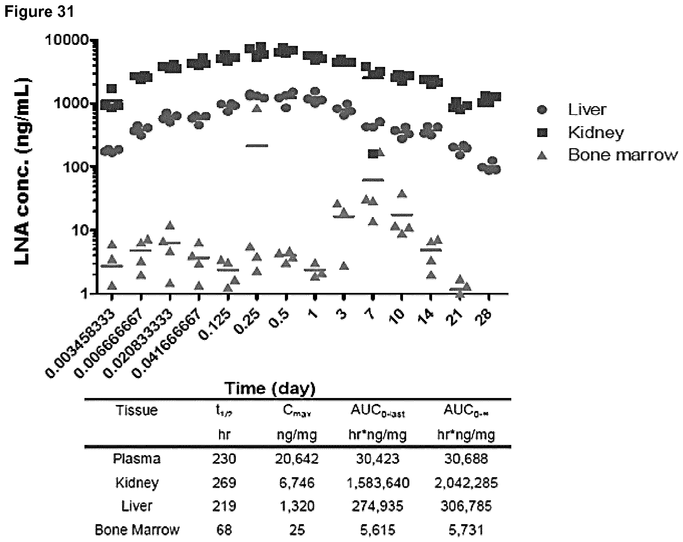

Briefly, wild-type mice were given a single dose (20 mg/kg) via subcutaneous route with selected antisense oligonucleotides. Sampling of plasma, liver, kidney and bone marrow were takine at several time points up to 28 days. The pharmacokinetic profile for each tissue sampled was determined. Exemplary results are shown in Figures 29-31.

The results demonstrated rapid distribution and observable distribution of antisense oligonucleotides to all sampled tissues, including bone marrow. The Cₘₐₓ was about 21 µg/mL at ten minutes post subcutaneous administration. Liver, kidney and plasma t_{1/2β} was about ten days. In the bone marrow, t_{1/2β} was about three days.

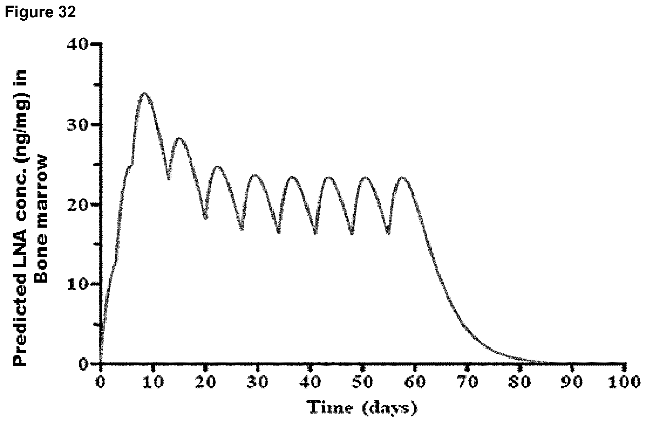

From this pharmacokinetic study, a predictive model for bone marrow exposure to antisense oligonucleotides was determined (Figure 32).

Taken together, this example demonstrates that antisense oligonucleotides provided by the present invention are effectively and safely absorbed by multiple tissues upon administration (e.g., subcutaneous). Further, antisense oligonucleotides provided by the present invention are distributed to multiple target tissues, including bone marrow, without any adverse effects.

Having thus described several aspects of at least one embodiment of this invention, it is to be appreciated that various alterations, modifications, and improvements will readily occur to those skilled in the art. Such alterations, modifications, and improvements are intended to be part of this disclosure, and are intended to be within the spirit and scope of the invention. Accordingly, the foregoing description and drawings are by way of example only and the invention is described in detail by the claims that follow.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification.

## Claims

1. An antisense oligonucleotide capable of decreasing expression of human BCL11A comprising a sequence that is at least 80% identical to the reverse complement of a continuous sequence within a region selected from nucleotides 410 to 450 of the human BCL11A gene of SEQ ID NO 1 or a messenger RNA (mRNA) isoform of BCL11A, wherein the antisense oligonucleotide is a gapmer.

2. The antisense oligonucleotide according to claim 1, wherein the antisense oligonucleotide is represented by the formula X*ₐ*-Y*_{b}*-X*_{a',}* wherein:
X is a nucleotide analogue;
Y is a continuous sequence of DNA;
*a* is 1, 2, 3, 4 or 5;
*a*' is 1, 2, 3, 4 or 5; and
*b* is an integer number between 5 and 15.

3. The antisense oligonucleotide according to claim 2, wherein *a* and/or *a*' is between 2 and 4.

4. The antisense oligonucleotide according to any one of claim 2 or 3, wherein b is an integer number between 7 and 10.

5. The antisense oligonucleotide according to any one of the preceding claims, wherein the antisense oligonucleotide is less than 19 nucleotides in length, such as 10 to 16 nucleotides in length.

6. The antisense oligonucleotide according to any one of the preceding claims, wherein the oligonucleotide comprises at least one nucleotide analogue selected from the group consisting of 2'-O-alkyl-RNA units, 2'-OMe-RNA units, 2'-O-alkyl-DNA, 2'-amino-DNA units, 2'-fluoro-DNA units, LNA units, arabino nucleic acid (ANA) units, 2'-fluoro-ANA units, HNA units, INA units and 2'MOE units.

7. The antisense oligonucleotide according to claim 6, wherein the nucleotide analogue is a LNA unit selected from the group consisting of beta-D-oxy-LNA, alpha-L-oxy-LNA, beta-D-amino-LNA, alpha-L-amino-LNA, beta-D-thio-LNA, alpha-L-thio-LNA, 5'-methyl-LNA, beta-D-ENA. cET and alpha-L-ENA.

8. The antisense oligonucleotide according to any of the preceding claims, wherein the oligonucleotide comprise at least one phosphorothioate linkage.

9. The antisense oligonucleotide according to any of the preceding claims, wherein the oligonucleotide is capable of recruiting an RNAaseH.

10. The antisense oligonucleotide according to any one of the preceding claims, wherein the antisense oligonucleotide comprises an oligonucleotide sequence motif selected from the group consisting of SEQ ID NO: 63, SEQ ID NO: 64, SEQ ID NO: 65, SEQ ID NO: 66 SEQ ID NO: 67, SEQ ID NO: 68, SEQ ID NO: 69, SEQ ID NO: 70, SEQ ID NO: 71, SEQ ID NO: 72, SEQ ID NO: 73 and SEQ ID NO: 74.

11. The antisense oligonucleotide according to any one of the preceding claims, wherein the antisense oligonucleotide has a sequence selected from SEQ ID NO: 11, SEQ ID NO: 15, SEQ ID NO: 32, SEQ ID NO: 21, SEQ ID NO: 34, SEQ ID NO:10, SEQ ID NO: 12, SEQ ID NO: 13, SEQ ID NO: 14, SEQ ID NO: 16, SEQ ID NO: 17, SEQ ID NO: 18, SEQ ID NO: 19, SEQ ID NO: 20, SEQ ID NO: 22, SEQ ID NO: 23, SEQ ID NO: 24, SEQ ID NO: 25, SEQ ID NO: 26, SEQ ID NO:27, SEQ ID NO: 28, SEQ ID NO: 29, SEQ ID NO: 30 SEQ ID NO: 31, SEQ ID NO: 33, SEQ ID NO: 35, SEQ ID NO:: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 60, SEQ ID NO: 61 or SEQ ID NO: 62.

12. The antisense oligonucleotide according to any one of the preceding claims, wherein the antisense oligonucleotide is 5'- ^{m}**C**ₛ° **A**ₛ° **T**ₛ° tₛ gₛ cₛ aₛ tₛ tₛ gₛ tₛ tₛ tₛ ^{m}**C**ₛ° ^{m}**C**ₛ° **G**°-3' (SEQ ID NO: 11), 5' - ^{m}**C**ₛ° **G**ₛ° **T**ₛ° tₛ tₛ gₛ tₛ gₛ Cₛ tₛ ^{m}Cₛgₛaₛ**T**ₛ°**A**ₛ°**A**°- 3' (SEQ I D NO: 15), 5' - ^{m}**C**ₛ° **G**ₛ° **T**ₛ° tₛ tₛ gₛ tₛ gₛ cₛ tₛ ^{m}Cₛ gₛ **A**ₛ° Tₛ° **A**°- 3' (SEQ ID NO: 32) 5' - **T**ₛ° **T**ₛ° **G**ₛ° tₛ gₛ cₛ tₛ ^{m}cₛ gₛ aₛ tₛ **A**ₛ° **A**ₛ° **A**°-3' (SEQ ID NO: 14) or 5' - ^{m}**C**ₛ°**G**ₛ°**T**ₛ° tₛ tₛ gₛ ts gₛ Cₛ ts cₛ **G**ₛ° **A**ₛ° **T**° - 3' (SEQ ID NO: 35), wherein upper case letters indicate locked nucleic acid (LNA) units, subscript "s" represents phosphorothioate linkage, and lower case letters represent deoxyribonucleotide (DNA) units, "^{m}C" represents 5' methyl-cytosine LNA unit, and "^{m}c" represents 5' methyl-cytosine DNA unit.

13. A pharmaceutical composition comprising the antisense oligonucleotide according to any one of claims 1 to 12 and a pharmaceutically acceptable carrier.

14. The antisense oligonucleotide according to any one of claims 1 to 12 or pharmaceutical composition claim 13, for use as a medicament, such as for the treatment of an anemic disease, disorder or condition, such as sickle cell disease or β-thalassemia.

15. The oligonucleotide according to any one of claims 1 to 12 or a pharmaceutical composition of claim 13 for use in the treatment of BCL11A related diseases, disorders and conditions.

16. The oligonucleotide according to any one of claims 1 to 12 or a pharmaceutical composition of claim 13 for use in the treatment of an anemic disease, disorder or condition.

17. The oligonucleotide for the use according to claim16, wherein the anemic disease, disorder or condition is sickle cell disease or β-thalassemia.

## Patentansprüche

1. Antisense-Oligonukleotid, das in der Lage ist, die Expression von humanem BCL11 A zu verringern, das eine Sequenz umfasst, die mindestens zu 80 % mit dem umgekehrten Komplement einer kontinuierlichen Sequenz innerhalb eines Bereiches identisch ist, der aus Nukleotiden 410 bis 450 des humanen BCL11A-Gens der SEQ-ID-NR. 1 oder einer Boten-RNA-(mRNA)-Isoform von BCL11A ausgewählt ist, wobei das Antisense-Oligonukleotid ein Gapmer ist.

2. Antisense-Oligonukleotid nach Anspruch 1, wobei das Antisense-Oligonukleotid durch die Formel X*ₐ*-Y*_{b}*-X*_{a'}* dargestellt wird, wobei:
X ein Nukleotidanalogon ist;
Y eine kontinuierliche Sequenz von DNA ist;
*a* 1, 2, 3, 4 oder 5 ist;
*a'* 1, 2, 3, 4 oder 5 ist; und
*b* eine ganze Zahl zwischen 5 und 15 ist.

3. Antisense-Oligonukleotid nach Anspruch 2, wobei *a* und/oder *a'* zwischen 2 und 4 liegt.

4. Antisense-Oligonukleotid nach einem der Ansprüche2 oder 3, wobei b eine ganze Zahl zwischen 7 und 10 ist.

5. Antisense-Oligonukleotid nach einem der vorangehenden Ansprüche, wobei die Länge des Antisense-Oligonukleotids weniger als 19 Nukleotide beträgt, wie etwa eine Länge von 10 bis 16 Nukleotiden.

6. Antisense-Oligonukleotid nach einem der vorangehenden Ansprüche, wobei das Oligonukleotid mindestens ein Nukleotidanalogon umfasst, ausgewählt aus der Gruppe bestehend aus 2'-O-Alkyl-RNA-Einheiten, 2'-OMe-RNA-Einheiten, 2'-O-Alkyl-DNA, 2'-Amino-DNA-Einheiten, 2'-Fluor-DNA-Einheiten, LNA-Einheiten, Arabinonukleinsäure-(ANA)-Einheiten, 2'-Fluor-ANA-Einheiten, HNA-Einheiten, INA-Einheiten und 2'MOE-Einheiten.

7. Antisense-Oligonukleotid nach Anspruch 6, wobei das Nukleotidanalogon eine LNA-Einheit ist, ausgewählt aus der Gruppe bestehend aus beta-D-Oxy-LNA, alpha-L-Oxy-LNA, beta-D-Amino-LNA, alpha-L-Amino-LNA, beta-D-Thio-LNA, alpha-L-Thio-LNA, 5'-Methyl-LNA, beta-D-ENA, cET und alpha-L-ENA.

8. Antisense-Oligonukleotid nach einem der vorangehenden Ansprüche, wobei das Oligonukleotid mindestens eine Phosphorothioatverknüpfung umfasst.

9. Antisense-Oligonukleotid nach einem der vorangehenden Ansprüche, wobei das Oligonukleotid in der Lage ist, eine RNase H zu rekrutieren.

10. Antisense-Oligonukleotid nach einem der vorangehenden Ansprüche, wobei das Antisense-Oligonukleotid ein Oligonukleotid-Sequenzmotiv umfasst, ausgewählt aus der Gruppe bestehend aus SEQ-ID-NR.: 63, SEQ-ID-NR.: 64, SEQ-ID-NR.: 65, SEQ-ID-NR.: 66 SEQ-ID-NR.: 67, SEQ-ID-NR.: 68, SEQ-ID-NR.: 69, SEQ-ID-NR.: 70, SEQ-ID-NR.: 71, SEQ-ID-NR.: 72, SEQ-ID-NR.: 73 und SEQ-ID-NR.: 74.

11. Antisense-Oligonukleotid nach einem der vorangehenden Ansprüche, wobei das Antisense-Oligonukleotid eine Sequenz umfasst, ausgewählt aus SEQ-ID-NR.: 11, SEQ-ID-NR.: 15, SEQ-ID-NR.: 32, SEQ-ID-NR.: 21, SEQ-ID-NR.: 34, SEQ-ID-NR.:10, SEQ-ID-NR.: 12, SEQ-ID-NR.: 13, SEQ-ID-NR.: 14, SEQ-ID-NR.: 16, SEQ-ID-NR.: 17, SEQ-ID-NR.: 18, SEQ-ID-NR.: 19, SEQ-ID-NR.: 20, SEQ-ID-NR.: 22, SEQ-ID-NR.: 23, SEQ-ID-NR.: 24, SEQ-ID-NR.: 25, SEQ-ID-NR.: 26, SEQ-ID-NR.:27, SEQ-ID-NR.: 28, SEQ-ID-NR.: 29, SEQ-ID-NR.: 30, SEQ-ID-NR.: 31, SEQ-ID-NR.: 33, SEQ-ID-NR.: 35, SEQ-ID-NR.: 36, SEQ-ID-NR.: 37, SEQ-ID-NR.: 38, SEQ-ID-NR.: 39, SEQ-ID-NR.: 40, SEQ-ID-NR.: 41, SEQ-ID-NR.: 42, SEQ-ID-NR.: 43, SEQ-ID-NR.: 44, SEQ-ID-NR.: 45, SEQ-ID-NR.: 46, SEQ-ID-NR.: 47, SEQ-ID-NR.: 48, SEQ-ID-NR.: 49, SEQ-ID-NR.: 50, SEQ-ID-NR.: 51, SEQ-ID-NR.: 52, SEQ-ID-NR.: 53, SEQ-ID-NR.: 54, SEQ-ID-NR.: 55, SEQ-ID-NR.: 56, SEQ-ID-NR.: 57, SEQ-ID-NR.: 58, SEQ-ID-NR.: 59, SEQ-ID-NR.: 60, SEQ-ID-NR.: 60, SEQ-ID-NR.: 61 oder SEQ-ID-NR.: 62.

12. Antisense-Oligonukleotid nach einem der vorangehenden Ansprüche, wobei das Antisense-Oligonukleotid 5'- ^{m}**Cₛ**° **Aₛ**° **Tₛ**° tₛ gₛ cₛ aₛ tₛ tₛ gₛ tₛ tₛ tₛ ^{m}**Cₛ**° ^{m}**Cₛ**° **G**°-3' (SEQ-ID-NR.: 11), 5' - ^{m}**Cₛ° Gₛ**°**Tₛ**° t**ₛ**t**ₛ**g**ₛ**t**ₛ**g**ₛ**c**ₛ**tₛ^{m}cₛgₛaₛ**T**ₛ°**A**ₛ°**A**°- 3' (SEQ-ID-NR.: 15), 5' - ^{m}**Cₛ**° **Gₛ**° **Tₛ**° tₛ tₛ gₛ tₛ gₛ cₛ tₛ ^{m}cₛ gₛ **Aₛ**° **Tₛ**° **A**°- 3' (SEQ-ID-NR.: 32) 5' - **Tₛ**° **Tₛ**° **Gₛ**° tₛ gₛ cₛ tₛ ^{m}**C**ₛ gₛ aₛ tₛ **A**ₛ° **A**ₛ° **A**° - 3' (SEQ-ID-NR.: 14) oder 5' - ^{m}**Cₛ**° **Gₛ**° **Tₛ**° tₛ tₛ gₛ tₛ gₛ Cₛtₛ Cₛ **Gₛ**° **Aₛ**° **T**° - 3' (SEQ-ID-NR.: 35) ist, wobei Großbuchstaben verbrückte Nukleinsäure-(LNA)-Einheiten anzeigen, ein tiefgestelltes "s" eine Phosphorothioatverknüpfung darstellt und Kleinbuchstaben Desoxyribonukleinsäure-(DNA)-Einheiten darstellen, "^{m}C" eine 5' Methylcytosin-LNA-Einheit darstellt und "^{m}c" eine 5' Methylcytosin-DNA-Einheit darstellt.

13. Pharmazeutische Zusammensetzung, umfassend das Antisense-Oligonukleotid nach einem der Ansprüche 1 bis 12 und einen pharmazeutisch geeigneten Träger.

14. Antisense-Oligonukleotid nach einem der Ansprüche 1 bis 12 oder pharmazeutische Zusammensetzung nach Anspruch 13 zur Verwendung als Arzneimittel, wie etwa für die Behandlung einer anämischen Krankheit, Störung oder eines anämischen Zustands wie etwa Sichelzellenanämie oder β-Thalassämie.

15. Oligonukleotid nach einem der Ansprüche 1 bis 12 oder pharmazeutische Zusammensetzung nach Anspruch 13 zur Verwendung bei der Behandlung von BCL11A-bedingten Krankheiten, Störungen oder Zuständen.

16. Oligonukleotid nach einem der Ansprüche 1 bis 12 oder pharmazeutische Zusammensetzung nach Anspruch13 zur Verwendung bei der Behandlung einer anämischen Krankheit, Störung oder eines anämischen Zustands.

17. Oligonukleotid für die Verwendung nach Anspruch 13, wobei es sich bei der anämischen Krankheit, Störung oder dem anämischen Zustand um Sichelzellenanämie oder β-Thalassämie handelt.

## Revendications

1. Oligonucléotide antisens capable de diminuer l'expression du BCL11A humain comprenant une séquence qui est au moins identique à 80 % au complément inverse d'une séquence continue dans une région sélectionnée des nucléotides 410 à 450 du gène humain BCL11A de SEQ ID NO: 1 ou une isoforme d'ARN messager (ARNm) du BCL11A, l'oligonucléotide antisens étant un gapmer.

2. Oligonucléotide antisens selon la revendication 1, l'oligonucléotide antisens étant représenté par la formule X*ₐ*,-Y*_{b}*-X_{*a*'}, dans laquelle :
X est un analogue nucléotidique ;
Y est une séquence d'ADN continue ;
*a* est 1, 2, 3, 4 ou 5 ;
*a*' est 1, 2, 3, 4 ou 5 ; et
*b* est un nombre entier compris entre 5 et 15.

3. Oligonucléotide antisens selon la revendication 2, dans lequel a et/ou a'sont compris entre 2 et 4.

4. Oligonucléotide antisens selon l'une quelconque des revendications 2 ou 3, dans lequel b est un entier compris entre 7 et 10.

5. Oligonucléotide antisens selon l'une quelconque des revendications précédentes, l'oligonucléotide antisens faisant moins de 19 nucléotides de long, par exemple 10 à 16 nucléotides de long.

6. Oligonucléotide antisens selon l'une quelconque des revendications précédentes, l'oligonucléotide comprenant au moins un analogue nucléotidique sélectionné dans le groupe des unités 2'-O-alkyl-ARN, unités 2'-OMe-ARN, unités 2'-O-alkyl-ADN, unités 2'-amino-ADN, unités 2'-fluoro-ADN, unités LNA, unités d'acide arabino-nucléique (ANA), unités 2'-fluoro-ANA, unités HNA, unités INA et unités 2'MOE.

7. Oligonucléotide antisens selon la revendication 6, l'analogue nucléotidique étant une unité LNA sélectionnée dans le groupe des bêta-D-oxy-LNA, alpha-L-oxy-LNA, bêta-D-amino-LNA, alpha-L-amino-LNA, bêta -D-thio-LNA, alpha-L-thio-LNA, 5'-méthyl-LNA, bêta-D-ENA, cET et alpha-L-ENA.

8. Oligonucléotide antisens selon l'une quelconque des revendications précédentes, l'oligonucléotide comprenant au moins une liaison phosphorothioate.

9. Oligonucléotide antisens selon l'une quelconque des revendications précédentes, l'oligonucléotide étant capable de recruter une ARNase H.

10. Oligonucléotide antisens selon l'une quelconque des revendications précédentes, l'oligonucléotide antisens comprenant un motif de séquence oligonucléotidique sélectionné dans le groupe consistant en SEQ ID NO: 63, SEQ ID NO: 64, SEQ ID NO: 65, SEQ ID NO: 66 SEQ ID NO: 67, SEQ ID NO: 68, SEQ ID NO: 69, SEQ ID NO: 70, SEQ ID NO: 71, SEQ ID NO: 72, SEQ ID NO: 73 et SEQ ID NO: 74.

11. Oligonucléotide antisens selon l'une quelconque des revendications précédentes, l'oligonucléotide antisens ayant une séquence sélectionnée parmi SEQ ID NO: 11, SEQ ID NO: 15, SEQ ID NO: 32, SEQ ID NO: 21, SEQ ID NO: 34, SEQ ID NO:10, SEQ ID NO: 12, SEQ ID NO: 13, SEQ ID NO: 14, SEQ ID NO: 16, SEQ ID NO: 17, SEQ ID NO: 18, SEQ ID NO: 19, SEQ ID NO: 20, SEQ ID NO: 22, SEQ ID NO: 23, SEQ ID NO: 24, SEQ ID NO: 25, SEQ ID NO: 26, SEQ ID NO:27, SEQ ID NO: 28, SEQ ID NO: 29, SEQ ID NO: 30 SEQ ID NO: 31, SEQ ID NO: 33, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 60, SEQ ID NO: 61 ou SEQ ID NO: 62.

12. Oligonucléotide antisens selon l'une quelconque des revendications précédentes, l'oligonucléotide antisens étant 5^{'m}**Cₛ**° **Aₛ**° **Tₛ**° tₛ gₛ cₛ aₛ tₛ tₛ gₛ tₛ tₛ tₛ ^{m}**Cₛ**° ^{m}**Cₛ**° **G**°-3' (SEQ ID NO:11), 5' - ^{m}**Cₛ**° **Gₛ**° **Tₛ**° tₛtₛgₛtₛgₛcₛtₛ^{m}cₛgₛaₛ**T**ₛ°**A**_{s°}**A**°- 3' (SEQ ID NO: 15), 5' - ^{m}**Cₛ**° **Gₛ**° **Tₛ**° tₛ tₛ gₛ tₛ g_{s Cs} tₛ ^{m}cₛ gₛ **Aₛ**° **Tₛ**° **A**°- 3' (SEQ ID NO: 32) 5' - **T**ₛ° **T**ₛ° **G**ₛ° tₛ gₛ cₛ tₛ ^{m}cₛ gₛ aₛ tₛ **Aₛ**° **Aₛ**° **A**° - 3' (SEQ ID NO: 14) ou 5' - ^{m}**C**ₛ° **Gₛ° Tₛ**° tₛ tₛ gₛ tₛ gₛ cₛ tₛ cₛ **G**ₛ° **A**ₛ° **T**° - 3' (SEQ ID NO: 35), dans lequel les lettres capitales indiquent les unités d'acide nucléique bloqué (LNA), l'indice « s » représente une liaison phosphorothioate et les lettres minuscules représentent des unités désoxyribonucléotide (ADN), « ^{m}C » représente une unité 5'-méthyl-cytosine-LNA et « ^{m}c -» représente une unité 5'méthyl-cytosine-ADN.

13. Composition pharmaceutique comprenant l'oligonucléotide antisens selon l'une quelconque des revendications 1 à 12 et un véhicule pharmaceutiquement acceptable.

14. Oligonucléotide antisens selon l'une quelconque des revendications 1 à 12 ou composition pharmaceutique selon la revendication 13, destinés à être utilisés sous forme de médicament, par exemple dans le traitement des maladies, troubles ou affections de type anémie, comme la drépanocytose ou la β-thalassémie.

15. Oligonucléotide selon l'une quelconque des revendications 1 à 12 ou composition pharmaceutique selon la revendication 13, destinés à être utilisés dans le traitement des maladies, troubles et affections liés au BCL11A.

16. Oligonucléotide selon l'une quelconque des revendications 1 à 12 ou composition pharmaceutique selon la revendication 13 destinés à être utilisés dans le traitement des maladies, troubles et affections de type anémie.

17. Oligonucléotide destiné à être utilisé selon la revendication 16, la maladie, trouble ou affection de type anémie étant la drépanocytose ou la β-thalassémie.


### SEQUENCE LISTING

<110> Santaris Pharma A/S
<120> oligonucleotide modulators of B-cell CLL/lymphoma 11A (BCL11AS) and uses thereof
<130> 1151 WO
<150> US 61/827484
   <151> 2013-05-24
<160> 77
<170> PatentIn version 3.5
<210> 1
   <211> 5946
   <212> DNA
   <213> Homo sapiens
<220>
   <221> exon1
   <222> (1)..(283)
<220>
   <221> exon2
   <222> (284)..(613)
<220>
   <221> exon3
   <222> (614)..(715)
<220>
   <221> exon4
   <222> (716)..(5946)
<400> 1
   tttttttttt ttttttgctt aaaaaaaagc catgacggct ctcccacaat tcatcttccc 60
   tgcgccatct ttgtattatt tctaatttat tttggatgtc aaaaggcact gatgaagata 120
   ttttctctgg agtctccttc tttctaaccc ggctctcccg atgtgaaccg agccgtcgtc 180
   cgcccgccgc cgccgccgcc gccgccgccg cccgccccgc agcccaccat gtctcgccgc 240
   aagcaaggca aaccccagca cttaagcaaa cgggaattct cgcccgagcc tcttgaagcc 300
   attcttacag atgatgaacc agaccacggc ccgttgggag ctccagaagg ggatcatgac 360
   ctcctcacct gtgggcagtg ccagatgaac ttcccattgg gggacattct tatttttatc 420
   gagcacaaac ggaaacaatg caatggcagc ctctgcttag aaaaagctgt ggataagcca 480
   ccttcccctt caccaatcga gatgaaaaaa gcatccaatc ccgtggaggt tggcatccag 540
   gtcacgccag aggatgacga ttgtttatca acgtcatcta gaggaatttg ccccaaacag 600
   gaacacatag cagataaact tctgcactgg aggggcctct cctcccctcg ttctgcacat 660
   ggagctctaa tccccacgcc tgggatgagt gcagaatatg ccccgcaggg tatttgtaaa 720
   gatgagccca gcagctacac atgtacaact tgcaaacagc cattcaccag tgcatggttt 780
   ctcttgcaac acgcacagaa cactcatgga ttaagaatct acttagaaag cgaacacgga 840
   agtcccctga ccccgcgggt tggtatccct tcaggactag gtgcagaatg tccttcccag 900
   ccacctctcc atgggattca tattgcagac aataacccct ttaacctgct aagaatacca 960
   ggatcagtat cgagagaggc ttccggcctg gcagaagggc gctttccacc cactcccccc 1020
   ctgtttagtc caccaccgag acatcacttg gacccccacc gcatagagcg cctgggggcg 1080
   gaagagatgg ccctggccac ccatcacccg agtgcctttg acagggtgct gcggttgaat 1140
   ccaatggcta tggagcctcc cgccatggat ttctctagga gacttagaga gctggcaggg 1200
   aacacgtcta gcccaccgct gtccccaggc cggcccagcc ctatgcaaag gttactgcaa 1260
   ccattccagc caggtagcaa gccgcccttc ctggcgacgc cccccctccc tcctctgcaa 1320
   tccgcccctc ctccctccca gcccccggtc aagtccaagt catgcgagtt ctgcggcaag 1380
   acgttcaaat ttcagagcaa cctggtggtg caccggcgca gccacacggg cgagaagccc 1440
   tacaagtgca acctgtgcga ccacgcgtgc acccaggcca gcaagctgaa gcgccacatg 1500
   aagacgcaca tgcacaaatc gtcccccatg acggtcaagt ccgacgacgg tctctccacc 1560
   gccagctccc cggaacccgg caccagcgac ttggtgggca gcgccagcag cgcgctcaag 1620
   tccgtggtgg ccaagttcaa gagcgagaac gaccccaacc tgatcccgga gaacggggac 1680
   gaggaggaag aggaggacga cgaggaagag gaagaagagg aggaagagga ggaggaggag 1740
   ctgacggaga gcgagagggt ggactacggc ttcgggctga gcctggaggc ggcgcgccac 1800
   cacgagaaca gctcgcgggg cgcggtcgtg ggcgtgggcg acgagagccg cgccctgccc 1860
   gacgtcatgc agggcatggt gctcagctcc atgcagcact tcagcgaggc cttccaccag 1920
   gtcctgggcg agaagcataa gcgcggccac ctggccgagg ccgagggcca cagggacact 1980
   tgcgacgaag actcggtggc cggcgagtcg gaccgcatag acgatggcac tgttaatggc 2040
   cgcggctgct ccccgggcga gtcggcctcg gggggcctgt ccaaaaagct gctgctgggc 2100
   agccccagct cgctgagccc cttctctaag cgcatcaagc tcgagaagga gttcgacctg 2160
   cccccggccg cgatgcccaa cacggagaac gtgtactcgc agtggctcgc cggctacgcg 2220
   gcctccaggc agctcaaaga tcccttcctt agcttcggag actccagaca atcgcctttt 2280
   gcctcctcgt cggagcactc ctcggagaac gggagtttgc gcttctccac accgcccggg 2340
   gagctggacg gagggatctc ggggcgcagc ggcacgggaa gtggagggag cacgccccat 2400
   attagtggtc cgggcccggg caggcccagc tcaaaagagg gcagacgcag cgacacttgt 2460
   gagtactgtg ggaaagtctt caagaactgt agcaatctca ctgtccacag gagaagccac 2520
   acgggcgaaa ggccttataa atgcgagctg tgcaactatg cctgtgccca gagtagcaag 2580
   ctcaccaggc acatgaaaac gcatggccag gtggggaagg acgtttacaa atgtgaaatt 2640
   tgtaagatgc cttttagcgt gtacagtacc ctggagaaac acatgaaaaa atggcacagt 2700
   gatcgagtgt tgaataatga tataaaaact gaatagaggt atattaatac ccctccctca 2760
   ctcccacctg acaccccctt tttcaccact ccccttcccc atcgccctcc agccccactc 2820
   cctgtaggat ttttttctag tcccatgtga tttaaacaaa caaacaaaca aacagaagta 2880
   acgaagctaa gaatatgaga gtgcttgtca ccagcacacc tgtttttttt ctttttcttt 2940
   ttcttttttc tttttccttt tttttttttt tcctttatgt tctcaccgtt tgaatgcatg 3000
   atctgtatgg ggcaatacta ttgcatttta cgcaaacttt gagcctttct cttgtgcaat 3060
   aatttacatg ttgtgtatgt ttttttttaa acttagacag catgtatggt atgttatggc 3120
   tattttaaat tgtccctaat tcgttgctga gcaaacatgt tgctgtttcc agttccgttc 3180
   tgagagaaaa agagagagag agagaaaaag accatgctgc atacattctg taatacatat 3240
   catgtacagt tttattttat aacgtgagga ggaaaaacag tctttggatt aaccctctat 3300
   agacagaata gatagcactg aaaaaaaatc tctatgagct aaatgtctgt ctctaaaggg 3360
   ttaaatgtat caattggaaa ggaagaaaaa aggccttgaa ttgacaaatt aacagaaaaa 3420
   cagaacaagt ttattctatc atttggtttt aaaatatgag tgccttggat ctattaaaac 3480
   cacatcgatg gttctttcta cttgttataa acttgtagct taattcagca ttgggtgagg 3540
   taataaacct taggaactag catataattc tatattgtat ttctcacaac aatggctacc 3600
   taaaaagatg acccattatg tcctagttaa tcatcatttt tcctttagtt taattttata 3660
   aacaaaactg attataccag tataaaagct actttgctcc tggtgagagc ttaaaagaaa 3720
   tgggctgttt tgcccaaagt tttatttttt ttaaacaatg attaaattga atgtgtaatg 3780
   tgcaaaagcc ctggaacgca attaaataca ctagtaagga gttcatttta tgaagatatt 3840
   tgctttaata atgtcttttt aaaaatactg gcaccaaaag aaatagatcc agatctactt 3900
   ggttgtcaag tggacaatca aatgataaac tttaagacct tgtataccat attgaaagga 3960
   agaggctgac aataaggttt gacagagggg aacagaagaa aataatatga tttattagca 4020
   caacgtggta ctatttgcca tttaaaacta gaacaggtat ataagctaat attgatacaa 4080
   tgatgattaa ctatgaattc ttaagacttg catttaaatg tgacattctt aaaaaaagaa 4140
   gagaaagaat tttaagagta gcagtatata tgtctgtgct ccctaaaagt tgtacttcat 4200
   ttcttttcca tacactgtgt gctatttgtg ttaacatgga agaggattca ttgtttttat 4260
   ttttattttt ttaatttttt cttttttatt aagctagcat ctgccccagt tggtgttcaa 4320
   atagcacttg actctgcctg tgatatctgt atcttttctc taatcagaga tacagaggtt 4380
   gagtataaaa taaacctgct cagataggac aattaagtgc actgtacaat tttcccagtt 4440
   tacaggtcta tacttaaggg aaaagttgca agaatgctga aaaaaaattg aacacaatct 4500
   cattgaggag cattttttaa aaactaaaaa aaaaaaaact ttgccagcca tttacttgac 4560
   tattgagctt acttacttgg acgcaacatt gcaagcgctg tgaatggaaa cagaatacac 4620
   ttaacataga aatgaatgat tgctttcgct tctacagtgc aaggattttt ttgtacaaaa 4680
   cttttttaaa tataaatgtt aagaaaaatt ttttttaaaa aacacttcat tatgtttagg 4740
   ggggaactgc attttagggt tccattgtct tggtggtgtt acaagacttg ttatccattt 4800
   aaaaatggta gtggaaattc tatgccttgg atacacaccg ctcttcaggt tgtaaaaaaa 4860
   aaaaacatac attggggaaa ggtttaagat tatatagtac ttaaatatag gaaaatgcac 4920
   actcatgttg attcctatgc taaaatacat ttatggtctt ttttctgtat ttctagaatg 4980
   gtatttgaat taaatgttca tctagtgtta ggcactatag tatttatatt gaagcttgta 5040
   tttttaactg ttgcttgttc tcttaaaagg tatcaatgta ccttttttgg tagtggaaaa 5100
   aaaaaagaca ggctgccaca gtatattttt ttaatttggc aggataatat agtgcaaatt 5160
   atttgtatgc ttcaaaaaaa aaaaaaagag agaaacaaaa aagtgtgaca ttacagatga 5220
   gaagccatat aatggcggtt tgggggagcc tgctagaatg tcacatggat ggctgtcata 5280
   ggggttgtac atatcctttt ttgttccttt ttcctgctgc catactgtat gcagtactgc 5340
   aagctaataa cgttggtttg ttatgtagtg tgctttttgt ccctttcctt ctatcaccct 5400
   acattccagc atcttacctt catatgcagt aaaagaaaga aagaaaaaaa aaggaaaaaa 5460
   aaaaaaaaac caatgttttg cagttttttt cattgccaaa aactaaatgg tgctttatat 5520
   ttagattgga aagaatttca tatgcaaagc atattaaaga gaaagcccgc tttagtcaat 5580
   acttttttgt aaatggcaat gcagaatatt ttgttattgg ccttttctat tcctgtaatg 5640
   aaagctgttt gtcgtaactt gaaattttat cttttactat gggagtcact atttattatt 5700
   gcttatgtgc cctgttcaaa acagaggcac ttaatttgat cttttatttt tctttgtttt 5760
   tatttttttt tttatttaga tgaccaaagg tcattacaac ctggcttttt attgtatttg 5820
   tttctggtct ttgttaagtt ctattggaaa aaccactgtc tgtgtttttt tggcagttgt 5880
   ctgcattaac ctgttcatac acccattttg tccctttatt gaaaaaataa aaaaaattaa 5940
   agtaca 5946
<210> 2
   <211> 3958
   <212> DNA
   <213> Homo sapiens
<220>
   <221> exon1
   <222> (1)..(283)
<220>
   <221> exon2
   <222> (284)..(613)
<220>
   <221> exon3
   <222> (614)..(715)
<220>
   <221> exon4
   <222> (716)..(2458)
<220>
   <221> exon5
   <222> (2459)..(3958)
<400> 2
   tttttttttt ttttttgctt aaaaaaaagc catgacggct ctcccacaat tcatcttccc      60
   tgcgccatct ttgtattatt tctaatttat tttggatgtc aaaaggcact gatgaagata     120
   ttttctctgg agtctccttc tttctaaccc ggctctcccg atgtgaaccg agccgtcgtc     180
   cgcccgccgc cgccgccgcc gccgccgccg cccgccccgc agcccaccat gtctcgccgc     240
   aagcaaggca aaccccagca cttaagcaaa cgggaattct cgcccgagcc tcttgaagcc     300
   attcttacag atgatgaacc agaccacggc ccgttgggag ctccagaagg ggatcatgac     360
   ctcctcacct gtgggcagtg ccagatgaac ttcccattgg gggacattct tatttttatc     420
   gagcacaaac ggaaacaatg caatggcagc ctctgcttag aaaaagctgt ggataagcca     480
   ccttcccctt caccaatcga gatgaaaaaa gcatccaatc ccgtggaggt tggcatccag     540
   gtcacgccag aggatgacga ttgtttatca acgtcatcta gaggaatttg ccccaaacag     600
   gaacacatag cagataaact tctgcactgg aggggcctct cctcccctcg ttctgcacat     660
   ggagctctaa tccccacgcc tgggatgagt gcagaatatg ccccgcaggg tatttgtaaa     720
   gatgagccca gcagctacac atgtacaact tgcaaacagc cattcaccag tgcatggttt     780
   ctcttgcaac acgcacagaa cactcatgga ttaagaatct acttagaaag cgaacacgga     840
   agtcccctga ccccgcgggt tggtatccct tcaggactag gtgcagaatg tccttcccag     900
   ccacctctcc atgggattca tattgcagac aataacccct ttaacctgct aagaatacca     960
   ggatcagtat cgagagaggc ttccggcctg gcagaagggc gctttccacc cactcccccc    1020
   ctgtttagtc caccaccgag acatcacttg gacccccacc gcatagagcg cctgggggcg    1080
   gaagagatgg ccctggccac ccatcacccg agtgcctttg acagggtgct gcggttgaat    1140
   ccaatggcta tggagcctcc cgccatggat ttctctagga gacttagaga gctggcaggg    1200
   aacacgtcta gcccaccgct gtccccaggc cggcccagcc ctatgcaaag gttactgcaa    1260
   ccattccagc caggtagcaa gccgcccttc ctggcgacgc cccccctccc tcctctgcaa    1320
   tccgcccctc ctccctccca gcccccggtc aagtccaagt catgcgagtt ctgcggcaag    1380
   acgttcaaat ttcagagcaa cctggtggtg caccggcgca gccacacggg cgagaagccc    1440
   tacaagtgca acctgtgcga ccacgcgtgc acccaggcca gcaagctgaa gcgccacatg 1500
   aagacgcaca tgcacaaatc gtcccccatg acggtcaagt ccgacgacgg tctctccacc 1560
   gccagctccc cggaacccgg caccagcgac ttggtgggca gcgccagcag cgcgctcaag 1620
   tccgtggtgg ccaagttcaa gagcgagaac gaccccaacc tgatcccgga gaacggggac 1680
   gaggaggaag aggaggacga cgaggaagag gaagaagagg aggaagagga ggaggaggag 1740
   ctgacggaga gcgagagggt ggactacggc ttcgggctga gcctggaggc ggcgcgccac 1800
   cacgagaaca gctcgcgggg cgcggtcgtg ggcgtgggcg acgagagccg cgccctgccc 1860
   gacgtcatgc agggcatggt gctcagctcc atgcagcact tcagcgaggc cttccaccag 1920
   gtcctgggcg agaagcataa gcgcggccac ctggccgagg ccgagggcca cagggacact 1980
   tgcgacgaag actcggtggc cggcgagtcg gaccgcatag acgatggcac tgttaatggc 2040
   cgcggctgct ccccgggcga gtcggcctcg gggggcctgt ccaaaaagct gctgctgggc 2100
   agccccagct cgctgagccc cttctctaag cgcatcaagc tcgagaagga gttcgacctg 2160
   cccccggccg cgatgcccaa cacggagaac gtgtactcgc agtggctcgc cggctacgcg 2220
   gcctccaggc agctcaaaga tcccttcctt agcttcggag actccagaca atcgcctttt 2280
   gcctcctcgt cggagcactc ctcggagaac gggagtttgc gcttctccac accgcccggg 2340
   gagctggacg gagggatctc ggggcgcagc ggcacgggaa gtggagggag cacgccccat 2400
   attagtggtc cgggcccggg caggcccagc tcaaaagagg gcagacgcag cgacacttgt 2460
   tcttcacaca cccccattcg gcgtagtacc cagagagctc aagatgtgtg gcagttttcg 2520
   gatggaagct cgagagccct taagttctga gaaaatttga agcccccagg ggtggggtgg 2580
   acgcgtgccg cccagtcgac gtcagcgtgg tctgtcatcc tgctagtttg tgatgttttc 2640
   tgacagtagc ctccaagaag ccgttgtgcg aagacagagt cctgcagagt ccttccagcc 2700
   taggcctgca gcgccatttt atttatattt tttaataaaa agtaaaaaca aaaaaacaga 2760
   cccacattgg aacagtgaat cagtcccata gagagggccc gtggaccatc gctgtcatga 2820
   gtgatgccct ggcccttctg aaaccagcca acctaattac ctgtattgtg gaaatgcgca 2880
   tgagtcccca accccttgtt tctatacatt ctatgttgtc ttttaaaaag tgtgcttaac 2940
   attgacacaa taaatgttgg agctttaggt ggtgtttgct tgttctttaa tttttaatgc 3000
   ttataagaca atgaggctgc ttatgatttt gtacttctgt acctgtttcc tacagacacc 3060
   catcgggtgg gtaggaggaa cagatttgag aaatgggcag gagatgtagg aggggaacta 3120
   ggttaccgct tatcagatgg cataaatttt caaggagaat caaaatgcaa aacttgggaa 3180
   taaatcatag caatatcata attaatgtag tagtaatatt gctgtttatt aatgctgaag 3240
   tgtggttttc ctaactgtct gacttataat ttgcatacca ttaaataatg cataatatgg 3300
   cacgccgaat cctgtttttc aaatatatgc ttttggtggc taccatgcag gatttgaatt 3360
   tgtcttttaa tttagcttag gaaagaacat cactgggcga gcggtaaatc ctaaagaagg     3420
   tgataaatgt cagtagtttc ttattaaata ttctaatttt aggttcccaa accttcagga     3480
   aatatatctt aatgcagaca aacaaacata aaacttcttt agtacttaca tcaggaaatt     3540
   tggggcagat tttagagggg ggaaattata ggaggaaaga agttcacatc agaacagaca     3600
   atcacagcaa tgctctattc cttagaaatt agtgccacaa ataagttaca tctacaaaca     3660
   ggtggtaaaa attctttctg gcccagttaa tttgcacaga acttttctca gtttggtatt     3720
   ttttactgct tggagatcca gaagagaatt agaaacaaca tagcaaatta aaataggttt     3780
   gtcaataata gagctcagac acctgtgtgc tgtagattca catacaggcc gtgaacctaa     3840
   gtggggaaaa tcctacctat ccaccttctg gctagattac ctagcttagt gaaaagatag     3900
   ccaaataatt ggcatgtgaa ttatttcctg cttattcata ataaataatg actgtcta       3958
<210> 3
   <211> 2358
   <212> DNA
   <213> Homo sapiens
<220>
   <221> exon1
   <222> (1)..(283)
<220>
   <221> exon2
   <222> (284)..(613)
<220>
   <221> exon3
   <222> (614)..(714)
<220>
   <221> exon4
   <222> (715)..(858)
<220>
   <221> exon5
   <222> (859)..(2358)
<400> 3
   tttttttttt ttttttgctt aaaaaaaagc catgacggct ctcccacaat tcatcttccc      60
   tgcgccatct ttgtattatt tctaatttat tttggatgtc aaaaggcact gatgaagata     120
   ttttctctgg agtctccttc tttctaaccc ggctctcccg atgtgaaccg agccgtcgtc     180
   cgcccgccgc cgccgccgcc gccgccgccg cccgccccgc agcccaccat gtctcgccgc     240
   aagcaaggca aaccccagca cttaagcaaa cgggaattct cgcccgagcc tcttgaagcc     300
   attcttacag atgatgaacc agaccacggc ccgttgggag ctccagaagg ggatcatgac     360
   ctcctcacct gtgggcagtg ccagatgaac ttcccattgg gggacattct tatttttatc     420
   gagcacaaac ggaaacaatg caatggcagc ctctgcttag aaaaagctgt ggataagcca 480
   ccttcccctt caccaatcga gatgaaaaaa gcatccaatc ccgtggaggt tggcatccag 540
   gtcacgccag aggatgacga ttgtttatca acgtcatcta gaggaatttg ccccaaacag 600
   gaacacatag cagataaact tctgcactgg aggggcctct cctcccctcg ttctgcacat 660
   ggagctctaa tccccacgcc tgggatgagt gcagaatatg ccccgcaggg tatttgtaaa 720
   gatgagccca gcagctacac atgtacaact tgcaaacagc cattcaccag tgcatggttt 780
   ctcttgcaac acgcacagaa cactcatgga ttaagaatct acttagaaag cgaacacgga 840
   agtcccctga ccccgcgggt tcttcacaca cccccattcg gcgtagtacc cagagagctc 900
   aagatgtgtg gcagttttcg gatggaagct cgagagccct taagttctga gaaaatttga 960
   agcccccagg ggtggggtgg acgcgtgccg cccagtcgac gtcagcgtgg tctgtcatcc 1020
   tgctagtttg tgatgttttc tgacagtagc ctccaagaag ccgttgtgcg aagacagagt 1080
   cctgcagagt ccttccagcc taggcctgca gcgccatttt atttatattt tttaataaaa 1140
   agtaaaaaca aaaaaacaga cccacattgg aacagtgaat cagtcccata gagagggccc 1200
   gtggaccatc gctgtcatga gtgatgccct ggcccttctg aaaccagcca acctaattac 1260
   ctgtattgtg gaaatgcgca tgagtcccca accccttgtt tctatacatt ctatgttgtc 1320
   ttttaaaaag tgtgcttaac attgacacaa taaatgttgg agctttaggt ggtgtttgct 1380
   tgttctttaa tttttaatgc ttataagaca atgaggctgc ttatgatttt gtacttctgt 1440
   acctgtttcc tacagacacc catcgggtgg gtaggaggaa cagatttgag aaatgggcag 1500
   gagatgtagg aggggaacta ggttaccgct tatcagatgg cataaatttt caaggagaat 1560
   caaaatgcaa aacttgggaa taaatcatag caatatcata attaatgtag tagtaatatt 1620
   gctgtttatt aatgctgaag tgtggttttc ctaactgtct gacttataat ttgcatacca 1680
   ttaaataatg cataatatgg cacgccgaat cctgtttttc aaatatatgc ttttggtggc 1740
   taccatgcag gatttgaatt tgtcttttaa tttagcttag gaaagaacat cactgggcga 1800
   gcggtaaatc ctaaagaagg tgataaatgt cagtagtttc ttattaaata ttctaatttt 1860
   aggttcccaa accttcagga aatatatctt aatgcagaca aacaaacata aaacttcttt 1920
   agtacttaca tcaggaaatt tggggcagat tttagagggg ggaaattata ggaggaaaga 1980
   agttcacatc agaacagaca atcacagcaa tgctctattc cttagaaatt agtgccacaa 2040
   ataagttaca tctacaaaca ggtggtaaaa attctttctg gcccagttaa tttgcacaga 2100
   acttttctca gtttggtatt ttttactgct tggagatcca gaagagaatt agaaacaaca 2160
   tagcaaatta aaataggttt gtcaataata gagctcagac acctgtgtgc tgtagattca 2220
   catacaggcc gtgaacctaa gtggggaaaa tcctacctat ccaccttctg gctagattac 2280
   ctagcttagt gaaaagatag ccaaataatt ggcatgtgaa ttatttcctg cttattcata 2340
   ataaataatg actgtcta 2358
<210> 4
   <211> 6122
   <212> DNA
   <213> Mus musculus
<220>
   <221> exon1
   <222> (1)..(390)
<220>
   <221> exon2
   <222> (391)..(720)
<220>
   <221> exon3
   <222> (721)..(822)
<220>
   <221> exon4
   <222> (823)..(6112)
<400> 4
   gacgttcaag ttcgcaggga cgtcacgtcc gcacttgaac ttgcagctca ggggggcttt      60
   tgccattttt ttcatctctc tctctccctc tatccctctt ctctcttcct ctctctcttt     120
   tttttcctta aaaaaaaaaa agccatgacg gctctcccac aattcatctt ccctgcgcca     180
   tctttgtatt atttctaatt tattttggat gtcaaaaggc actgatgaag atattttctc     240
   tggagtctcc ttctttctaa cccggctctc ccgatgtgaa ccgagccgtc gtccgcacgc     300
   cgccgccgcc gccgccgccc gccccgcagc ccaccatgtc tcgccgcaag caaggcaaac     360
   cccagcactt aagcaaacgg gaattctcgc ccgaacctct tgaagccatt cttacagatg     420
   atgaaccaga ccatggcccg ttgggagctc cagaagggga ccacgacctt ctcacctgtg     480
   ggcagtgcca gatgaatttc ccactggggg acattcttat ttttatcgag cacaaacgga     540
   aacaatgcaa tggcagcctc tgcttagaaa aaggtgtgga taagccgcct tccccttctc     600
   ccatcgagat gaaaaaggca tccaatcctg tggaggttgg catccaggtc acgccagagg     660
   atgacgattg tttatcaacg tcatctagag gaatttgccc caaacaggaa cacatagcag     720
   ataaacttct gcactggagg ggcctgtcct ctcctcggtc tgcacacgga gctctaatcc     780
   ccacgcccgg gatgagtgca gaatatgccc cgcagggtat ttgtaaagat gagcccagca     840
   gctacacatg tacaacttgc aaacagccat tcaccagtgc atggtttctc ttgcaacacg     900
   cacagaacac tcatggatta agaatctact tagaaagtga acacggaagt cccctgaccc     960
   cgcgggttgg tatcccttca ggactaggtg cagaatgtcc ttcccagcca cctctccatg    1020
   ggattcatat tgcagacaat aaccccttta acctgctaag aataccagga tcagtatcga    1080
   gagaggcttc cggcctggca gaagggcgct ttccacccac tccccccctg tttagtccac 1140
   caccgagaca tcacttggac ccccaccgca tagagcgcct gggggcggaa gagatggccc 1200
   tggccaccca tcacccgagt gcctttgaca gggtgctgcg gttgaatcca atggctatgg 1260
   agcctcccgc catggatttc tctaggagac ttagagagct ggcagggaac acgtctagtc 1320
   caccgctgtc cccaggccgg cccagtccta tgcaaaggtt actgcaacca ttccagccag 1380
   gtagcaagcc acccttcctg gcgacgcccc ccctccctcc tctgcaatcc gcccctcctc 1440
   cctcccaacc cccggtcaag tccaagtcat gcgagttctg cggcaagacg ttcaaatttc 1500
   agagcaactt ggtggttcac cgacgcagcc atactggtga gaagccctat aagtgcaacc 1560
   tgtgcgacca cgcgtgcaca caggccagca agctgaagcg tcacatgaag acacacatgc 1620
   acaaatcgtc ccccatgaca gtcaagtccg acgatggcct ctccacagcc agctccccgg 1680
   aacctggtac cagcgacctg gtgggcagcg ccagcagtgc gctcaagtca gtggtggcca 1740
   agttcaagag tgagaacgac cccaacttga tcccagagaa cggggatgag gaggaagagg 1800
   aggacgacga ggaagaagaa gaagaggagg aagaggagga ggaggagctg acggagagcg 1860
   agagggtgga ctacggcttc gggctgagcc tggaggctgc acgccaccat gagaacagct 1920
   ctcggggcgc agtggtgggc gtgggcgacg agggccgcgc cctgcccgat gtcatgcagg 1980
   gcatggtgct cagctccatg cagcacttca gcgaggcctt ccaccaggtc ctgggcgaaa 2040
   agcataagcg tagccacctg gccgaggccg agggccatag ggacacttgt gatgaagact 2100
   cggtggccgg tgagtcagac cgcatagacg atggcactgt taatggtcgt ggctgctccc 2160
   ccggcgaatc ggcttcgggg ggtctgtcca aaaagctgct gctgggtagc cccagctcgc 2220
   tgagcccctt ctccaagcgc atcaagctgg agaaggagtt tgacctgccc ccggccgcga 2280
   tgcctaacac ggagaacgtg tattcgcagt ggctcgctgg ctatgcggcc tccaggcagc 2340
   tcaaagatcc cttccttact ttcggagact ccagacaatc gccttttgcc tcctcatcag 2400
   agcactcctc ggagaacggg agcttgcgct tctccacacc gcccggggag ctggacggag 2460
   ggatctcagg gcgcagcggc acaggaagtg gagggagcac gccccatatt agtggtccgg 2520
   gcccgggcag gcccagctca aaagagggca gacgcagcga cacttgtgag tactgtggga 2580
   aagtcttcaa gaactgtagc aatctcactg tccacaggag aagccacacg ggcgaaaggc 2640
   cttataaatg cgagctgtgc aactatgcct gtgcccagag tagcaagctc accaggcaca 2700
   tgaaaacgca tggccaggtg gggaaggacg tttacaaatg tgaaatttgt aagatgcctt 2760
   ttagcgtgta cagtaccctg gagaaacaca tgaaaaaatg gcacagtgat cgagtgttga 2820
   ataatgatat aaaaactgaa tagaggtata ttaataccct ccctcactcc cacttgatgc 2880
   cccccttcca ccccttcccc attgtccttc cagccctact ccctgtagga tttttctagt 2940
   cccatgtgat caaacaaaca aacaaacaaa caacagaggt aatggaagct aagaatatga 3000
   atgagtgctt gtcaccagca cacctggttt tttgttttgt ttttcctttt ttttttcttt 3060
   ttcttcttct tttttttaaa ttttaaattc tttatgttct caccgtttga atgcatgttt 3120
   ggggcaatac tattgcattt tacgcaaact ttgagccttt ctcttgtgca ataatttaca 3180
   tgttgtgtat gtttttcccc ccttaactta gacagcatgt atggtatgtt acggctattt 3240
   taaattgtcc ctaattcatt atgagcaaac atgttgttgc tgtttccagt tccattctga 3300
   gagtgtgagg gagggagggt agaaaacaac gcatgctgca tacataattc tgtaatacag 3360
   atcatgtgca gctttatttt ataacacgag gagggaaacg gtgtctgggt tgactaaccc 3420
   tctgcagaca gagcagatag cagtgaaaaa aaggtgctaa atgtctgtct ctaaagggtt 3480
   acatgtatta attggagagg gaaaaaaggc cttgaattga caaattaaca gaagaacaag 3540
   tttattctat catttggctt ttaaaatgag tgccttggat ctattcaaac catgttgatg 3600
   gttttttttc tgcttgttat aaacttgtag tttaatctag cattgggtga ggtaataaac 3660
   tttaggacct agcatgtaat tctgtgttgt atttctcaca acaatggcta cctaaaaata 3720
   tgacccatta tgtcctagtt aatcattgat ttttgccttt aactttgtga acaaaactga 3780
   ttataccagt ataaaagcta ctttgctcct tgtgagagca taaaagaaat gggctgtttc 3840
   gcctaaagtt ttattttatt ttatttttaa atggttatca aattgaatgt gaaatgtgca 3900
   aaggccctgg aatgtgatga aatacattag caagaagttc atcttgtgac aatacttgtt 3960
   taaaatgatg catttaatga aattctggcg ccaaaagaag tagatccggc tctagttggt 4020
   tgttgagtgg acaataaaat gataaagccc tttagaggac atttgaaaga acaggctgat 4080
   atgatgagag agagagagag agacagaaaa cggtagtgat tggttagcac gatgtagtac 4140
   tgtttgccat ttgaaactag aacaggtgta taaggggcca gcgatacaat gatgattaac 4200
   tctgaagctc taagacttgc atttaatgtg acagtcttca aaaagaagag gaagaccttt 4260
   aagagcagca gtatctatgt ctgtgctccc tggaagttgt acttcatttc ttttccatac 4320
   actgtgtgct atttgtgtta acattgaaga ggatttgttt ttattttgtt ttattctttc 4380
   tttctttctt ttttcttcct tctttgtttt aagctagcat ctgccccagt tggtgttcaa 4440
   atagcacttg actctgccta tgatacctgt atcttttctc taatcagaga tacagaggtt 4500
   gagtataaaa taaacctgct cagataggac aattaagtgc actgtacagt tttcccagtt 4560
   tacaggtctt tattaaggga aacgttgcaa gaatgctgaa aacaattgaa cacaatctca 4620
   atgatgagca ttaaaaaata ataaaaaaaa acaagcaaac ctaaaaaaaa ctaagacaga 4680
   cgttgccagc cattgacttg actattgagc ttcctcactt ggatgcaaca ttgcaagcgc 4740
   tgtggatgga aacaacacac ttaacataga aacgaatgac tcctttgctt ctacagtgca 4800
   aggatttttg tacaaaactt ttttaagtat aaatgttaag aaaagaattt ttaaaaagac 4860
   acttcattat gtttaggggg gaacagcatt ttagggttcc attgtcttgg tggtgttaca 4920
   agacctgtta tccatttaaa aatggtagtg gaaattctat gccttggatc acacaccgct 4980
   cttcaggttg taaaaaaaaa tgaaaacaaa acaaaacaaa aaaaaaacat acatggggaa 5040
   aggtttaaga ttatatagta cttaaaaata ggaaaacgca cactcatgtt gattcctatg 5100
   ctaaaacaca gttatggtct tctttctgta tttctagaat ggtatttgaa ttaaatgttc 5160
   atctagtgtt aggcactata gtatttatat tgaagcttgt atttttaact gttgcttgtt 5220
   cactcaaaag gtatcaatgt acctttttgt tagtagaaaa aaaaagacag gctgccacag 5280
   tatatttttt taatttggca ggataatata gtgcaaatta tttgtatgct gagagagaga 5340
   gagagagaga gagagagaga gagagaggtg tgacattgta cagagaagcc atataatggc 5400
   ggtttgggga gcctgctaga atgtcacatg gatggctgtc ataggggttg tacatatcct 5460
   tttccccctt tcctgctgcc atgctgtacg cagcactgca agctaatagc gttggtttgt 5520
   tatgtagtgt gctttggccc ctcctccccg ctcacccgac attccagcat cttaccttca 5580
   tatgcagtaa aagaaagaaa gaaaaaaaaa aaggaaaaaa aaaacaacaa caatgttttg 5640
   cagttttttt cattgccaaa aactaaatgg tgctttatat ttagattgga gagaatttct 5700
   tatgcaaagc atattaaaga gaaagcccgc tttagtcaat acttttttgt aaatggcaat 5760
   gcagaatatt ttgttattgg ccttttctat tcctgtaatg aaagctgttt gtcgtaactt 5820
   gaaattttat cttttactat gggagtcact atttattatt gcttatatgc cctgttcaaa 5880
   acagaggcac ttaatttgat cttttatttt tctctgtttt tattattatt ttttttaatt 5940
   tggatgacca aaggtcattg caacctggct ttttactgta tttgtttctg gtctttgtta 6000
   agttctattg gaaaaaccac tgtctgtgtt tttttggcag ttgtctgcat taacctgttc 6060
   atacacccat tttgtccctt tattgaaaaa aaataaaaaa attaaagtac taaaaaaaaa 6120
   aa 6122
<210> 5
   <211> 3425
   <212> DNA
   <213> Mus musculus
<220>
   <221> exon1
   <222> (1)..(390)
<220>
   <221> exon2
   <222> (391)..(720)
<220>
   <221> exon3
   <222> (721)..(822)
<220>
   <221> exon4
   <222> (823)..(2565)
<220>
   <221> exon5
   <222> (2566)..(3425)
<400> 5
   gacgttcaag ttcgcaggga cgtcacgtcc gcacttgaac ttgcagctca gggggctttt        60
   tgccattttt ttcatctctc tctctccctc tatccctctt ctctcttcct ctctctcttt       120
   tttttcctta aaaaaaaaaa agccatgacg gctctcccac aattcatctt ccctgcgcca       180
   tctttgtatt atttctaatt tattttggat gtcaaaaggc actgatgaag atattttctc       240
   tggagtctcc ttctttctaa cccggctctc ccgatgtgaa ccgagccgtc gtccgcacgc       300
   cgccgccgcc gccgccgccc gccccgcagc ccaccatgtc tcgccgcaag caaggcaaac       360
   cccagcactt aagcaaacgg gaattctcgc ccgaacctct tgaagccatt cttacagatg       420
   atgaaccaga ccatggcccg ttgggagctc cagaagggga ccacgacctt ctcacctgtg       480
   ggcagtgcca gatgaatttc ccactggggg acattcttat ttttatcgag cacaaacgga       540
   aacaatgcaa tggcagcctc tgcttagaaa aaggtgtgga taagccgcct tccccttctc       600
   ccatcgagat gaaaaaggca tccaatcctg tggaggttgg catccaggtc acgccagagg       660
   atgacgattg tttatcaacg tcatctagag gaatttgccc caaacaggaa cacatagcag       720
   ataaacttct gcactggagg ggcctgtcct ctcctcggtc tgcacacgga gctctaatcc       780
   ccacgcccgg gatgagtgca gaatatgccc cgcagggtat ttgtaaagat gagcccagca       840
   gctacacatg tacaacttgc aaacagccat tcaccagtgc atggtttctc ttgcaacacg       900
   cacagaacac tcatggatta agaatctact tagaaagtga acacggaagt cccctgaccc       960
   cgcgggttgg tatcccttca ggactaggtg cagaatgtcc ttcccagcca cctctccatg      1020
   ggattcatat tgcagacaat aaccccttta acctgctaag aataccagga tcagtatcga      1080
   gagaggcttc cggcctggca gaagggcgct ttccacccac tccccccctg tttagtccac      1140
   caccgagaca tcacttggac ccccaccgca tagagcgcct gggggcggaa gagatggccc      1200
   tggccaccca tcacccgagt gcctttgaca gggtgctgcg gttgaatcca atggctatgg      1260
   agcctcccgc catggatttc tctaggagac ttagagagct ggcagggaac acgtctagtc      1320
   caccgctgtc cccaggccgg cccagtccta tgcaaaggtt actgcaacca ttccagccag      1380
   gtagcaagcc acccttcctg gcgacgcccc ccctccctcc tctgcaatcc gcccctcctc      1440
   cctcccaacc cccggtcaag tccaagtcat gcgagttctg cggcaagacg ttcaaatttc      1500
   agagcaactt ggtggttcac cgacgcagcc atactggtga gaagccctat aagtgcaacc      1560
   tgtgcgacca cgcgtgcaca caggccagca agctgaagcg tcacatgaag acacacatgc      1620
   acaaatcgtc ccccatgaca gtcaagtccg acgatggcct ctccacagcc agctccccgg 1680
   aacctggtac cagcgacctg gtgggcagcg ccagcagtgc gctcaagtca gtggtggcca 1740
   agttcaagag tgagaacgac cccaacttga tcccagagaa cggggatgag gaggaagagg 1800
   aggacgacga ggaagaagaa gaagaggagg aagaggagga ggaggagctg acggagagcg 1860
   agagggtgga ctacggcttc gggctgagcc tggaggctgc acgccaccat gagaacagct 1920
   ctcggggcgc agtggtgggc gtgggcgacg agggccgcgc cctgcccgat gtcatgcagg 1980
   gcatggtgct cagctccatg cagcacttca gcgaggcctt ccaccaggtc ctgggcgaaa 2040
   agcataagcg tagccacctg gccgaggccg agggccatag ggacacttgt gatgaagact 2100
   cggtggccgg tgagtcagac cgcatagacg atggcactgt taatggtcgt ggctgctccc 2160
   ccggcgaatc ggcttcgggg ggtctgtcca aaaagctgct gctgggtagc cccagctcgc 2220
   tgagcccctt ctccaagcgc atcaagctgg agaaggagtt tgacctgccc ccggccgcga 2280
   tgcctaacac ggagaacgtg tattcgcagt ggctcgctgg ctatgcggcc tccaggcagc 2340
   tcaaagatcc cttccttact ttcggagact ccagacaatc gccttttgcc tcctcatcag 2400
   agcactcctc ggagaacggg agcttgcgct tctccacacc gcccggggag ctggacggag 2460
   ggatctcagg gcgcagcggc acaggaagtg gagggagcac gccccatatt agtggtccgg 2520
   gcccgggcag gcccagctca aaagagggca gacgcagcga cacttgtcct tcacacaccc 2580
   ccgttcggcg tagtaccccg cgagctcaag atgtgtggca gttttcggat ggaagctcaa 2640
   gaacccttaa gttctgagaa actttgaagc cccaagggcg ggggcggaca tgcgccgccc 2700
   agccgacgtc aacgtgctcc gttatcctgc tagattgtga tgttttctga cagtagcctc 2760
   caagaagaca agagtcctgc cgagtcctcc cagcctgggc ctgcagtgcc attttattta 2820
   tattttttaa taaaacgtaa aaacaaaaaa aaccagaccc acattggaac agtgaacccg 2880
   tcccatccag agggccctag gactgccgca gttggagcga cgtccaaccc ttttgaaacc 2940
   agccaaccta attacccgta ctgtggaaat gagcatgacc cctgacccct tgtttctata 3000
   cattctatgt tgtcttttaa aaagtgtgct taacattgac ataataaatg ttggagcttt 3060
   aggcggtgtg tgcttgtttt ttaattttta atgctcgtaa gacaatgtgg ctgcttcagg 3120
   ctttatgtct gtgtactttt tttccttcag aagctcatag ggtgagcaga aggaccagac 3180
   tcaagtgcca ggcaggagac ctagaaaagg aagtaggctt ttcagatggc atacattttc 3240
   aaagaaaatc aaaatgcaaa gctaggggat aaatcatagt aatatcataa ttaatgtagt 3300
   agtattgctg tttattaatg ctgacgtgtg tttttcctct ctgacttata atttgcatac 3360
   cattaaataa tgcataaata tggcacactg aatccttttt caaatacacg cttttggtga 3420
   ctacc 3425
<210> 6
   <211> 1825
   <212> DNA
   <213> Mus musculus
<220>
   <221> exon1
   <222> (1)..(390)
<220>
   <221> exon2
   <222> (391)..(720)
<220>
   <221> exon3
   <222> (721)..(822)
<220>
   <221> exon4
   <222> (823)..(965)
<220>
   <221> exon5
   <222> (966)..(1825)
<400> 6
   gacgttcaag ttcgcaggga cgtcacgtcc gcacttgaac ttgcagctca ggggggcttt 60
   tgccattttt ttcatctctc tctctccctc tatccctctt ctctcttcct ctctctcttt 120
   tttttcctta aaaaaaaaaa agccatgacg gctctcccac aattcatctt ccctgcgcca 180
   tctttgtatt atttctaatt tattttggat gtcaaaaggc actgatgaag atattttctc 240
   tggagtctcc ttctttctaa cccggctctc ccgatgtgaa ccgagccgtc gtccgcacgc 300
   cgccgccgcc gccgccgccc gccccgcagc ccaccatgtc tcgccgcaag caaggcaaac 360
   cccagcactt aagcaaacgg gaattctcgc ccgaacctct tgaagccatt cttacagatg 420
   atgaaccaga ccatggcccg ttgggagctc cagaagggga ccacgacctt ctcacctgtg 480
   ggcagtgcca gatgaatttc ccactggggg acattcttat ttttatcgag cacaaacgga 540
   aacaatgcaa tggcagcctc tgcttagaaa aaggtgtgga taagccgcct tccccttctc 600
   ccatcgagat gaaaaaggca tccaatcctg tggaggttgg catccaggtc acgccagagg 660
   atgacgattg tttatcaacg tcatctagag gaatttgccc caaacaggaa cacatagcag 720
   ataaacttct gcactggagg ggcctgtcct ctcctcggtc tgcacacgga gctctaatcc 780
   ccacgcccgg gatgagtgca gaatatgccc cgcagggtat ttgtaaagat gagcccagca 840
   gctacacatg tacaacttgc aaacagccat tcaccagtgc atggtttctc ttgcaacacg 900
   cacagaacac tcatggatta agaatctact tagaaagtga acacggaagt cccctgaccc 960
   cgcgggtcct tcacacaccc ccgttcggcg tagtaccccg cgagctcaag atgtgtggca 1020
   gttttcggat ggaagctcaa gaacccttaa gttctgagaa actttgaagc ccccaagggc 1080
   ggggcggaca tgcgccgccc agccgacgtc aacgtgctcc gttatcctgc tagattgtga 1140
   tgttttctga cagtagcctc caagaagaca agagtcctgc cgagtcctcc cagcctgggc 1200
   ctgcagtgcc attttattta tattttttaa taaaacgtaa aaacaaaaaa aaccagaccc 1260
   acattggaac agtgaacccg tcccatccag agggccctag gactgccgca gttggagcga 1320
   cgtccaaccc ttttgaaacc agccaaccta attacccgta ctgtggaaat gagcatgacc 1380
   cctgacccct tgtttctata cattctatgt tgtcttttaa aaagtgtgct taacattgac 1440
   ataataaatg ttggagcttt aggcggtgtg tgcttgtttt ttaattttta atgctcgtaa 1500
   gacaatgtgg ctgcttcagg ctttatgtct gtgtactttt tttccttcag aagctcatag 1560
   ggtgagcaga aggaccagac tcaagtgcca ggcaggagac ctagaaaagg aagtaggctt 1620
   ttcagatggc atacattttc aaagaaaatc aaaatgcaaa gctaggggat aaatcatagt 1680
   aatatcataa ttaatgtagt agtattgctg tttattaatg ctgacgtgtg tttttcctct 1740
   ctgacttata atttgcatac cattaaataa tgcataaata tggcacactg aatccttttt 1800
   caaatacacg cttttggtga ctacc 1825
<210> 7
   <211> 1480
   <212> DNA
   <213> Mus musculus
<220>
   <221> exon1
   <222> (1)..(45)
<220>
   <221> exon2
   <222> (46)..(375)
<220>
   <221> exon3
   <222> (376)..(477)
<220>
   <221> exon4
   <222> (478)..(620)
<220>
   <221> exon5
   <222> (621)..(1480)
<400> 7
   agagcagacg cggcgcgcga cggtgtgaag ttacagcccg gccagccgaa cctcttgaag        60
   ccattcttac agatgatgaa ccagaccatg gcccgttggg agctccagaa ggggaccacg       120
   accttctcac ctgtgggcag tgccagatga atttcccact gggggacatt cttattttta       180
   tcgagcacaa acggaaacaa tgcaatggca gcctctgctt agaaaaaggt gtggataagc       240
   cgccttcccc ttctcccatc gagatgaaaa aggcatccaa tcctgtggag gttggcatcc       300
   aggtcacgcc agaggatgac gattgtttat caacgtcatc tagaggaatt tgccccaaac      360
   aggaacacat agcagataaa cttctgcact ggaggggcct gtcctctcct cggtctgcac      420
   acggagctct aatccccacg cccgggatga gtgcagaata tgccccgcag ggtatttgta      480
   aagatgagcc cagcagctac acatgtacaa cttgcaaaca gccattcacc agtgcatggt      540
   ttctcttgca acacgcacag aacactcatg gattaagaat ctacttagaa agtgaacacg      600
   gaagtcccct gaccccgcgg gtccttcaca cacccccgtt cggcgtagta ccccgcgagc      660
   tcaagatgtg tggcagtttt cggatggaag ctcaagaacc cttaagttct gagaaacttt      720
   gaagccccca agggcggggc ggacatgcgc cgcccagccg acgtcaacgt gctccgttat      780
   cctgctagat tgtgatgttt tctgacagta gcctccaaga agacaagagt cctgccgagt      840
   cctcccagcc tgggcctgca gtgccatttt atttatattt tttaataaaa cgtaaaaaca      900
   aaaaaaacca gacccacatt ggaacagtga acccgtccca tccagagggc cctaggactg      960
   ccgcagttgg agcgacgtcc aacccttttg aaaccagcca acctaattac ccgtactgtg     1020
   gaaatgagca tgacccctga ccccttgttt ctatacattc tatgttgtct tttaaaaagt     1080
   gtgcttaaca ttgacataat aaatgttgga gctttaggcg gtgtgtgctt gttttttaat    1140
   ttttaatgct cgtaagacaa tgtggctgct tcaggcttta tgtctgtgta ctttttttcc    1200
   ttcagaagct catagggtga gcagaaggac cagactcaag tgccaggcag gagacctaga     1260
   aaaggaagta ggcttttcag atggcataca ttttcaaaga aaatcaaaat gcaaagctag     1320
   gggataaatc atagtaatat cataattaat gtagtagtat tgctgtttat taatgctgac     1380
   gtgtgttttt cctctctgac ttataatttg cataccatta aataatgcat aaatatggca     1440
   cactgaatcc tttttcaaat acacgctttt ggtgactacc                           1480
<210> 8
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (12)..(14)
   <223> beta-D-oxy LNA
<400> 8
   ctatgtgttc ctgt 14
<210> 9
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (14)..(16)
   <223> beta-D-oxy LNA
<220>
   <221> misc_feature
   <222> (14)..(14)
   <223> 5-methyl cytosine
<400> 9
   gagacatggt gggctg 16
<210> 10
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (14)..(16)
   <223> beta-D-oxy LNA
<220>
   <221> misc_feature
   <222> (14)..(14)
   <223> 5-methyl cytosine
<400> 10
   attgcattgt ttccgt 16
<210> 11
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (14)..(16)
   <223> beta-D-oxy LNA
<220>
   <221> misc_feature
   <222> (14)..(15)
   <223> 5-methyl cytosine
<400> 11
   cattgcattg tttccg 16
<210> 12
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (5)..(5)
   <223> 5-methyl cytosine
<220>
   <221> misc_feature
   <222> (13)..(15)
   <223> beta-D-oxy LNA
<220>
   <221> misc_feature
   <222> (13)..(14)
   <223> 5-methyl cytosine
<400> 12
   attgcattgt ttccg 15
<210> 13
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (13)..(15)
   <223> beta-D-oxy LNA
<220>
   <221> misc_feature
   <222> (13)..(14)
   <223> 5-methyl cytosine
<400> 13
   attgcattgt ttccg 15
<210> 14
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (8)..(8)
   <223> 5-methyl cytosine
<220>
   <221> misc_feature
   <222> (12)..(14)
   <223> beta-D-oxy LNA
<400> 14
   ttgtgctcga taaa 14
<210> 15
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (14)..(16)
   <223> beta-D-oxy LNA
<400> 15
   cgtttgtgct cgataa 16
<210> 16
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (1)..(2)
   <223> 5-methyl cytosine
<220>
   <221> misc_feature
   <222> (12)..(14)
   <223> beta-D-oxy LNA
<220>
   <221> misc_feature
   <222> (12)..(12)
   <223> 5-methyl cytosine
<400> 16
   ccgtttgtgc tcga 14
<210> 17
   <211> 13
   <212> DNA
   <213> ARTIFICIAL
<220>
   <223> oligonucleotide
<220>
   <221> misc_feature
   <222> (1)..(13)
   <223> Phosphorothioate linkages
<220>
   <221> misc_feature
   <222> (1)..(2)
   <223> beta-D-oxy LNA
<220>
   <221> misc_feature
   <222> (8)..(8)
   <223> 5-methyl cytosine
<220>
   <221> misc_feature
   <222> (11)..(13)
   <223> beta-D-oxy LNA
<400> 17
   ttgtgctcca taa 13
<210> 18
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (5)..(5)
   <223> 5-methyl cytosine
<220>
   <221> misc_feature
   <222> (14)..(16)
   <223> beta-D-oxy LNA
<220>
   <221> misc_feature
   <222> (15)..(15)
   <223> 5-methyl cytosine
<400> 18
   tttccgtttg tgctcg 16
<210> 19
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (6)..()
   <223> 5-methyl cytosine
<220>
   <221> misc_feature
   <222> (6)..(6)
   <223> 5-methyl cytosine
<220>
   <221> misc_feature
   <222> (14)..(16)
   <223> beta-D-oxy LNA
<220>
   <221> misc_feature
   <222> (14)..(15)
   <223> 5-methyl cytosine
<400> 19
   cattgcattg tttccg 16
<210> 20
   <211> 16
   <212> DNA
   <213> ARTIFICIAL
<220>
   <223> oligonucleotide
<220>
   <221> misc_feature
   <222> (1) .. (16)
   <223> Phosphorothioate linkages
<220>
   <221> misc_feature
   <222> (1)..(2)
   <223> beta-D-oxy LNA
<220>
   <221> misc_feature
   <222> (1)..(1)
   <223> 5-methyl cytosine
<220>
   <221> misc_feature
   <222> (14)..(16)
   <223> beta-D-oxy LNA,
<220>
   <221> misc_feature
   <222> (14)..(15)
   <223> 5-methyl cytosine
<400> 20
   cattgcattg tttccg 16
<210> 21
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (13)..(16)
   <223> beta-D-oxy LNA
<220>
   <221> misc_feature
   <222> (14)..(15)
   <223> 5-methyl cytosine
<400> 21
   cattgcattg tttccg 16
<210> 22
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(4)
   <223> beta-D-oxy LNA
<220>
   <221> misc_feature
   <222> (1)..(1)
   <223> 5-methyl cytosine
<220>
   <221> misc_feature
   <222> (14)..(16)
   <223> beta-D-oxy LNA
<220>
   <221> misc_feature
   <222> (14)..(15)
   <223> 5-methyl cytosine
<400> 22
   cattgcattg tttccg 16
<210> 23
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(4)
   <223> beta-D-oxy LNA
<220>
   <221> misc_feature
   <222> (1)..(1)
   <223> 5-methyl cytosine
<220>
   <221> misc_feature
   <222> (13)..(16)
   <223> beta-D-oxy LNA
<220>
   <221> misc_feature
   <222> (14)..(15)
   <223> 5-methyl cytosine
<400> 23
   cattgcattg tttccg 16
<210> 24
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (12)..(16)
   <223> beta-D-oxy LNA
<220>
   <221> misc_feature
   <222> (14)..(15)
   <223> 5-methyl cytosine
<400> 24
   cattgcattg tttccg 16
<210> 25
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(5)
   <223> beta-D-oxy LNA
<220>
   <221> misc_feature
   <222> (1)..(1)
   <223> 5-methyl cytosine
<220>
   <221> misc_feature
   <222> (14)..(16)
   <223> beta-D-oxy LNA
<220>
   <221> misc_feature
   <222> (14)..(15)
   <223> 5-methyl cytosine
<400> 25
   cattgcattg tttccg 16
<210> 26
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (10)..(10)
   <223> 5-methyl cytosine
<220>
   <221> misc_feature
   <222> (14)..(16)
   <223> beta-D-oxy LNA
<400> 26
   gtttgtgctc gataaa 16
<210> 27
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (9)..(9)
   <223> 5-methyl cytosine
<220>
   <221> misc_feature
   <222> (13)..(15)
   <223> beta-D-oxy LNA
<400> 27
   tttgtgctcg ataaa 15
<210> 28
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (9)..(9)
   <223> 5-methyl cytosine
<220>
   <221> misc_feature
   <222> (12)..(14)
   <223> beta-D-oxy LNA
<400> 28
   tttgtgctcg ataa 14
<210> 29
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (10)..(10)
   <223> 5-methyl cytosine
<220>
   <221> misc_feature
   <222> (13)..(15)
   <223> beta-D-oxy LNA
<400> 29
   gtttgtgctc gataa 15
<210> 30
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (10)..(10)
   <223> 5-methyl cytosine
<220>
   <221> misc_feature
   <222> (12)..(14)
   <223> beta-D-oxy LNA
<400> 30
   gtttgtgctc gata 14
<210> 31
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(2)
   <223> 5-methyl cytosine
<220>
   <221> misc_feature
   <222> (12)..(12)
   <223> 5-methyl cytosine
<220>
   <221> misc_feature
   <222> (14)..(16)
   <223> beta-D-oxy LNA
<400> 31
   ccgtttgtgc tcgata 16
<210> 32
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(15)
   <223> beta-D-oxy LNA
<400> 32
   cgtttgtgct cgata 15
<210> 33
   <211> 13
   <212> DNA
   <213> ARTIFICIAL
<220>
   <223> oligonucleotide
<220>
   <221> misc_feature
   <222> (1)..(13)
   <223> Phosphorothioate linkages
<220>
   <221> misc_feature
   <222> (1)..(2)
   <223> beta-D-oxy LNA
<220>
   <221> misc_feature
   <222> (11)..(13)
   <223> beta-D-oxy LNA
<400> 33
   gtttgtgctc gat 13
<210> 34
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (12)..(14)
   <223> beta-D-oxy LNA
<400> 34
   cgtttgtgct cgat 14
<210> 35
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (1)..(2)
   <223> 5-methyl cytosine
<220>
   <221> misc_feature
   <222> (13)..(15)
   <223> beta-D-oxy LNA
<400> 35
   ccgtttgtgc tcgat 15
<210> 36
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (2)..(3)
   <223> 5-methyl cytosine
<220>
   <221> misc_feature
   <222> (14)..(16)
   <223> beta-D-oxy LNA
<400> 36
   tccgtttgtg ctcgat 16
<210> 37
   <211> 13
   <212> DNA
   <213> ARTIFICIAL
<220>
   <223> oligonucleotide
<220>
   <221> misc_feature
   <222> (1)..(13)
   <223> Phosphorothioate linkages
<220>
   <221> misc_feature
   <222> (1)..(2)
   <223> beta-D-oxy LNA
<220>
   <221> misc_feature
   <222> (1)..(1)
   <223> 5-methyl cytosine
<220>
   <221> misc_feature
   <222> (11)..(13)
   <223> beta-D-oxy LNA
<220>
   <221> misc_feature
   <222> (11)..(11)
   <223> 5-methyl cytosine
<400> 37
   cgtttgtgct cga 13
<210> 38
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (3)..(4)
   <223> 5-methyl cytosine
<220>
   <221> misc_feature
   <222> (14)..(16)
   <223> beta-D-oxy LNA
<220>
   <221> misc_feature
   <222> (14)..(14)
   <223> 5-methyl cytosine
<400> 38
   ttccgtttgt gctcga 16
<210> 39
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (2)..(3)
   <223> 5-methyl cytosine
<220>
   <221> misc_feature
   <222> (13)..(15)
   <223> beta-D-oxy LNA
<220>
   <221> misc_feature
   <222> (13)..(13)
   <223> 5-methyl cytosine
<400> 39
   tccgtttgtg ctcga 15
<210> 40
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA
<220>
   <221> misc_feature
   <222> (2)..(3)
   <223> 5-methyl cytosine
<220>
   <221> misc_feature
   <222> (12)..(14)
   <223> beta-D-oxy LNA
<220>
   <221> misc_feature
   <222> (13)..(13)
   <223> 5-methyl cytosine
<400> 40
   tccgtttgtg ctcg 14
<210> 41
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (3)..(4)
   <223> 5-methyl cytosine
<220>
   <221> misc_feature
   <222> (13)..(15)
   <223> beta-D-oxy LNA
<220>
   <221> misc_feature
   <222> (14)..(14)
   <223> 5-methyl cytosine
<400> 41
   ttccgtttgt gctcg 15
<210> 42
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (6)..(6)
   <223> 5-methyl cytosine
<220>
   <221> misc_feature
   <222> (14)..(16)
   <223> beta-D-oxy LNA
<220>
   <221> misc_feature
   <222> (14)..(14)
   <223> 5-methyl cytosine
<220>
   <221> misc_feature
   <222> (16)..(16)
   <223> 5-methyl cytosine
<400> 42
   gtttccgttt gtgctc 16
<210> 43
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (5)..(5)
   <223> 5-methyl cytosine
<220>
   <221> misc_feature
   <222> (13)..(15)
   <223> beta-D-oxy LNA
<220>
   <221> misc_feature
   <222> (13)..(13)
   <223> 5-methyl cytosine
<220>
   <221> misc_feature
   <222> (15)..(15)
   <223> 5-methyl cytosine
<400> 43
   tttccgtttg tgctc 15
<210> 44
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(2)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (14)..(16)
   <223> beta-D-oxy LNA
<400> 44
   cgtttgtgct cgataa 16
<210> 45
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(16)
   <223> beta-D-oxy LNA
<400> 45
   cgtttgtgct cgataa 16
<210> 46
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(4)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (14)..(16)
   <223> beta-D-oxy LNA
<400> 46
   cgtttgtgct cgataa 16
<210> 47
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(4)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(16)
   <223> beta-D-oxy LNA
<400> 47
   cgtttgtgct cgataa 16
<210> 48
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (12)..(16)
   <223> beta-D-oxy LNA
<400> 48
   cgtttgtgct cgataa 16
<210> 49
   <211> 16
   <212> DNA
   <213> ARTIFICIAL
<220>
   <223> oligonucleotide
<220>
   <221> misc_feature
   <222> (1)..(16)
   <223> Phosphorothioate linkages
<220>
   <221> misc_feature
   <222> (1)..(5)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (14)..(16)
   <223> beta-D-oxy LNA
<400> 49
   cgtttgtgct cgataa 16
<210> 50
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(2)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(15)
   <223> beta-D-oxy LNA
<400> 50
   cgtttgtgct cgata 15
<210> 51
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (14)..(15)
   <223> beta-D-oxy LNA
<400> 51
   cgtttgtgct cgata 15
<210> 52
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(4)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (14)..(15)
   <223> beta-D-oxy LNA
<400> 52
   cgtttgtgct cgata 15
<210> 53
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(2)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (12)..(15)
   <223> beta-D-oxy LNA
<400> 53
   cgtttgtgct cgata 15
<210> 54
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(4)
   <223> beta-D-oxy LNA
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(15)
   <223> beta-D-oxy LNA
<400> 54
   cgtttgtgct cgata 15
<210> 55
   <211> 15
   <212> DNA
   <213> ARTIFICIAL
<220>
   <223> oligonucleotide
<220>
   <221> misc_feature
   <222> (1)..(15)
   <223> Phosphorothioate linkages
<220>
   <221> misc_feature
   <222> (1)..(3)
   <223> beta-D-oxy LNA,
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (12)..(15)
   <223> beta-D-oxy LNA,
<400> 55
   cgtttgtgct cgata 15
<210> 56
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA,
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (12)..(14)
   <223> beta-D-oxy LNA,
<400> 56 14
   cgtttgtgct cgat 14
<210> 57
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(2)
   <223> beta-D-oxy LNA,
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(14)
   <223> beta-D-oxy LNA,
<400> 57
   cgtttgtgct cgat 14
<210> 58
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(2)
   <223> beta-D-oxy LNA,
<220>
   <221> misc_feature
   <222> (1)..(1)
   <223> 5-methyl cytosine
<220>
   <221> misc_feature
   <222> (12)..(14)
   <223> beta-D-oxy LNA,
<400> 58
   cgtttgtgct cgat 14
<210> 59
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(3)
   <223> beta-D-oxy LNA,
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(14)
   <223> beta-D-oxy LNA,
<400> 59
   cgtttgtgct cgat 14
<210> 60
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(4)
   <223> beta-D-oxy LNA,
<220>
   <221> misc_feature
   <222> (1)..(1)
   <223> 5-methyl cytosine
<220>
   <221> misc_feature
   <222> (11)..(11)
   <223> 5-methyl cytosine
<220>
   <221> misc_feature
   <222> (13)..(14)
   <223> beta-D-oxy LNA,
<400> 60
   cgtttgtgct cgat 14
<210> 61
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(2)
   <223> beta-D-oxy LNA,
<220>
   <221> misc_feature
   <222> (1)..(1)
   <223> 5-methyl cytosine
<220>
   <221> misc_feature
   <222> (11)..(14)
   <223> beta-D-oxy LNA,
<220>
   <221> misc_feature
   <222> (11)..(11)
   <223> 5-methyl cytosine
<400> 61
   cgtttgtgct cgat 14
<210> 62
   <211> 14
   <212> DNA
   <213> ARTIFICIAL
<220>
   <223> oligonucleotide
<220>
   <221> misc_feature
   <222> (1)..(14)
   <223> Phosphorothioate linkages
<220>
   <221> misc_feature
   <222> (1)..(4)
   <223> beta-D-oxy LNA,
<220>
   <221> misc_feature
   <222> (1)..(1)
   <223> 5-methyl cytosine
<220>
   <221> misc_feature
   <222> (11)..(14)
   <223> beta-D-oxy LNA,
<220>
   <221> misc_feature
   <222> (11)..(11)
   <223> 5-methyl cytosine
<400> 62
   cgtttgtgct cgat 14
<210> 63
   <211> 15
   <212> DNA
   <213> ARTIFICIAL
<220>
   <223> oligonucleotide
<400> 63
   attgcattgt ttccg 15
<210> 64
   <211> 13
   <212> DNA
   <213> ARTIFICIAL
<220>
   <223> oligonucleotide
<400> 64
   gtttgtgctc gat 13
<210> 65
   <211> 16
   <212> DNA
   <213> ARTIFICIAL
<220>
   <223> oligonucleotide
<400> 65
   cattgcattg tttccg 16
<210> 66
   <211> 14
   <212> DNA
   <213> ARTIFICIAL
<220>
   <223> oligonucleotide
<400> 66
   cgtttgtgct cgat 14
<210> 67
   <211> 16
   <212> DNA
   <213> ARTIFICIAL
<220>
   <223> oligonucleotide
<400> 67
   cgtttgtgct cgataa 16
<210> 68
   <211> 14
   <212> DNA
   <213> ARTIFICIAL
<220>
   <223> oligonucleotide
<400> 68
   ccgtttgtgc tcga 14
<210> 69
   <211> 13
   <212> DNA
   <213> ARTIFICIAL
<220>
   <223> oligonucleotide
<400> 69
   cgtttgtgct cga 13
<210> 70
   <211> 14
   <212> DNA
   <213> ARTIFICIAL
<220>
   <223> oligonucleotide
<400> 70
   tttgtgctcg ataa 14
<210> 71
   <211> 13
   <212> DNA
   <213> ARTIFICIAL
<220>
   <223> oligonucleotide
<400> 71
   ttgtgctcca taa 13
<210> 72
   <211> 16
   <212> DNA
   <213> ARTIFICIAL
<220>
   <223> oligonucleotide
<400> 72
   tttccgtttg tgctcg 16
<210> 73
   <211> 16
   <212> DNA
   <213> ARTIFICIAL
<220>
   <223> oligonucleotide
<400> 73
   attgcattgt ttccgt 16
<210> 74
   <211> 15
   <212> DNA
   <213> ARTIFICIAL
<220>
   <223> oligonucleotide
<400> 74
   cgtttgtgct cgata 15
<210> 75
   <211> 19
   <212> DNA
   <213> ARTIFICIAL
<220>
   <223> oligonucleotide
<400> 75
   tccgtttgtg ctcgataaa 19
<210> 76
   <211> 19
   <212> DNA
   <213> ARTIFICIAL
<220>
   <223> oligonucleotide
<400> 76
   tttgtgctcg ataaaaata 19
<210> 77
   <211> 19
   <212> DNA
   <213> ARTIFICIAL
<220>
   <223> oligonucleotide
<400> 77
   attgtttccg tttgtgctc 19